(19) Europäisches Patentamt / European Patent Office / Office européen des brevets

(11) **EP 3 237 595 B1**

(12) **EUROPEAN PATENT SPECIFICATION**

(45) Date of publication and mention
of the grant of the patent:
**26.01.2022 Bulletin 2022/04**

(21) Application number: **15874282.5**

(22) Date of filing: **22.12.2015**

(51) International Patent Classification (IPC):
*C12M 1/18* (2006.01)    *C12M 1/24* (2006.01)
*C12Q 1/68* (2018.01)    *C12N 1/02* (2006.01)
*C12N 5/078* (2010.01)    *C12N 9/12* (2006.01)
*C12N 9/22* (2006.01)    *C08L 27/12* (2006.01)
*C08F 255/00* (2006.01)

(52) Cooperative Patent Classification (CPC):
(C-Sets available)
**C12M 47/04; C08F 259/08; C08J 7/18;
G01N 33/543; G01N 33/54353**            (Cont.)

(86) International application number:
**PCT/US2015/067281**

(87) International publication number:
**WO 2016/106283 (30.06.2016 Gazette 2016/26)**

(54) **CAPTURE SYSTEM OF CELLS AND METHODS**

ERFASSUNGSSYSTEM VON ZELLEN UND VERFAHREN

SYSTÈME DE CAPTURE DE CELLULES ET PROCÉDÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.12.2014 US 201462095140 P
21.12.2015 US 201514976043**

(43) Date of publication of application:
**01.11.2017 Bulletin 2017/44**

(73) Proprietor: **Saint-Gobain Performance Plastics
Corporation
Solon, OH 44139 (US)**

(72) Inventors:
• **CIVEL, Edouard**
  **75018 Paris (FR)**
• **CLARK, Sarah Louise**
  **Somerville, Massachusetts 02143 (US)**
• **CULLIS, Herbert Myers**
  **Gaithersburg, Maryland 20877 (US)**
• **BOGHOSIAN, Natasha Anna**
  **Boston, Massachusetts 02118 (US)**
• **MIRIPOL, Jeffrey Ellis**
  **Hockessin, DE 19707 (US)**
• **GARCES, Camila A.**
  **Brookline, Massachusetts 02445 (US)**

(74) Representative: **Zimmermann & Partner
Patentanwälte mbB
Postfach 330 920
80069 München (DE)**

(56) References cited:
**WO-A1-03/008011        WO-A1-2013/067399
US-A1- 2010 304 978      US-A1- 2014 148 358
US-A1- 2014 255 976      US-B2- 6 949 355**

• **CHAN-HEE JUNG ET AL: "Poly(acrylic
acid)-Grafted Fluoropolymer Films for Highly
Sensitive Fluorescent Bioassays", ACS APPLIED
MATERIALS & INTERFACES, vol. 5, no. 6, 14
March 2013 (2013-03-14), pages 2155-2160,
XP055466502, US ISSN: 1944-8244, DOI:
10.1021/am303197n**

- SADURNI PATRICIA ET AL: "Immobilization of streptavidinhorseradish peroxidase onto a biotinylated poly(acrylic acid) backbone that had been radiation-grafted to a PTFE film", JOURNAL OF BIOMATERIALS SCIENCE. POLYMER EDIT, VSP, UTRECHT, NL, vol. 16, no. 2, 1 January 2005 (2005-01-01), pages 181-187, XP009099187, ISSN: 0920-5063
- T G VARGO ET AL: "Synthesis and characterization of fluoropolvmeric substrata with immobilized minimal peptide sequences for cell adhesion studies. I", JOURNAL OF BIOMEDICAL MATERIALS RESEARCH, vol. 101, no. 1, 1 June 1995 (1995-06-01), page 767, XP055466506,
- J P Ranieri ET AL: "Neuronal cell attachment to fluorinated ethylene propylene films with covalently immobilized laminin oligopeptides YIGSR and IKVAV. II", Journal of biomedical materials research, 1 June 1995 (1995-06-01), pages 779-785, XP055466504, UNITED STATES DOI: 10.1002/jbm.820290614 Retrieved from the Internet: URL:https://onlinelibrary.wiley.com/doi/pd f/10.1002/jbm.820290614
- K Lachmann ET AL: "Surface modification of closed plastic bags for adherent cell cultivation", , 31 December 2011 (2011-12-31), XP55747276, DOI: 10.1051/epjap/2011100467 Retrieved from the Internet: URL:https://www.epjap.org/articles/epjap/a bs/2011/07/ap100467/ap100467.html [retrieved on 2020-11-05]

(52) Cooperative Patent Classification (CPC): (Cont.)

C-Sets
C08F 259/08, C08F 220/06

**Description**

TECHNICAL FIELD

The present disclosure concerns at least the fields of cell biology, molecular biology, materials science, cell therapy, and medicine. Certain embodiments of the disclosure relate to systems for capturing desired cells and, optionally, processing them, including for *in vivo* delivery.

BACKGROUND

The immune system of humans can be divided into two parts, the innate immune system, which answers quickly to the main part of the invaders, and the adaptive immune system, which is able to design a very specific answer to the intrusion of a pathogen. Dendritic cells link those two systems and are the key element that will initiate a strong response against invader pathogen. Monocytes are pluripotent cells that can differentiate into various kinds of cells, including macrophages and dendritic cells. In general, monocytes are present in the whole blood at a concentration of 1000 cells/$\mu$L. They represent approximately 5% of the white blood cells present in the blood of an adult.

Dendritic cells activate T cells in the lymph node. Once activated by dendritic cells, T cells are the most effective defense component, stimulating the other parts of the immune system, and being able to specifically kill the infected cells on a large scale. Thus, a key part of the adaptive immune system is the activation of T cells by the dendritic cells. The dendritic cells bridge the innate immune system and the adaptive immune system, engulfing the invaders to present their antigens to the T cells and activating them to kill infected cells.

Because monocytes can differentiate into cells useful to the innate immune system and the adaptive immune system, they are powerful tools to influence an individual's immune system. Given their relatively low concentration in blood, their unscathed isolation in high yield and their purity remains a challenge. US 2014/255976 A1 relates to a surface coating for capture circulating rare cells, comprising a nonfouling composition to prevent the binding of non-specific cells and adsorption of serum components; a bioactive composition for binding the biological substance, such as circulating tumor cells; with or without a linker composition that binds the nonfouling and bioactive compositions. CHAN-HEE JUNG ET AL: "Poly(acrylic acid)-Grafted Fluoropolymer Films for Highly Sensitive Fluorescent Bioassays", ACS APPLIED MATERIALS & INTERFACES, vol. 5, no. 6, 114 March 2013 (2013-03-14), pages 2155-2160 relates to poly(acrylic acid)-grafted fluoropolymer films for highly selective fluorescent bioassays. SADURNI PATRICIA ET AL: "Immobilization of streptavidinhorseradish peroxidase onto a biotinylated poly(acrylic acid) backbone that had been radiation-grafted to a PTFE film", JOURNAL OF BIOMATERIALS SCIENCE. POLYMER EDIT. VSP, UTRECHT, NL, vol. 16, no. 2, 1 January 2005 (2005-01-01), pages 181 -187 describes methods of immobilization of streptavidinhorseradish peroxidase onto a biotinylated poly(acrylic acid) backbone.

T. G. VARGO ET AL: "Synthesis and characterization of fluoropolvmeric substrata with immobilized minimal peptide sequences for cell adhesion studies. 1", Journal of biomedical materials research, vol. 101, no. 1, p.767 describes immobilization of oligopeptides onto fluoropolymer substrates for the purpose of cell adhesion. J. P. RANIERI ET AL: "Neuronal cell attachment to fluorinated ethylene propylene films with covalently immobilized laminin oligopeptides YIGSR and IKVAV. II", Journal of biomedical materials research, 1 June 1995 (1 995-06-01 ), pages 779-785 relates to neuronal cell attachment to FEP films using covalently immobilized laminin peptides. K. Lachmann ET AL: "Surface modification of closed plastic bags for adherent cell cultivation", 31 December 2011 (2011-12-31) describes the surface modification of closed plastic bags for adherent cell cultivation. There remains an on-going need to provide a more effective approach to isolate monocytes without resorting to traditional, less-efficient procedures. As such, an improved biological system for isolation of these and other cells is desired.

BRIEF SUMMARY

The present invention is defined in the claims. Embodiments of the disclosure include systems and methods for isolation of cells, from a sample. The disclosure provides a system comprising a modified surface for the capture of certain desired cells from a sample. Embodiments of the disclosure include a closed anergic system for isolating desired cells from a sample and, also further processing the cells, including at least culturing the cells (such as immune cells) for cell therapy. Certain embodiments of the disclosure include an *ex vivo* system for cell isolation and, growth and/or processing thereafter. The systems of the disclosure allow for isolation of cells in a one-step process or one-system process without harming the cells such that they may be later utilized, including at least for *in vivo* applications.

The system comprises at least one container, namely a bag, that comprises a surface that is configured for the collection of at least one particular type of cells from a sample. In particular aspects, more than one cell type can be captured by the same moiety, given that cells have a variety of surface markers. In certain aspects, more than one cell type can be captured by a mixture of non-identical moieties in the same system. In specific embodiments, the system comprises a container having sheets of film, such as wherein the sheets are laser welded in a serpentine bag, for example. In some embodiments, the container comprises a bead, microstructure, or apparatus other than a tube or bag or in addition to the bag. Any cell may be isolated using embodiments of the disclosure, but in particular embodiments the cell is a blood cell or an immune cell, for example. Exemplary immune cells for capture include at least monocytes, although other immune cells may be obtained with systems and methods of the disclosure.

The system comprises the container comprising an inner wall comprising a polymer with a functionalized surface. The container has a continuous coverage of cell-specific aptamers (oligonucleic acid molecules that bind to a specific target molecule), in specific embodiments. The inner wall of the container is comprised of polymer of a total organic carbon (TOC) in water of less than $0.1 mg/cm^2$, $0.09 mg/cm^2$, $0.08 mg/cm^2$, $0.07 mg/cm^2$, $0.06 mg/cm^2$, $0.05 mg/cm^2$, $0.04 mg/cm^2$, $0.03 mg/cm^2$, $0.02 mg/cm^2$, $0.01 mg/cm^2$, $0.009 mg/cm^2$, $0.008 mg/cm^2$, $0.007 mg/cm^2$, $0.006 mg/cm^2$, $0.005 mg/cm^2$, $0.004 mg/cm^2$, $0.003 mg/cm^2$, $0.002 mg/cm^2$, $0.001 mg/cm^2$, or is nondetectable. In particular embodiments, the TOC in water is less than an amount in a range from $0.001 mg/cm^2$ to $0.1 mg/cm^2$, $0.001 mg/cm^2$ to $0.095 mg/cm^2$, $0.001 mg/cm^2$ to $0.075 mg/cm^2$, $0.001 mg/cm^2$ to $0.05 mg/cm^2$, $0.001 mg/cm^2$ to $0.01 mg/cm^2$, $0.001 mg/cm^2$ to $0.005 mg/cm^2$, or $0.001 mg/cm^2$ to $0.025 mg/cm^2$. In particular embodiments, the TOC in water is less than an amount in a range from $0.01 mg/cm^2$ to $0.1 mg/cm^2$, $0.01 mg/cm^2$ to $0.075 mg/cm^2$, $0.01 mg/cm^2$ to $0.05 mg/cm^2$, or $0.01 mg/cm^2$ to $0.025 mg/cm^2$. In particular embodiments, the TOC in water is less than an amount in a range from $0.05 mg/cm^2$ to $0.1 mg/cm^2$, $0.05 mg/cm^2$ to $0.09 mg/cm^2$, $0.05 mg/cm^2$ to $0.075 mg/cm^2$, or $0.05 mg/cm^2$ to $0.06 mg/cm^2$. In particular embodiments, the TOC in water is less than an amount in a range from $0.005 mg/cm^2$ to $0.1 mg/cm^2$, $0.005 mg/cm^2$ to $0.095 mg/cm^2$, $0.005 mg/cm^2$ to $0.075 mg/cm^2$, $0.005 mg/cm^2$ to $0.05 mg/cm^2$, $0.005 mg/cm^2$ to $0.025 mg/cm^2$, or $0.005 mg/cm^2$ to $0.01 mg/cm^2$.

In specific embodiments, the TOC of fluorinated ethylene propylene (FEP) is $0.00005 mg/cm^2$ of interior wetted surface of an article (0.001 mg/g of article); the TOC of silicone materials, such as silicone tubing, is $0.021 mg/cm^2$ of interior wetted surface of an article (0.023 mg/cm of tubing) and $0.008 mg/cm^2$ (0.009 mg/cm); the TOC for a historically used cell culture bag is $0.002 mg/cm^2$ of interior wetted surface of an article (0.032 mg/g of article).

The polymer is a fluoropolymer (fluorocarbon-based polymer with multiple strong carbon-fluorine bonds), namely fluorinated ethylene propylene (FEP). Functionalization of the inner wall may be by any means that is suitable for the intended application of the system and/or reagents for use in the system. In specific embodiments, functionalization of the inner wall includes functionalizing the wall so that there is a specific starting surface with a carboxy group, or amine group. In specific embodiments, the functionalization is with a carboxylic acid, followed by linking the carboxylate to an avidin protein *via* peptide linkage. In specific embodiments, the immobilized avidin protein serves as a bonding site for biotinylated primers or biotinylated aptamers. The functional group may have linked thereto, directly or indirectly, a DNA sequence as a primer to build an aptamer using RCA (rolling circle amplification), in at least some cases. Thus, in specific aspects, the disclosure includes use of aptamer "tentacles" to catch and release specific cells in a sample without harming them. Isolation of specific cells from a sample in a one-step process or one-system process without deleteriously affecting the function of the cells is encompassed in the disclosure. In specific embodiments, the systems of the disclosure are single-use.

Embodiments include an aptamer (such as one designed by a cell SELEX process) with a high specificity and affinity for desired cells, such as monocytes. A closed system (including at least one container, such as a bag or tube and comprised at least in part of fluoropolymer) is employed, which interior surface is functionalized with long sequences of DNA, for example. These tentacles may be generated by any suitable method, although in specific embodiments they are generated with RCA of a first template directed to the aptamer. Then, each tentacle has at least several sites highly specific to the desired cells. In certain embodiments, the length of an aptamer is at least about 5, 6, 7, 8, 9, 10, 11, 12, 13, 14, 15, 16, 17, 18, 19, 20, 21, 22, 23, 24, 25, 26, 27, 28, 29, or 30 or more basepairs in length and so forth. In specific embodiments, the length of an aptamer is no more than 5,6, 7, 8, 9, 10, 11, 12, 13, 14, 15, 16, 17, 18, 19, 20, 21, 22, 23, 24, 25, 26, 27, 28, 29, or 30 or more basepairs in length. The sample is processed on the surface of the system (through the at least one container that may be a bag, tubing, bead, plate, or microstructure), and thereafter only the desired cells remain attached to the tentacles. Then, a method is used to effect release of the desired cells from the system that were separated from the other undesired cells. Restriction enzyme digestion, heating, and/or complementary DNA may be employed to release the cells.

In at least some cases, the bag is further defined as a tube, and the tube may be comprised of pliable material such that it is capable of being configured in a shape for ease of use or storage (such as a serpentine configuration, for example). The fluoropolymer layer may include at least a major surface and a reactive moiety comprising a functional group. The reactive moiety (RM) can be attached to the major surface of the fluoropolymer, such as covalently attached, for example. The reactive moiety may be the amount of chemical groups that geometrically fits on a surface, in at least certain aspects. The reactive moiety may have a surface concentration of at least 50 reactive moieties per square micron, *i.e.* 50 $RM/\mu m^2$ of the major surface. In certain embodiments, the reactive moiety can have a surface concentration of at least 55 $RM/\mu m^2$, 60 $RM/\mu m^2$, 65 $RM/\mu m^2$, 70 $RM/\mu m^2$, 75 $RM/\mu m^2$, 80 $RM/\mu m^2$, 85 $RM/\mu m^2$, 90 $RM/\mu m^2$, 95 $RM/\mu m^2$, 100 $RM/\mu m^2$, 200 $RM/\mu m^2$, 500 $RM/\mu m^2$, 750 $RM/\mu m^2$, or 1000 $RM/\mu m^2$.

In one embodiment, the system for isolation of the biological matter comprises an inlet and the container (such as tubing) connected to the inlet at a first end. The tubing includes a fluoropolymer layer. The fluoropolymer layer comprises a major surface and a reactive moiety that comprises the functional group. The reactive moiety is covalently attached to the major surface of the fluoropolymer. The reactive moiety may have a surface concentration of at least 50 reactive moieties per square micron (50 RM/$\mu$m$^2$). In some embodiments, the system further includes an outlet connected to a second end of the tubing.

In particular embodiments of the system, a process of isolating a biological matter comprises providing a biological sample. The process can further include applying the biological sample through a tubing as defined above. The tubing includes a fluoropolymer layer. The fluoropolymer layer comprises a major surface and a reactive moiety. The reactive moiety is attached to the surface of the fluoropolymer, namely covalently attached. The covalently attached reactive moiety has a surface concentration $S_c$. In particular embodiments, the process comprises immobilizing a single desired cells from a biological sample onto the surface. The process can further include removing the biological sample, less the desired cells, from the tubing. In one embodiment, at least 90 mol% of the single cells is immobilized on the surface based on surface concentration $S_c$ and the length L, although in some cases at least 80 mol%, 81 mol%, 82 mol%, 83 mol%, 84 mol%, 85 mol%, 86 mol%, 87 mol%, 88 mol%, 89 mol%, 90 mol%, 91 mol%, 92mol%, 93 mol%, 94 mol%, 95 mol%, 96 mol%, 97 mol%, 98 mol% or 99 mol% of the single desired cells is immobilized based on surface concentration $S_c$ and the length $L$ In some embodiments, the process further comprises obtaining a biological sample from an individual. The obtaining may occur by any means, such as drawing blood from an individual, collecting bone marrow from the individual, liposuction, surgical sampling (with a catheter, endoscopy, and the like), other methods of sampling solid and liquid tissues, and so forth.

In a certain embodiment, the system comprises a bag that includes an inlet. In specific embodiments, the bag comprises an outlet. In some cases, the bag can further include a capture element. The capture element comprises a surface and a capturing moiety. In a specific aspect, the capturing moiety can include a biotin compound. The capturing moiety can include an avidin protein. The capturing moiety may include either a biotin compound or an avidin protein, but not both. In an aspect of the system, a bag comprises a capturing moiety that may be covalently attached to the surface of the bag. In specific embodiments, the capturing moiety can have a specificity to immobilize cells of any kind (specific examples include at least white blood cells). In particular embodiments, the avidin is a direct or indirect link to attach the DNA and does not capture the cell itself.

In one embodiment, the system comprises a bag that comprises at least 10 million capturing sites for cells per 100 mL volume, although the bag may comprise at least 15 million, 20 million, 25 million, 30 million, 50 million, 75 million, and so forth number of capturing sites for cells per 100 mL volume; in a specific case, the cells are hematopoietic stem cells, for example. Each capturing site can include a capture element. The capture element can include a surface and a capturing moiety. The capturing moiety may be attached to the surface such as covalently attached.

In some embodiments, there is a method of isolating cells from a sample, as defined in claim 10, the method comprising providing the sample. In certain cases, there are methods of isolating blood or immune cells (including at least white blood cells) that includes providing a sample from an individual. In specific embodiments, the method comprises providing a blood or bone marrow sample from an individual. The method may include extraction of the blood or bone marrow sample from the individual. The method may include transferring a sample (such as a blood sample) into an apparatus of the disclosure, including, for example, the aforementioned container.

In certain embodiments, a method of separating certain cells from a biological sample includes providing a sample from a mammal, such as a human, dog, cat, horse, pig, sheep, chimp, baboon, gorilla, or goat, for example. In some cases, a method of separating monocytes from a biological sample includes providing a blood sample from a mammal. The method can include transferring the sample (such as blood) into one of the aforementioned containers.

Embodiments of the disclosure also include an artificial blood vessel that induces attachment of monocytes on the surface of the vessel structure, and then attachment of monocytes to the surface is induced with the use of various cytokines, allowing separation of monocytes from whole blood as well as removing them from the surface of the vessel thereafter. The environment of the vessel may comprise one or more chemokines for stimulation of the adhesion of monocytes and, once adhered, the monocytes may develop a stronger interaction based on their own actin skeleton. Thus, specific embodiments of the disclosure allow for absence of high shear stress.

In specific embodiments, the TOC of fluorinated ethylene propylene (FEP) is 0.0005 mg/cm$^2$ of interior wetted surface of an article (0.001 mg/g of article); the TOC of silicone materials, such as silicone tubing, is 0.021 mg/cm$^2$ of interior wetted surface of an article (0.023 mg/cm of tubing) and 0.008 mg/cm$^2$ of interior wetted surface of an article (0.009 mg/cm of tubing); the TOC for a historically used cell culture bag is 0.002 mg/cm$^2$ of interior wetted surface of an article (0.032 mg/g of article). The functional groups are directly or indirectly attached to the cell-capturing moiety. In some embodiments, the functional groups are attached to the polymer through a linker, and the linker may be a linear alkylene group (a methylene group, an ethylene group, a propylene group, a butylene group, a pentylene group, or a hexylene group), a branched alkylene group, a cyclic alkylene group, an arenediyl group, an oligomeric ethylene glycol group such as tetra ethylene glycol, a saccharide group, or a combination thereof. In specific embodiments, the functional groups have attached directly thereto a first member of a binding pair. In certain aspects, the cell-capturing moiety comprises a second member of a binding pair. In certain embodiments, the functional groups have attached directly thereto a first member of a binding pair and the cell- capturing moiety comprises a second member of a binding pair, wherein said first and second members of the binding pair are bound to each other. In cases when the first member of the binding pair is an avidin species, the second member of the binding pair is biotin, for example. When the first member of the binding pair is biotin, the second member of the binding pair is an avidin species, for example. Examples of avidin species include avidin, streptavidin, or neutravidin. In some cases, the functional groups are a carboxy group, hydroxyl group, aldehyde group, carbonyl group, amine group, imine group, amide group, an alkyne group, an alkene group, an aziridine group, an epoxy group, an isonitrile group, an isocyanide group, a tetrazine group, alkyl group, an aminoethyl amide group, an ester group, a thiol group, an anhydride group, a disulfide group, a phenol group, a guanidine group, a thioether group, an indole group, an imidazole group, a diazonium group, or a combination thereof. In some embodiments, the cell-capturing moiety binds the cell directly. In particular embodiments, the cell- capturing moiety indirectly binds the cell or produces a molecule that directly binds the cell. The cell-capturing moiety or the molecule produced by the cell-capturing moiety is nucleic acid. In specific embodiments, the cell-capturing moiety comprises a nucleic acid template, and it may be a circular DNA template. The nucleic acid template may comprise sequence that is complementary to sequence that directly binds to the cell. In particular embodiments, molecules produced by the cell-capturing moiety comprises one or more aptamers. The polymer is fluorinated ethylene propylene (FEP).

The system is a closed system.

In embodiments for the container of the cell isolation, the container further comprises an enzyme, such as a polymerase, including at least phi29 polymerase. The container comprises a bag. The container may comprise two or more apertures. In certain embodiments, the system comprises one or more additional containers attached in-line to the container. In specific embodiments, a second container comprises one or more cell growth agents. In certain cases, there is a third container that comprises one or more antigens, such as tumor antigens. In some cases, there is a fourth container that is configured to concentrate biological agents, such as cells, which may be cells from a sample. Examples of cells include monocytes, blood cells, immune cells, or a mixture thereof.

In certain embodiments of the system, there is a second container that comprises one or more membranes (see at least certain embodiments in U.S. Provisional Patent Application Serial No. 62/095,197 and U.S. Provisional Patent Application Serial No. 62/095,116, both of which applications are incorporated by reference herein in their entirety). The membranes may be porous. In some cases, there is a second container that comprises two or more apertures. In particular embodiments, a second container comprises two inlets and two outlets. In particular embodiments, there is a second container that comprises one or more membranes and comprises two, three, or four apertures. In specific embodiments, a first membrane is configured to selectively fluidly separate a first inlet from a chamber in the container and a second membrane is configured to selectively fluidly separate a first outlet from the cavity. In particular embodiments, a second inlet is positioned at the chamber, a second outlet is positioned at the chamber, or both. In specific embodiments, the second container comprises an inner surface comprising a polymer having a total organic carbon (TOC) in water of less than $0.1 mg/cm^2$. In specific embodiments, the TOC of fluorinated ethylene propylene (FEP) is $0.0005 mg/cm^2$ of interior wetted surface of an article (0.001 mg/g of article); the TOC of silicone materials, such as silicone tubing, is $0.021 mg/cm^2$ of interior wetted surface of an article (0.023 mg/cm of tubing) and $0.008 mg/cm^2$ of interior wetted surface of an article (0.009 mg/cm of tubing); the TOC for a historically used cell culture bag is $0.002 mg/cm^2$ of interior wetted surface of an article (0.032 mg/g of article).

Further described herein is a method of preparing a system as contemplated herein, comprising the steps of providing a container comprising an inner surface comprising a polymer having a TOC in water of less than $0.1 mg/cm^2$; and attaching functional groups to the inner surface. In specific embodiments, the attaching step is by oxidation, by Grignard reagent, or corona treatment. In some embodiments, the method further comprises the step of attaching a first member of a binding pair to the functional groups. In specific embodiments, the step of attaching a first member of a binding pair to the functional groups comprises activation of the functional groups. Activation may comprise exposure of the functional groups to sodium acetate with a mixture of 1-ethyl-3-(3-dimethylaminopropyl) carbodiimide (EDC) and N-hydroxysuccinimide (NHS). In specific embodiments, a method further comprises the step of attaching a second member of a binding pair to a biological agent- capturing moiety, such as a biological agent-capturing moiety that is attached directly or indirectly to the functional groups through the second member of a binding pair. The biological agent-capturing moiety may be a circular DNA template and primer and the method further comprises the step of providing a polymerase (such as one that extends the DNA template under suitable conditions) and nucleotides to the container. The polymerase may be phi29 polymerase.

Further described herein is a method for isolating particular cells from a sample from at least one individual comprising the steps of: providing a system as contemplated herein; and subjecting to the system a sample comprising a mixture of cells under conditions that the particular cells in the mixture are able to selectively bind to the cell- capturing moiety or selectively bind to a molecule produced by the cell- capturing moiety (such as nucleic acid). The sample may be blood or bone marrow. In certain embodiments, the subjecting step further comprises providing an enzyme to the system, such as a polymerase. Methods of the disclosure may further comprise the step of obtaining the sample from an individual. The individual may be in need of cell therapy, such as cell therapy for cancer. In particular embodiments, following isolation of the particular cells from the sample, the cells are collected from the system. They may be collected by release of the cells from the cell- capturing moiety or the molecule generated by the cell- capturing moiety. In specific embodiments, the release is by heating under suitable conditions. In particular embodiments, when the cell- capturing moiety or the molecule generated by the cell-capturing moiety is a nucleic acid, the cells are released by exposure to one or more endonucleases and/or exposure to a nucleic acid complementary to the respective moiety or molecule. In specific embodiments, following isolation of the particular cells from the sample, the cells are further processed, and they may be further processed in one or more additional containers in the system. The cells may be further processed by culturing the cells, exposing the cells to one or more antigens, differentiating the cells, expanding the cells, concentrating the cells, purifying the cells, or a combination thereof. In specific embodiments, a therapeutically effective amount of the collected cells are provided to an individual in need of cell therapy.

In some embodiments, there is a kit comprising a system as contemplated herein, said system housed in a suitable container. In specific embodiments, the system further comprises an apparatus for sample collection or sample storage or both. The apparatus may be a vial, syringe, cup, catheter, bag, tube, or a combination thereof. The kit may further comprise a polymerase, such as phi29 polymerase. The kit may further comprise an endonuclease, a buffer, and/or one or more cytokines, chemokines, or growth factors.

BRIEF DESCRIPTION OF THE DRAWINGS

For a more complete understanding of the present disclosure, reference is now made to the following descriptions taken in conjunction with the accompanying drawings, in which:

FIGS. 1A-1B illustrate exemplary embodiments for a closed system for isolating and culturing cells for cell therapy;

FIG. 2 demonstrates an embodiment of exposure of an aptamer-comprising bag to blood for capture of particular immune cells;

FIG. 3 demonstrates generation of oxidized FEP from Saint-Gobain® Norton C-treated FEP film, as an example;

FIG. 4 shows beads that are attached to an FEP substrate for isolation of cells of interest;

FIG. 5 shows an FTIR spectrum of treated film oxidized, treated film non-oxidized, and untreated film oxidized;

FIG. 6 illustrates an example of isolation of desired cells using biotinylated aptamers added to a blood sample and introduced to a tube comprising a coating of FEP with neutravidin;

FIG. 7 includes an illustration of a system for isolation of biological matter in accordance with an embodiment;

FIG. 8 includes an example of a flow chart for a process to modify a surface in accordance with an embodiment;

FIG. 9 includes an example of a process illustration for isolating a cells from a biological sample;

FIG. 10 illustrates FTIR spectrums into 4000-1400 $cm^{-1}$ range of the untreated film after Avidin and Neutravidin (NAv) adsorption. Control sample was in contact with suspension and rinse buffer (NaOAc and deionized $H_2O$). Amide presence is observed at 1630$cm^{-1}$. Higher peaks at 3400 $cm^{-1}$ for protein sample show also presence of protein to surface;

FIG. 11 shows FTIR spectrums into 4000-1400 $cm^{-1}$ range of the C-Treated film after Avidin and Neutravidin (NAv) adsorption. Control sample was in contact with suspension and rinse buffer (NaOAc and deionized $H_2O$). Amide presence is observed at 1630$cm^{-1}$. Higher peaks at 3400 $cm^{-1}$ for protein sample show also presence of protein to surface;

FIG. 12 demonstrates absorbance at 600 nm of methylene blue molecules binding to carboxyl group on Untreated (1st column), C-Treated (2nd column) and oxidized C-Treated FEP film (4th column). 3rd column shows the absorbance of the oxidized C-Treated film before its reaction with methylene blue (blank control);

FIG. 13 shows total absorbance and absorbance at 600 nm of methylene blue molecules bound to carboxyl group

on Untreated (1st column), C-Treated (2nd column) and oxidized C-Treated FEP film (4th column). 3rd column shows the absorbance of the oxidized C-Treated film before its reaction with methylene blue (blank control);

FIG. 14 shows a typical spectrum of the FEP film dye with the methylene blue into UV-Vis range 350-1050 nm. Oxidized control is the oxidized C-Treated film before reaction with methylene blue;

FIGS. 15A-15D provide SEM images of Untreated FEP with Adsorbed Neutravidin (A to D). Image D shows attachment of biotin coating beads to the functionalized Neutravidin surface (Untreated FEP with Adsorbed Neutravidin);

FIG. 16 shows SEM images of C-Treated FEP with Adsorbed Neutravidin (left) and Oxidized C-Treated with Conjugated Neutravidin (right);

FIG. 17 provides Biotin coated microbeads on untreated FEP with adsorbed Neutravidin;

FIG. 18 shows an optical image of biotin microbeads (black spots of d ~ 0.8-1$\mu$m) on Untreated FEP with adsorbed neutravidin takes with Olympus DSX 500 microscope. Scale: 341-342 $\mu$m, Magnification: 3X on 20X lens;

FIG. 19 provides absorbance at 660 nm of the stained protein on different types of modified FEP films including an untreated control. The first column of each group of columns represents control sample exposed only to buffer solution, the second column of each group represents the adsorbed Avidin, and the third column of each group represents the sample with adsorbed Neutravidin. All measurements were obtained after performing the protein staining reaction;

FIG. 20 demonstrates a typical spectrum into UV-Vis range 350-1050 nm on C-Treated film before and after dye reaction with proteins;

FIG. 21 shows FTIR spectrums of 1% SDS washed Oxidized C-Treated film previously modified with adsorbed neutravidin (NAv);

FIG. 22 provides an FTIR spectrum on Untreated FEP with adsorbed neutravidin after ultrasonication step for 5 min at high power (US-A) and low power (US-B). No change was observed. The thin solid line represents the spectrum of adsorbed protein prior to ultrasonication;

FIG. 23 shows an FTIR spectrum on C-Treated FEP after ultrasonication step for 5 min at high power (US-A) and low power (US-B);

FIG. 24 illustrates an example of Enzyme-Linked ImmunoSorbent Assay on attached Neutravidins to FEP film;

FIG. 25 shows FTIR spectrum in the 4000-1400 cm-1 range of polyacrylic acid functionalized FEP film (pAA-FEP), a spectrum of pAA-FEP film that has been activated by EDC/NHS chemistry in MES buffer, a spectrum of further conjugation reaction of the activated pAA-FEP film surface conjugated with avidin protein, and a spectrum of avidin protein adsorbed on the surface for reference;

FIG. 26 provides a fluorescence image of fluorophore-tagged chemically attached avidin to pAA-FEP surfaces adjacent to a control section of FEP that was not functionalized with pAA; and

FIG. 27 shows FTIR spectrum in the 4000-1400 cm-1 range of polyacrylic acid functionalized FEP film (pAA-FEP), a spectrum of pAA-FEP film exposed to a DNA-free magnesium ion coupling buffer, a spectrum of pAA-FEP film exposed to EDC/NHS reagents; a spectrum of EDC/NHS activated pAA-FEP film after a conjugation reaction with amine-terminated DNA; a spectrum of pAA-FEP film soaked in a DNA solution.

DETAILED DESCRIPTION

It is an object of the present description to provide systems and methods, to isolate at least one biological agent (i.e. cells) from a biological sample with high specificity. This description also includes one or more processes for preparing such systems. The biological agents include entire cells. In certain embodiments, the biological agents comprise any type of blood cell, any type of stem cell, or any type of immune cell, for example. The cell from the sample may be normal or may be diseased. In another particular embodiment, the biological agents comprise a blood cell, such as a white blood cell. In some particular embodiments, the biological agents comprises an immune cell, such as a monocyte or T cell. In specific embodiments, the biological agent is a virus, cell group, or microorganism, for example. Accordingly, the biological sample can include samples from any organ or tissue of an animal, including human. For example, blood can be the biological sample to isolate monocytes. In another embodiment, bone marrow can be the biological sample to isolate hematopoietic stem cells, *i.e.,* a precursor to monocytes. In a particular embodiment, the systems allow isolation of macromolecular compounds, cell subunits, or cells with a specificity while maintaining the biological activity of the species, *i.e.,* with a low degradation rate.

Embodiments of the disclosure provide for a system that allows for isolation of desired cells from a mixture of cells, such as the mixture of cells being from a sample. The sample may be from an individual, including a mammal, such as a human. In particular embodiments, the system includes a container that is configured to isolate the desired cells. In some embodiments, the system is multi-partite, having additional containers other than the container for cell isolation. Such containers may be configured in a sequential path for movement of the cells, in some cases; in such embodiments, the cells are subjected to different environments or manipulations for further processing. In a particular embodiment, the cell isolation container is the first container in a path for sequential processing of the cells. In cases wherein there are two or more containers in the system, the containers may be configured in-line, such that the sample or cells being processed move successively from one container to another. In specific embodiments the multi-container system is configured linearly, and such a linear path may be horizontal or linear, or neither.

In particular embodiments, the sample is not processed prior to delivery to the system, although the sample may have been stored under sufficient conditions prior to delivery to the system. In specific embodiments, the sample is not subjected to separation techniques (including centrifugation) prior to delivery to the system, is not subjected to addition of one or more biological agents prior to delivery to the system (including, for example, an anti-clotting agent), and so forth. In specific embodiments, the sample is not subjected to contact with surfaces that will trigger an immune cell response. The sample may have been obtained from the individual by another party than the party that performs the system processing.

In one embodiment, the system can be complete and ready for isolation of a biological agent prior to its use. In another embodiment, the systems can be provided to a user at a preliminary stage and a user, such as a lab technician, modifies the system as to the specificity of the desired biological agent. In one embodiment, the system can be isolated from further processing. In another embodiment, the systems of the disclosure can be included in a sequence of processes, where the isolation of a biological agent is one part of a multi-part system that includes the system of the disclosure. Given the sensitivity of some biological agents, at least the system of the disclosure can be a closed system, where isolation of the biological agent and further processing thereof are conducted in a single controlled system.

In certain embodiments of the system, a container comprises one or more biological agent-capturing moieties for capture of desired matter, such as desired cells, for example. In particular aspects of the invention, the biological agent-capturing moieties of the system and methods comprise aptamers and/or produce aptamers, which aptamers may be oligonucleotides that bind to a specific target molecule (in this case, the desired biological agents to be captured). The aptamer sequence may or may not be known by the user or preparer of the system. The particular aptamer is specific for binding of particular desired cells, in particular embodiments. Aptamers may be generated or identified by the user of the system or the aptamer may be obtained from another, including commercially obtained. For example, oligonucleotide aptamers may be generated by selecting them from a large random synthetically-generated sequence pool, although the aptamers may exist in nature. The user or preparer of the system of the disclosure may not be the party that identifies the sequence of the aptamer specific for a particular biological agent. For nucleic acid embodiments, the aptamers may comprise DNA or RNA and may comprise multimers of oligonucleotides. In specific embodiments, the length range for the aptamer tentacle is at least 10, 25, 50, 75, 100, 200, 300, 400, 500, 600, 700, 800, 900, or 1000 or more aptamers within the tentacle. In particular embodiments, the length range for an aptamer tentacle is no more than 10, 25, 50, 75, 100, 200, 300, 400, 500, 600, 700, 800, 900, or 1000 or more aptamers within the tentacle.

In particular embodiments of the disclosure, the container is part of a closed system comprising at least one bag or tube comprised of fluoropolymer or coated in fluoropolymer such that its inner surface is functionalized with nucleic acid (such as DNA, including long sequences of DNA), and the nucleic acid may be attached indirectly to the inner surface. In specific embodiments, the fluoropolymer has attached thereto tentacles of long sequences of DNA that may be generated (for example, by RCA or PCR) of a first template that comports to the aptamer. Thereby, each tentacle of DNA has at least two or more sites highly specific to the biological agent of interest.

In one specific embodiment, a sample (such as blood, for example) is obtained from an individual and provided to the container that comprises particular polymers modified for capture of desired biological agents in the sample (such as desired cells). The polymers, in specific embodiments, are modified to have attached thereto nucleic acid aptamer tentacles that are capable of binding the cells. Upon binding of the desired cells to the tentacles, the tentacles with bound cells may be released from the polymers for collection. The release may be by any suitable means, but in specific embodiments it is through the use of heating, an endonuclease (such as a restriction enzyme), and/or complementary DNA for example. When one or more restriction enzymes are employed, the selection of the restriction enzyme may be tailored to the particular sequence of the DNA tentacle. Collection of the desired cells may be through a tube into a bag, for example.

In particular embodiments of the disclosure, there is a multi-step process for producing a system for isolation of cells. The skilled artisan recognizes that there are a variety of chemical methods for the immobilization of aptamers, including attachment to gold, covalent attachment to functionally modified surfaces, including useful parameters for linker design, as an example (Balamurugan *et al.,* 2008). However, in specific embodiments a multi-step process is contemplated herein. In one embodiment, one step is to select a particular starting FEP surface for subsequent immobilization of a protein, such as avidin, or one of its derivatives. In specific embodiments, a biological capture agent is linked directly to the surface. Modifications of surface energy and surface chemistry can be considered. Another step may be to attach avidin, or one of its derivatives, to the chosen surface of FEP, and such a step may comprise either physical adsorption or chemical conjugation, in specific embodiments. The starting functionalization of the FEP surface may be different depending on which method is chosen. Another step involves coupling biotinylated aptamers with the specific DNA sequence of a cell surface marker (such as a monocyte surface marker) to avidin or neutravidin. Finally, the desired cell is isolated from the sample (such as isolation of monocytes from the whole blood). In this step, cells will bind to aptamers by their specific cell surface markers and will be separated from other cells in a closed-system.

In particular embodiments, the system is enclosed and comprises at least one surface. The surface may or may not be contiguous throughout the system, such as throughout multiple containers in the system. In specific embodiments, the surface in different containers of the system is substantially the same, although in other embodiments the surface in different containers of the system is different. The inner surface of containers of the system may have one or more layers in at least part of the system, although in some cases the surface has one or more layers throughout the system. Different surfaces in the system may have different modifications.

In one embodiment, a first layer of a surface in the system may be considered a base layer, such as the inner surface of which the container is comprised. Such a layer may be comprised of a material that has low leachability, low extractability, is non-reactive to biological agents, such as cells, and so forth. The first layer may be the inner surface of the structure of a container of the system or the first layer may be a coating on the inner surface of the structure of a container of the system.

In one embodiment, a second layer of the surface in the system may comprise functional groups attached to the first layer. The functional groups may be chemically configured for linking the first layer with a layer that comprises a biological agent-capturing moiety or a layer that is associated with a biological agent- capturing moiety. Such functional groups may be of any kind, depending on the nature of one or more layers of the system. In certain cases, the functional groups comprise an acid for further modification. In specific embodiments, the functional group is a carboxy group, hydroxyl group, aldehyde group, carbonyl group, amine group, imine group, amide group, ester group, anhydride group, thiol group, disulfides group, phenol group, guanidine group, thioether group, indole group, imidazole group, aminoethyl amide group, alkyne group, alkene group, aziridine group, epoxy group, isonitrile group, isocyanide group, tetrazine group, a diazonium surface group, an alkyne group, an alkene group, an aziridine group, an epoxy group, an isonitrile group, an isocyanide group, a tetrazine group, alkyl group, an aminoethyl amide group, an ester group, a diazonium group, or a combination thereof. The second layer may also comprise a functional group attached to first member of a binding pair, such as an avidin species (including avidin, neutravidin, or streptavidin) or a biotin molecule.

In one embodiment, a third layer of the surface in the system may comprise a biological agent-capturing moiety. The biological agent-capturing moiety may be of any kind, but in specific embodiments the biological agent- capturing moiety is a direct or indirect cell-binding moiety. In specific embodiments, the biological agent- capturing moiety allows direct or indirect cell selectivity. The biological agent- capturing moiety may comprise a nucleic acid or may produce a nucleic acid, in specific embodiments. The biological agent-capturing moiety may comprise a single aptamer, an aptamer tentacle, or an antibody, for example. In specific embodiments, the biological agent- capturing moiety comprises an avidin protein (including avidin, neutravidin, or streptavidin) or a biotin molecule or is indirectly associated with an avidin protein or a biotin molecule. In cases where the second layer comprises one of either an avidin species or biotin, the third layer comprises the respective counterpart biotin or avidin species.

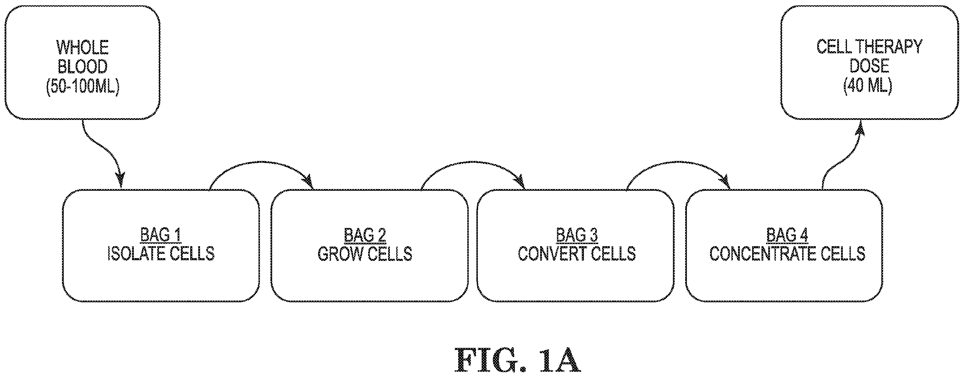

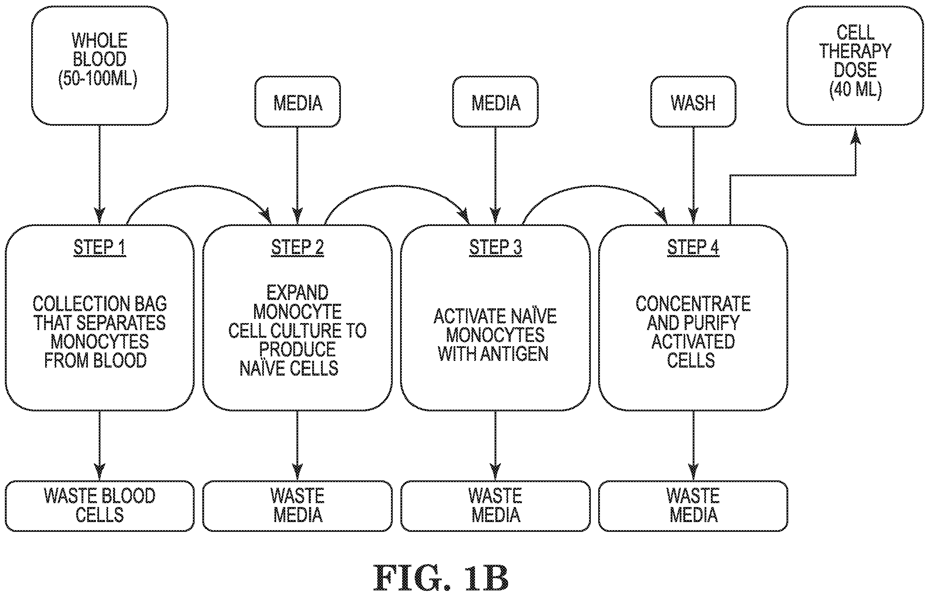

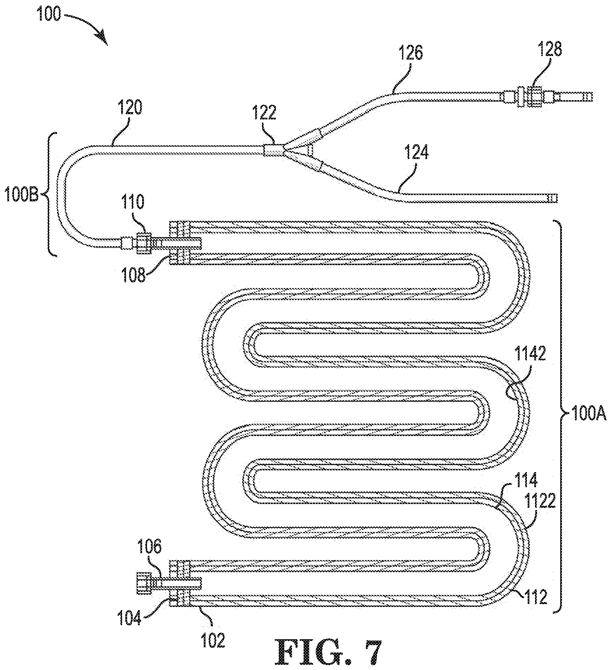

Referring to FIG. 7, a system for isolation of a biological agent 100 comprises an isolation unit 100a and a transfer unit 100b. The isolation unit 100a can include a serpentine bag 102 having an inlet port 104 and with an inlet 106 (the inlet may comprise a restriction device, such as a valve or clamp, for example) and an outlet port 108 with an outlet 110 (the outlet may comprise a restriction device, such as a valve or clamp, for example). The bag 102 may be configured as a tube, in some embodiments. As shown in FIG. 1, the isolation unit can be arranged in a serpentine fashion to save space and/or to facilitate temperature maintenance of the isolation unit, for example. In other embodiment, the isolation unit can be a straight tube or a helical tube, for example.

Still referring to FIG. 7, the isolation unit may comprise an outer material 112, although in other embodiments the unit lacks an outer material (for example, when the unit comprises 100% fluoropolymer). The outer material may be comprised of a thermoplastic polymer, a thermoplastic elastomer, a silicone, a rubber, or any combination thereof, in certain aspects. The outer material may have a thickness of at least 0.0005 inches, 0.0010 inches, 0.0050 inches, 0.0075 inches, 0.01 inches, 0.02 inches, 0.03 inches, 0.04 inches, 0.05 inches, at least 0.06 inches, at least 0.07 inches, at least 0.08 inches, at least 0.09 inches, at least 0.1 inches, or at least 0.11 inches. In another embodiment, the outer material may have a thickness of not greater than 0.2 inches, not greater than 0.18 inches, not greater than 0.16 inches, not greater than 0.14 inches, or not greater than 0.12 inches. In one embodiment the thickness of the outer material 112 may range from 0.06 inches to 0.13 inches, such as from 0.09 to 0.126 inches, in certain embodiments. The outer material 112 has an inner surface 1122.

As shown in FIG. 7, the inner surface 1122 of outer material 112 may be covered by a fluoropolymer material 114, as an example. The fluoropolymer material 114 comprises FEP. FEP is a convenient material, as it maintains good workability for cell isolation, including at least white blood cell isolation. For example, FEP does not trigger the innate immune response of a biological sample. Accordingly, in one embodiment, the fluoropolymer material 114 consists of or consists essentially of FEP.

In certain cases there is no outer surface to a tube and the wall is completely fluoropolymer. In any event, the fluoropolymer material 114 may have a thickness of at least 0.0003 inches, at least 0.0004 inches, at least 0.0005 inches, at least 0.0006 inches, at least 0.001 inches, at least 0.10 inches, and so forth, in certain embodiments. In another embodiment, the fluoropolymer material comprises a thickness of not greater than 0.100 inches, not greater than 0.080 inches, not greater than 0.070 inches, not greater than 0.050 inches, not greater than 0.030 inches, not greater than 0.018 inches, not greater than 0.016 inches, not greater than 0.014 inches, or not greater than 0.012 inches. In one embodiment the thickness of the fluoropolymer material 114 can range from 0.001 inches to 0.015 inches, such as from 0.002 to 0.01 inches. In specific embodiments the wall thickness may be up to and including 0.100 inches. The fluoropolymer material 114 may overlie the inner surface 1122, as shown in FIG.7. In particular embodiments, the fluoropolymer material 114 overlies at least the majority of the inner surface 1122. In one embodiment, the fluoropolymer material is in direct contact with outer material 112. The fluoroplymer may have a major surface 1142.

The major surface 1142 may be the exposed lumen of the tubing 102. The major surface 1142 of the fluoropolymer material 114 is modified. In specific embodiments, the fluoropolymer material 114 may be modified to include at least a reactive moiety. A reactive moiety can include a functional group that can be chemically modified to form a binding site for a biological agent-capturing moiety. In another embodiment, the reactive moiety can be a conjugate moiety. A conjugate moiety comprises a macromolecular complex of a protein and/or nucleotide (for example) including at least one binding site for a biological agent. For reactive moieties comprising more than one binding site, the reactive moiety may form a capturing moiety.

Further referring to FIG. 7, a transfer unit 100b allows for controlling processing of the biological sample after passage through the isolation unit 100a. The transfer unit 100b may include a tube 120 connected to outlet valve 110. The tube 120 may be made of thermoplastic polymer, thermoplastic elastomer, or silicone, for example. In one embodiment, the tube 120 may be lined with a fluoropolymer. The fluoropolymer may be the same as in the isolation unit 100a but with an unmodified major surface, *i.e.,* the fluoropolymer in a tube 120 does not include a reactive moiety, in particular embodiments.The fluoropolymer material comprises FEP.

The transfer unit in at least some cases may further include a splitter 122 for diverting the biological sample after passage through unit 100a from the system through tube 124. Conversely, tube 126 allows for connecting isolation unit 100a to another system through connector 128. In particular embodiments, a connection made to another system is a sterile connection that may comprise sterile welding of tubes or using a sterile connector.

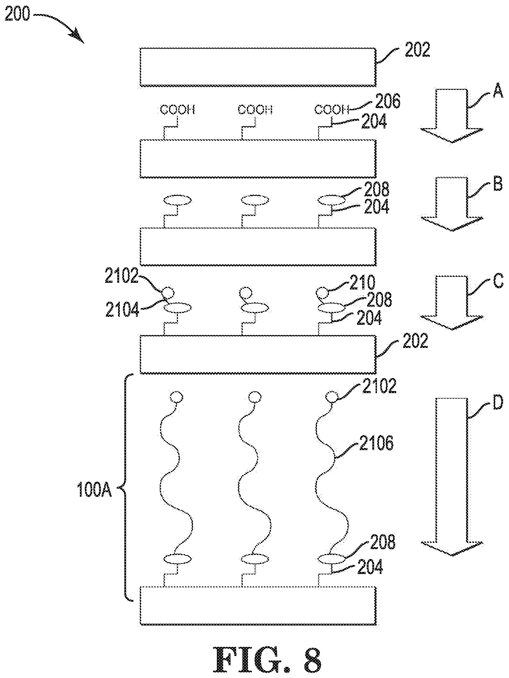

Referring to FIG. 8, this illustration depicts an exemplary process 200 to prepare the cell-capturing moiety with a functional group onto a fluoropolymer 202. In an initial step A, a reactive moiety comprising a linker 204 and a functional group 206 is prepared on a major surface of the fluoropolymer 202. As shown in FIG. 8, the functional group 206 is a carboxy group or an amine group ($-NH_2$).

The reactive moiety may also include a linker 204, in at least some cases. In one embodiment, the linker may be a covalent bond, connecting the carboxy group (or the other functional group) directly to the fluoropolymer. In another embodiment, the linker group may be an organic group. For example, the linker group may be linear alkylene group that includes a methylene group, an ethylene group, a propylene group, a butylene group, a pentylene group, a hexylene group, or any combination thereof, for example. In certain embodiments, the functional group (such as COOH) surfaces comprise a stable coating rather than a covalently bound group.

Surface modification of fluoropolymers provides a modified fluoropolymer useful in certain embodiments of the present invention. Generally, polar functionalities are attached to or are created in the fluoropolymer surface, rendering it easier to wet and provides opportunities for chemical bonding. There are several methods to functionalize a fluoropolymer surface including, for example, chemical etch, physical-mechanical etch, plasma etch, plasma activation at varied pressures, corona activation, chemical treatment, corona treatment, chemical vapor deposition, or any combination thereof. In an embodiment, the chemical etch includes sodium ammonia or sodium naphthalene. An exemplary physical-mechanical etch can include sandblasting and air abrasion with silica. In another embodiment, plasma etching includes reactive plasmas such as hydrogen, oxygen, acetylene, methane, and mixtures thereof with nitrogen, argon, and helium. Lachmann *et al.* (2011) describe a surface modification process with gas mixtures of helium and suitable reactive species or film-forming agents. Plasma activation can include formation of reactive species in the surface by treatment with gases including but not limiting argon, hydrogen, nitrogen, carbon dioxide, and combinations. The plasma activation could be achieved at low pressure such as 0.1 Torr to 0.6 Torr to or closed to atmospheric pressure such as 700 Torr to 760 Torr. Corona activation of the surface under gases including but not limiting argon, nitrogen and hydrogen or combination of them can be achieved to create active sites in the surface that could be further use in chemical treatments. Chemical treatment consist on sequential chemical modification of the active or existing surface by chemical reaction that includes grafting polymerization, coupling, click chemistry, condensation, and addition reactions. As example, grafting polymerization in solution can be achieved by polymerizing vinyl monomers via radical polymerization. Vinyl monomers included but not limited to acrylic acid,(metha) acrylates, (metha) alkyl acrylates, styrenes, dienes, alpha-olefines, halogenated alkenes, (meth)acrylonitriles, acrylamides, N-vinyl carbazoles and N-vinyl pyrrolidones, and maleic anhydride. Corona treatment can include the reactive hydrocarbon vapors such as ketones, e.g., acetone, alcohols, p-chlorostyrene, acrylonitrile, propylene diamine, anhydrous ammonia, styrene sulfonic acid, carbon tetrachloride, tetraethylene pentamine, cyclohexyl amine, tetra isopropyl titanate, decyl amine, tetrahydrofuran, diethylene triamine, tertiary butyl amine, ethylene diamine, toluene-2,4-diisocyanate, glycidyl methacrylate, triethylene tetramine, hexane, triethyl amine, methyl alcohol, vinyl acetate, methylisopropyl amine, vinyl butyl ether, methyl methacrylate, 2-vinyl pyrrolidone, methylvinylketone, xylene or mixtures thereof.

Some techniques use a combination of steps including one of these methods. For example, surface activation can be accomplished by plasma or corona in the presence of an excited gas species. For example surface activation can be accomplished by corona treatment in the presence of a solvent gas such as acetone. Another example includes the surface activation via plasma at low pressure or atmospheric pressure activation or corona activation in a gas such as argon that is further modified using a chemical treatment. The chemical treatment could be a grafting polymerization reaction of vinyl monomers including but not limiting acrylic acid, acrylates, (meth) acrylates, (meth) alkyl acrylates, styrenes, dienes, alpha-olefines, halogenated alkenes, (meth)acrylonitriles, acrylamides, N-vinyl carbazoles, and N-vinyl pyrrolidones, and maleic anhydride.

Not to be limited by theory, the method has been found to provide strong interlayer adhesion between a modified fluoropolymer and a non-fluoropolymer interface (or a second modified fluoropolymer). In one way, a fluoropolymer and a non-fluoropolymer shape are each formed separately. Subsequently, the fluoropolymer shape is surface treated by the treatment process described in U.S. Patents 3030290, 3255099, 3274089, 3274090, 3274091, 3275540, 3284331, 3291712, 3296011, 3391314, 3397132, 3485734, 3507763, 3676181, 4549921 and 6,726,979, the teachings of which are incorporated herein in their entirety for all purposes. Then, the resultant modified fluoropolymer and non-fluoropolymer shapes are contacted together for example by heat lamination to form a multilayer film. Finally, the multilayer film can be submitted to a UV radiation with wavelengths in the UVA; UVB and/or UVC range.

In one aspect, the surface of the fluoropolymer substrate is treated with a corona discharge where the electrode area was flooded with acetone, tetrahydrofuran methylethyl ketone, ethyl acetate, isopropyl acetate or propyl acetate vapors.

Corona discharge is produced by capacitative exchange of a gaseous medium which is present between two spaced electrodes, at least one of which is insulated from the gaseous medium by a dielectric barrier. Corona discharge is somewhat limited in origin to alternating currents because of its capacitative nature. It is a high voltage, low current phenomenon with voltages being typically measured in kilovolts and currents being typically measured in milliamperes. Corona discharges may be maintained over wide ranges of pressure and frequency. Pressures of from 0.2 to 10 atmospheres generally define the limits of corona discharge operation and atmospheric pressures generally are preferred. Frequencies ranging from 20 Hz to 100 MHz can conveniently be used: in particular ranges are from 500 Hz, especially 3000 Hz to 10 MHz.

When dielectric barriers are employed to insulate each of two spaced electrodes from the gaseous medium, the corona discharge phenomenon is frequently termed an electrodeless discharge, whereas when a single dielectric barrier is employed to insulate only one of the electrodes from the gaseous medium, the resulting corona discharge is frequently termed a semi-corona discharge. The term "corona discharge" is used throughout this specification to denote both types of corona discharge, *i.e.* both electrodeless discharge and semi-corona discharge.

All details concerning the corona discharge treatment procedure are provided in a series of U.S. Patents assigned to E. I. du Pont de Nemours and Company, USA, described in expired U.S. Patent No. 3,676,181, and Saint-Gobain Performance Plastics Corporation U.S. Patent No. 6,726,979, the teachings of which are incorporated herein in their entirety for all purposes. An example of the proposed technique may be found in U.S. Pat. No. 3,676,181 (Kowalski). The atmosphere for the enclosed treatment equipment is a 20% acetone (by volume) in nitrogen and is continuous. The outer layer of a constantly fed multilayer film or particulate filled film, for example, is subjected to between 0.15 and 2.5 Watt hrs per square foot of the film/sheet surface. The fluoropolymer can be treated on both sides of the film/shape to increase the adhesion. The material can then be placed on a non-siliconized release liner for storage. Materials that are C-treated last more than 1 year without significant loss of surface wettability, cementability and adhesion.

In another aspect, the surface of the fluoropolymer substrate is treated with a plasma. The phrase "plasma enhanced chemical vapor deposition" (PECVD) is known in the art and refers to a process that deposits thin films from a gas state (vapor) to a solid state on a substrate. There are some chemical reactions involved in the process, which occur after creation of a plasma of the reacting gases. The plasma is generally created by RF (AC) frequency or DC discharge between two electrodes where in between the substrate is placed and the space is filled with the reacting gases. A plasma is any gas in which a significant percentage of the atoms or molecules are ionized, resulting in reactive ions, electrons, radicals and UV radiation.

In specific embodiments, there may be utilization of a two-step process of a plasma surface activation of the FEP that leads to radicals/peroxides on the surface. The activated FEP surface is then exposed to wet chemistry methods to allow the surface to act as a free-radical polymerization initiator. For example, one can use acrylic acid monomers that create a dense surface of COOH groups, although other free-radical monomers could be used.

Further referring to FIG. 8, in step B, the functional group is linked to a macromolecular species 208, such as a protein. In one embodiment, such protein can include an avidin protein. In step C, a biotinylated species may be connected to the avidin. While the biotinylated species may include specific ligands to the desired biological agents, such as antibodies, the biotinylated species may also include solely a biotinylated nucleotide primer as shown in FIG. 7, element 2104. In one embodiment, the nucleotide template can include or be utilized with a polymerase enzyme 2102. In order to form the ligand, polymerase technology (for example) can be applied to form a ligand-specific DNA sequence 2106 in step D of the process. Ligand specific DNAs include aptamers, in at least some cases.

In continued reference to FIG. 8, the forgoing description addressing the preparation of an aptamer is exemplified in step D where a DNA tentacle 2106 is generated using polymerase enzyme 2102. A DNA comprising the antisense sequence of the at least one aptamer and the antisense sequence of a non-binding DNA backbone that acts as a spacer between aptamer sequences is provided, and in some cases the DNA is circular. In specific embodiments, the circular DNA is single stranded and a primer is provided, such as a biotinylated primer. In another embodiment, the spaced DNA sequence may include some additional function. For example, the spacer may include a fluorescent moiety for the purpose of determining the amount of biological agents bound onto to the reaction moiety 212.

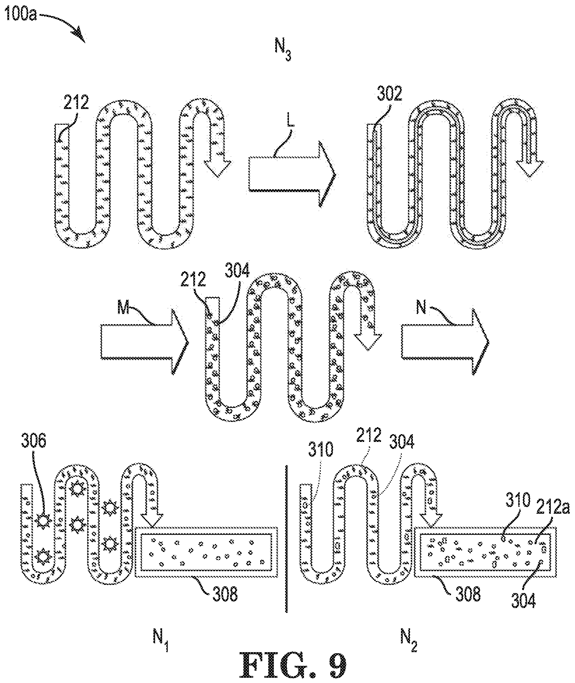

FIG. 9 depicts a capturing process employing the isolation unit 100a in a tubing with an immobilized reactive moiety 212. An example of a tubing includes the aforementioned reactive moiety equipped with a desired cell-specific moiety, such as an aptamer. In step L, a sample (such as whole blood) 302 is passed through the tube 100a. During passage, desired cells 304 bind specifically on units 212, while the remainder of the sample exits the tubing. In step M, the sample has passed the tubing and the tubing has been rinsed with a cell free buffer to remove non-specific material from the tubing and to wash the immobilized desired cells. In step N, the cells are then dislodged from the units 212. In one embodiment, $N_1$, the tube can be heated by heating unit 306 until the association energy between the cell and the conjugation moiety *i.e.* the DNA tentacle, is overcome. Care is taken not to heat the cells to damaging levels. During and after the heating, a medium may be passed through the tubing to receive the cells and transport them to container 308.

In another embodiment, $N_2$, the contents of the tube can be treated with restriction enzymes or DNAases 310 which destroy the DNA sequence of unit 212, thereby freeing the desired cells 304. During the digestion, a medium is passed through the tubing to receive the desired cells and transport them and the enzymes to container 308. The enzymes 310 can be removed through simple filtration. In another embodiment, the cells are displaced from their attachment on DNA by adding DNA segments complementary to the segment attached to the cells.

Note that not all of the activities described above in the general description or the examples are required, that a portion of a specific activity may not be required, and that one or more further activities may be performed in addition to those described. Still further, the order in which activities are listed are not necessarily the order in which they are performed.

## I. Cells for Isolation and Processing Thereof

In embodiments of the disclosure, any cell may be isolated by the system described herein. Cells for isolation include cells such as immune cells, blood cells, or stem cells (including embryonic stem cells, adult stem cells, or induced pluripotent stem cells). Blood cells that may be isolated with the system described herein include red blood cells, white blood cells, or platelets. Examples of white blood cells include granulocytes (such as neutrophils, basophils, or eosinophils) or agranulocytes (such as lymphocytes, monocytes, or macrophages). Examples of immune cells that may be isolated include phagocytes (macrophages and neutrophils); B cells; T cells (including helper T cells and cytotoxic T cells); monocytes; dendritic cells; natural killer cells; regulatory T cells; and so forth.

Monocytes, in particular embodiments, are isolated in the systems of the disclosure. Monocytes are pluripotent cells, which means they can differentiate into a variety of kinds of cells. The main types of cells that monocytes can differentiate into are macrophages and dendritic cells. Dendritic cells have a key role in the design of a specific answer of the immune system against a pathogen. Indeed, the antigen presentation by the dendritic cells is a vital step to stimulate the T cells.

## II. Total Organic Carbon

Total Organic Carbon (TOC) is the amount of carbon bound in an organic compound and is often used as a non-specific indicator of pharmaceutical manufacturing equipment, among other things. TOC is utilized as a process control attribute in the biotechnology industry to monitor the performance of unit operations that employ purification and distribution systems

In specific embodiments, TOC may be measured according to US Pharmacopeia (USP) 643 and with equipment that utilizes a high temperature wet oxidation reaction of UV-promoted chemical oxidation (Ultra-Clean Technology Handbook: Volume 1: Ultra-Pure Water, Ohmi, Tadahiro; CRC Press, 1993, pp. 497-517). Purified water is placed in contact with the polymer for 24 hours at 70°C, for example at a ratio of 3 $cm^2$ of article surface area to 1 mL of water. The water is removed from contact with the polymer and tested in a TOC analyzer. A suitable piece of equipment is a TEKMAR DOHRMANN Model Phoenix 8000 TOC analyzer.

In particular embodiments, TOC may be measured for a container employed in a system of the disclosure including, for example by extraction from an internal surface area of the container (with results reflected as $mg/cm^2$ are for the TOC per square centimeter of the internal area). In specific embodiments, and merely as an example, the container may be extracted in purified water $70\pm2$ °C for $24\pm2$ hours. The extract may be analyzed for TOC by converting TOC to carbon dioxide by acidification and chemical wet oxidation with sodium persulfate, for example. The carbon dioxide liberated from the container may be measured using an infrared detector. An example of an extraction ratio for a FEP container is 3 $cm^2$/mL (a typical extraction ratio of 1 mL of water per 3 $cm^2$ of internal wetted surface area of the container). For some containers (such as FEP bags), no dilution is required because the level of TOC is less than the upper limit of a cal curve, whereas for other embodiments (such as silicone tubing), dilution is required because of the levels of the TOC detected in the extract.

An example of TOC for a FEP container is 0.145 mg/L (0.00005mg/$cm^2$ or 0.001 mg/g). For embodiments that employ silicone tubing, extraction ratios may be 14.6 $cm^2$/mL (such as for Biosil) or may be 15.9 $cm^2$/mL (such as for SR139), and an example of TOC for silicone Biosil tube is 302 mg/L (0.021 mg/$cm^2$ or 0.023 mg/cm), and an example of TOC for silicone SR139 tubing is 120 mg/L (0.008 mg/$cm^2$ or 0.0009 mg/cm). In at least certain silicone tubing embodiments, the samples may be diluted, as the volume and concentration of the extraction cause the value to be above the maximum detection of the machine. The dilution and different extraction ratio requires the comparison of these samples with the bag samples to be made on the weight/area value basis instead.

One of skill in the art recognizes that TOC values may be characterized in weight/volume. However, persons of skill in the art acknowledge that ratios for the container (particularly a FEP bag material) vs. ratios for silicone tubing are distinguishable; silicone tubing values can only be considered on a mg/$cm^2$ starting basis, as this value is independent of extraction ratio/dilution. One of skill in the art can calculate a "normalized" weight/volume ratio using a weight/area result as a basis and assuming a standard 3$cm^2$/mL extraction ratio (as an example) in order to compare values on a weight/volume value.

In specific embodiments, the TOC of thermoplastic elastomers (TPE) is 0.002 mg/$cm^2$ (0.032 mg/g or 5.88 mg/mL). In certain embodiments, the TOC of FEP is 0.00005 mg/$cm^2$ of interior wetted surface of an article (0.001 mg/g or 0.145 mg/mL of article). In specific embodiments, the TOC of silicone of interior wetted surface of an article is 0.021 mg/$cm^2$ or 63 mg/mL of interior wetted surface of an article.

In some cases, the inner wall of the container is comprised of polymer of a total organic carbon (TOC) in water of less than 0.1mg/$cm^2$, 0.09 mg/$cm^2$, 0.08 mg/$cm^2$, 0.07 mg/$cm^2$, 0.06 mg/$cm^2$, 0.05 mg/$cm^2$, 0.04 mg/$cm^2$, 0.03 mg/$cm^2$, 0.02 mg/$cm^2$, 0.01 mg/$cm^2$, 0.009 mg/$cm^2$, 0.008 mg/$cm^2$, 0.007 mg/$cm^2$, 0.006 mg/$cm^2$, 0.005 mg/$cm^2$, 0.004 mg/$cm^2$, 0.003 mg/$cm^2$, 0.002 mg/$cm^2$, 0.001 mg/$cm^2$, or is nondetectable. In particular embodiments, the TOC in water is less than an amount in a range from 0.001 mg/$cm^2$ to 0.1mg/$cm^2$, 0.001 mg/$cm^2$ to 0.095 mg/$cm^2$, 0.001 mg/$cm^2$ to 0.075 mg/$cm^2$, 0.001 mg/$cm^2$ to 0.05 mg/$cm^2$, 0.001 mg/$cm^2$ to 0.01 mg/$cm^2$, 0.001 mg/$cm^2$ to 0.005 mg/$cm^2$, or 0.001 mg/$cm^2$ to 0.025 mg/$cm^2$. In particular embodiments, the TOC in water is less than an amount in a range from 0.01 mg/$cm^2$ to 0.1 mg/$cm^2$, 0.01 mg/$cm^2$ to 0.075 mg/$cm^2$, 0.01 mg/$cm^2$ to 0.05 mg/$cm^2$, or 0.01 mg/$cm^2$ to 0.025 mg/$cm^2$. In particular embodiments, the TOC in water is less than an amount in a range from 0.05 mg/$cm^2$ to 0.1 mg/$cm^2$, 0.05 mg/$cm^2$ to 0.09 mg/$cm^2$, 0.05 mg/$cm^2$ to 0.075 mg/$cm^2$, or 0.05mg/$cm^2$ to 0.06 mg/$cm^2$ In particular embodiments, the TOC in water is less than an amount in a range from 0.005 mg/$cm^2$ to 0.1 mg/$cm^2$, 0.005 mg/$cm^2$ to 0.095 mg/$cm^2$, 0.005 mg/$cm^2$ to 0.075 mg/$cm^2$, 0.005 mg/$cm^2$ to 0.05 mg/$cm^2$, 0.005 mg/$cm^2$ to 0.025 mg/$cm^2$, or 0.005 mg/$cm^2$ to 0.01 mg/$cm^2$.

In specific embodiments, the TOC of fluorinated ethylene propylene (FEP) is 0.00005 mg/$cm^2$ (0.001 mg/g); the TOC of silicone materials, such as silicone tubing, is 0.021 mg/$cm^2$ (0.023 mg/cm) and 0.008 mg/$cm^2$ (0.009 mg/cm) of interior wetted surface of an article; the TOC for a historically used cell culture bag is 0.002 mg/$cm^2$ of interior wetted surface of an article (0.032 mg/g of article).

One of skill in the art recognizes that TOC values may be compared across different extraction ratios/dilutions if mg/$cm^2$ units are employed. If units are mg/L, an extraction ratio must be known. A conversion may occur as follows: the machine outputs a value in mg/L, dilution is factored in, and then this number is converted to mg/$cm^2$ using the

surface area and total volume to extract. An example for Silicone Biosil is provided:

**[0104]** Silicone Biosil Sample: 302 mg/L * 1L/1000mL * 23.7 mL/347 cm$^2$ = .021 mg/cm$^2$

In a particular embodiments, TOC is compared in mg/cm$^2$ units because the extraction ratio or any dilution is not needed.

Below is an example of TOC calculation on a Silicone Tube Biosil sample and on Silicone Tube SR139 sample.

Test Article Extraction

| Sample | Internal Surface Area (cm$^2$) | Length (cm) | Volume of Purified Water (mL) |
|---|---|---|---|
| Silicone Tube Biosil Sample | 347 | 314 | 23.7 |
| Silicone Tube SR139 Sample | 342 | 295 | 21.5 |

Results for TOC Analysis

| Sample | mg/L | mg/cm$^2$ | mg/cm | Detection Limit (mg/L) |
|---|---|---|---|---|
| Silicone Tube Biosil Sample | 302 | 0.021 | 0.023 | 0.1 |
| Silicone Tube SR139 Sample | 120 | 0.008 | 0.009 | 0.1 |

Below is an example of TOC calculation on a FEP Bag and on a Baxter Bag (a single layer bag mostly composed of a SEBS (styrene block copolymer) but also may contain EVA and PP):

Test Article Extraction

| Sample | Internal Surface Area (cm$^2$) | Weight (g) | Volume of Purified Water (mL) |
|---|---|---|---|
| FEP Bag | 650 | 30.9 | 217 |
| Baxter Bag | 362.9 | 22.5 | 121 |

Results for TOC Analysis

| Sample | mg/L | mg/cm$^2$ | mg/g | Detection Limit (mg/L) |
|---|---|---|---|---|
| FEP Bag | 0.145 | 0.00005 | 0.001 | 0.1 |
| Baxter Bag | 5.88 | 0.002 | 0.032 | 0.1 |

In a specific embodiment, a TOC for a PMP film is 0.07 ppm (0.00002 mg/cm$^2$).

In specific embodiments, a container comprises an inner surface comprising a polymer having a total organic carbon (TOC) in water of less than 1 mg/cm$^2$, 0. 1mg/cm$^2$, 0.09 mg/cm$^2$, 0.08 mg/cm$^2$, 0.07 mg/cm$^2$, 0.06 mg/cm$^2$, 0.05 mg/cm$^2$, 0.04 mg/cm$^2$, 0.03 mg/cm$^2$, 0.02 mg/cm$^2$, 0.01 mg/cm$^2$, 0.009 mg/cm$^2$, 0.008 mg/cm$^2$, 0.007 mg/cm$^2$, 0.006 mg/cm$^2$, 0.005 mg/cm$^2$, 0.004 mg/cm$^2$, 0.003 mg/cm$^2$, 0.002 mg/cm$^2$, 0.001 mg/cm$^2$, and so forth.

## III. Production of Aptamers and Aptamer Tentacles

Embodiments of the disclosure allow one to isolate one kind of cells form a blood sample that contains hundreds of different kinds of cells. Such a process requires a very specific tool that would be specific to the desired cells (such as monocytes) and will not recognize any other cells (including other blood cells). Antibodies that can have such specificities have a number of disadvantages, including complicated procedures, requiring *in vivo* synthesis, and they can generate shear stress and steric effect on the cell, potentially altering its functionality; furthermore, antibodies only provide one point of attachment to the cell.

Aptamers are oligonucleic acid molecules that bind to a specific target molecule. They may be single stranded DNA or RNA oligonucleotides that can bind to small molecules or macromolecules of nearly all classes with high specificity and affinity and low toxicity. Aptamers are usually created by selection from a large random sequence pool, such as through a process known as SELEX: which stands for the *Systematic Evolution of Ligands by Exponential Enrichment.* They have an unmatched specificity to their target, as their small size and 3D conformation make them more specific to a target than an antibody. For example, aptamers can even differentiate enantiomers and protein isoforms. A cell-type-specific SELEX process that produces the aptamers may be of any kind, particularly live cell-based SELEX (see, for example, Ye *et al.* (2012); Ozer *et al.* (2014); Sun *et al.* (2014); and Zhou and Rossi (2014).

In particular embodiments, a particular aptamer sequence for isolating a desired cell type is applicable for isolating the same cell type from any individual from the same species or genus or family of organisms. In other embodiments, a particular aptamer sequence for isolating a desired cell type is specific for a particular individual only.

In particular embodiments, more than one aptamer sequence is identified that is useful for isolating a desired cell type, and the multiple aptamer sequences are employed in the systems of the disclosure.

In some cases, the aptamers have a moiety attached thereto, wherein the moiety may be detectable and/or may be able to bind to another moiety. The moiety may be one member of a binding pair, such as biotin or an avidin species. Methods of attaching biotin or avidin to DNA are known in the art.

## A. Production of Aptamers

In an embodiment of the disclosure, aptamers are designed in order to separate desired cells from a sample, including, for example, monocytes from whole blood samples. Although the aptamers may be designed in a variety of ways, in specific embodiments a SELEX process is utilized.

SELEX is an *in vitro* process that can target any small molecule, biopolymer, or cell. Once the sequence of the aptamer is known, one can synthesize it, such as *in vitro.* Aptamers can be rapidly produced in high quantities and are very stable: their shelf-life is unlimited. Furthermore, one can add to them a backbone that makes much easier their binding to a surface. Aptamers are already used in the biopharmaceutical industry and have not shown evidence of immunogenicity.

In specific embodiments, a Cell-SELEX process is utilized to generate aptamers for the systems of the disclosure. An aptamer is essentially a portion of DNA, and it can be multimerized. It is a sequence of nucleotides, a polymer made of a unique combination of four different units: A, T, C, G. Once the code of an aptamer specific to a target is known, it is rather easy to synthetize it. The Cell-SELEX process is adapted from the SELEX process to generate an aptamer highly specific to one kind of cell. In this process, a single-stranded DNA (ssDNA) library pool is incubated with the target cells. Nonbinding sequences are washed off, and bound sequences are recovered from the cells, such as by heating cell-DNA complexes at 95 °C, followed by centrifugation. The recovered pool is incubated with the control cell line to filter out the sequences that bind to common molecules on both the target and the control, leading to the enrichment of specific binders to the target. Binding sequences are amplified, such as by Polymerase Chain Reaction (well known as PCR). This is followed by removal of antisense strands to generate an ssDNA pool for subsequent rounds of selection. The enrichment of the selected pools may be monitored by flow cytometry binding assays, with selected pools having increased fluorescence compared with the unselected DNA library.

Once one or more aptamers are identified, one can functionalize a surface with it to isolate the desired cells, such as a bag or tube. Alternative techniques that rely on aptamers to separate cells can be used, such as affinity chromatography, magnetic, plastic, or glass beads functionalized with aptamers, or hydrogels containing aptamers, or plates containing aptamers, so long as these techniques are employed in a closed system, for example. In particular embodiments, the structures may comprise or otherwise have a layer of fluoropolymer.

## B. Production of Aptamer Tentacles

In particular embodiments, more than one aptamer per molecule in the system is used to maximize the binding of the desired cells to the container of the system (although in alternative cases, only one aptamer is employed in a tentacle). In particular embodiments, two or more aptamers of the same type are utilized repeatedly in a single tentacle molecule. In such cases, not only one aptamer is bonded to the surface, but a long strand of DNA having at least several times the sequence of the aptamer. The aptamer tentacle is analogous to an octopus tentacle, with a plurality of "suckers" very specific to the desired cells.

Utilization of these tentacles with the cell-specific aptamers is useful to capture gently the desired cells. Indeed, the capture by these tentacles is not harmful, because several tentacles are able to capture one cell, resulting in low shear stress yet high efficiency for the capture.

To release the cells from the tentacle, one or more steps may be taken. Examples of ways to release the cells includes the use of restriction enzymes to cut the DNA, the use of nucleic acid (DNA or RNA) to displace the cells, and/or heating. In certain embodiments when heating is used, the heating may be for about 10 seconds to about five minutes duration of time at a temperature range of 45-50°C, for example.

The strategy of tentacles presents many advantages: one-step separation from the sample; single-use; low shear stress, so no or minimal harming of the cells; high specificity; and improved rates of capture compared to antibodies or single aptamers.

IV. **Exemplary Materials for the System and Preparation of the System**

**A. General Aspects**

Materials for use in the surfaces of the system include polymers that have a total organic carbon (TOC) in water of less than 0. 1mg/cm$^2$. Fluoropolymers are useful in embodiments of the system because they are inert, contain low extractables, have low leachability, have sufficient $O_2$ and $CO_2$ gas permeability, have low water permeability, are flexible, and are strong.

In particular embodiments of the disclosure, different steps for the system occur in separate containers. In specific embodiments, the containers are closed entities except for one or more inlet/outlets. In specific aspects the system in its entirety is a closed entity except for one or more inlet/outlets. The containers may be of any kind, but in specific embodiments the containers are a bag, tube, bead (such as the bead being encapsulated in a closed container ), flask, roller bottle, or rigid container. In particular embodiments, one or more of the containers in the system are modified to trap desired cells from a sample.

In certain embodiments, each step of the system occurs in a separate container, although in alternative embodiments one or more steps occur in the same container. In exemplary cases, a first container is utilized for isolation of cells, a second container is utilized for growth of cells (in some embodiments, such as expansion of monocytes to produce naive cells), a third container is utilized for conversion of cells (which may be considered activation of cells including, for example, monocyte activation with an antigen), and a fourth container is utilized for concentration of cells (and, at least in some cases purification of activated cells) (FIGS. 1A-1B).

In specific embodiments, the first container allows separation of desired cells from a sample. Although the cells from a sample may be of any kind of cells from any kind of sample, in specific embodiments the cells are desired cells (such as monocytes) from a blood sample, including an untreated blood sample.

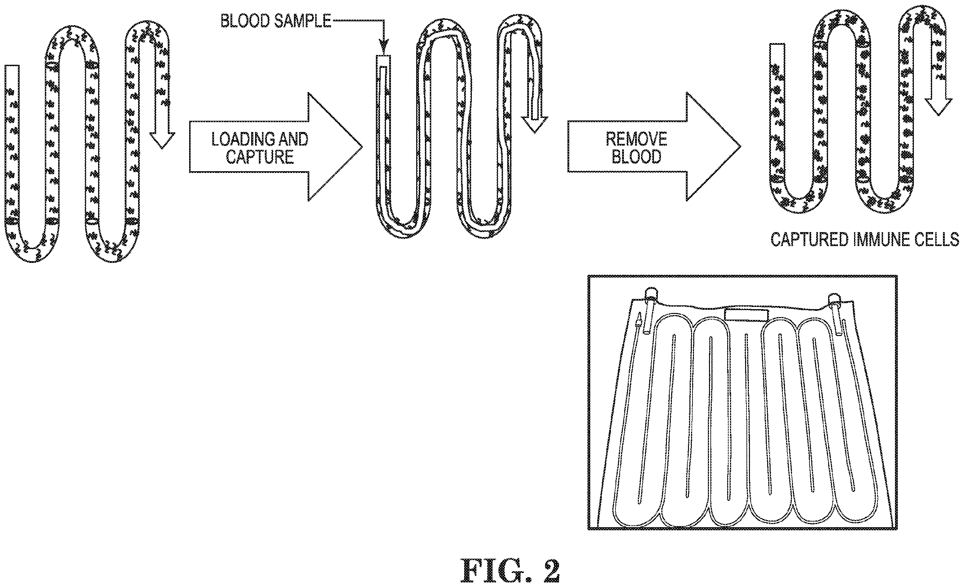

Embodiments of the disclosure include functionalization of a container (such as a bag) with DNA-based aptamer tentacles to select cells (for example, immune cells) from a sample (such as blood). The aptamers physically interact with specific structures of the cell surface and capture the cells (FIG. 2 or 8). In some cases, the specific structures of the cell surface that interact with the aptamers are known by the user of the system, whereas in other cases the structures are not known. This process can be realized in a physical bag, such as is pictured in FIG. 2.

In a particular example, the area needed in the container to allow sufficient exposure for a blood sample is 380 cm$^2$ for 94mL of blood.

FIG. 9 illustrates release of cells once they have been trapped by the aptamer-comprising container. The cells may be released by any appropriate method, but in specific embodiments the container is heated sufficiently such that the aptamer separates (which may be referred to as melts) from the bound cell. The cells may then be harvested or further processed. Other means for releasing the cells is to digest the nucleic acid aptamer with an enzyme (and the resultant mixture of released cells and enzymes may or may not be further processed to remove the enzyme proteins), such as a restriction enzyme. An additional method for releasing the trapped cells includes displacing the cell from the aptamer with complementary DNA to the aptamer (part or all of the aptamer sequence).

An example of a specific process of the disclosure related to isolating monocytes focuses on the following:

1) harvesting monocytes from an individual;

2) culturing cells *ex vivo* and differentiating them into naive dendritic cells

3) maturing the naive dendritic cells by exposing them to a specific antigen; and

4) injecting a therapeutically effective amount of activated, mature dendritic cells back into the individual and allow for the cell therapy to activate the immune system and fight the target antigen (such as a cancer cell expressing the antigen). Therefore, the dendritic cells are put back into an individual's body to make it fight the medical condition on his own.

Thus, in some cases, monocytes are captured with systems of the disclosure. Monocytes are very sensitive cells, which can be activated upon contact with almost any surface. Fluoropolymer surfaces do not activate monocytes, and therefore a system that uses fluoropolymer materials could avoid this activation. Additionally, there is a need to expand the cells in a closed-system, requiring a continuous input of $O_2$ and export of $CO_2$ while remaining impermeable to water These are all characteristics of FEP, a fluoropolymer with a good permeability to oxygen and carbon dioxide, and a low permeability to water. Additionally, for being able to transport and to preserve the cells, a system is needed that has the capability to withstand very low temperatures (*i.e.* cryogenic storage). FEP is a material that continues to function very well at low temperatures. All of this together makes a good case for FEP being a useful choice for the system material.

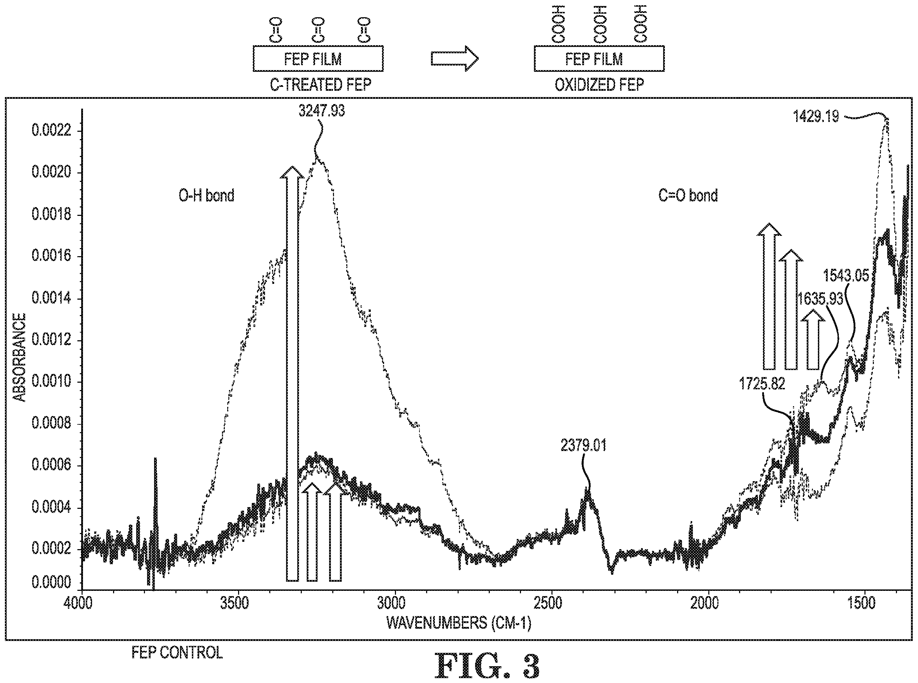

FIG. 8 illustrates exemplary embodiments of steps for producing the systems, including grafting of aptamer chains to the surface of the container. In exemplary cases, step 1 encompasses functionalization of a surface of a substrate (such as a fluoropolymer, including a fluorinated ethylene propylene (FEP) film with carboxylic acids. The next step includes covalent linking of a protein to the carboxylic acid *via* a peptide bond; in specific embodiments, the protein has a ligand that binds to it such that further processing will allow binding of another entity to the protein. In specific cases, the protein is avidin such that biotin that is bound to another entity can bind indirectly to the functionalized surface. FIG. 8 allows coupling of the protein to the ligand, such as coupling of avidin to a biotinylated circular template for an aptamer. The final step includes rolling circle amplification of the template to produce aptamer tentacles. FIG. 3 demonstrates production of an example of a functionalized substrate, wherein Saint-Gobain® Norton C-treated FEP film is converted to oxidized FEP.

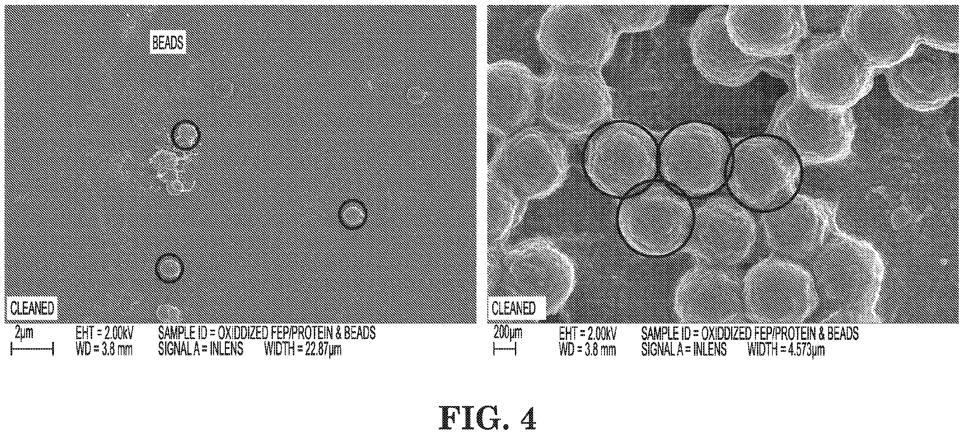

In alternative embodiments, biotinylated beads are attached to an FEP substrate for isolation of cells of interest (FIG. 4). Aptamers may be generated on the surface of the bead by rolling circle amplification. In particular embodiments, biotinylated beads are utilized with aptamers that have avidin attached thereto. In specific embodiments, beads that are coated with avidin can capture biotinylated apatamer/antibodies. In particular embodiments, beads may or may not be attached to FEP. In certain embodiments, the beads are comprised of FEP and provide an alternative way to separate out desired cells from a sample. For example, avidin-coated FEP beads (or another material) may be exposed to biotinylated aptamers to result in the FEP beads to be coated with aptamers, and then these are exposed to a sample, such as a blood sample. Monocytes, for example, would attach to such beads, and then one could flow these through a particular filter/membrane that would keep the beads with the cells of interest attached thereto separate from the rest of the blood. Then, one could use disassociation methods (such as heating, enzymes, *etc.*) to remove the cells from the beads.

In specific embodiments, biotin is added to the end of the aptamers. Biotin is a vitamin that is often used to link bioproteins to a surface. Biotin has a strong affinity with neutravidin, so neutravidin may be linked to the surface of the substrate, and then a template of biotinylated aptamer is thereby linked indirectly to the surface. In particular aspects, neutravidin (or avidin) is linked with a sufficiently high density on the surface, and in specific embodiments the FEP surface is functionalized with COOH. In specific embodiments, peptide bonds between the amino groups of a protein such as neutravidin and of carboxylic acids on a surface are utilized to link neutravidin to the surface.

In specific embodiments, the environment of the system is conducive to maintaining the integrity of any cells isolated by systems of the disclosure. The system is configured to allow sufficient exposure of the cells to oxygen, water, cytokines, and/or glucose. The system is also configured to prevent exposure of the cells to deleterious levels of harmful substances, such as carbon dioxide, carbonic acid, and/or lactic acid.

### B. Substrate

At least some system embodiments include multiple layers. In a particular embodiment, the first layer is comprised of a material that has low leachability, low extractability, and does not react deleteriously with cells. The first layer may be considered a film or membrane. In certain embodiments, the layer comprises a polymer. In specific embodiments, the polymer comprising the wetted surface of the container has a total organic carbon (TOC) in water of less than 0. 1mg/cm$^2$ and may be measured on the surface that will comprise the wetted surface of the container. In some embodiments, the polymer is comprised of Saint-Gobain® Norton C-treated FEP film. The fluoropolymer is fluoro ethylene propylene..

In particular embodiments, the first layer comprises a particular thickness. In certain aspects, the minimum thickness of the layer is 0.0003 inches. In at least some cases, the maximum thickness of the layer is 0.010 inches.

## C. Functionalization of a Substrate

Embodiments of the disclosure allow for functionalization of an appropriate substrate so that it is useful for direct or indirect attachment of an aptamer. In specific embodiments, the surface does not need functionalization because the aptamer is directly attached to the surface. For example, an avidin/biotin embodiment of the system may not be utilized, and in certain embodiments there is direct attachment of the biological moiety to the COOH (as an example) surface of the fluoropolymer. In cases wherein an aptamer is directly attached to the surface, one can conjugate a DNA aptamer, for example, directly to a modified surface using similar pathways of an NH2-containing protein in cases wherein the aptamer is functionalized with a NH2 end (such as by glutaraldehyde linker or maleimide linker; see, for example Ponche et al., 2012). In such an embodiment one can avoid using an avidin layer and attach an aptamer directly to a modified surface. In specific embodiments, a DNA aptamer is immobilized to a surface via a specific end group, such as an amino group, aldehyde group, or epoxy group, for example (see Oh et al., 2006). For example, when starting with an aldehyde-functionalized surface, one can immobilize a DNA aptamer using Schiff base reactions (McGettrick et al., 2009). Other functional DNA end groups can include amino, biotin, azide, thiol, dithiol, digoxigenin, NHS ester, octadiynyl, a carboxy group, hydroxyl group, aldehyde group, carbonyl group, amine group, imine group, amide group, ester group, anhydride group, thiol group, disulfides, group, phenols group, guanidines group, thioether groups, indoles group, imidazoles group, aminoethyl amide group, alkyne group, alkene group, aziridine group, epoxy group, isonitrile group, isocyanide group, tetrazine group, dor a diazonium surface groups., an alkyne group, an alkene group, an aziridine group, an epoxy group, an isonitrile group, an isocyanide group, a tetrazine group, alkyl group, an aminoethyl amide group, an ester group, and/or a diazonium group.

In embodiments wherein a surface needs to be functionalized, there are at least four general immobilization techniques that may be employed: 1) physical adsorption; 2) electrochemical activation; 3) electrochemical grafting; and 4) avidin-biotin affinity (reviewed in the context of graphite composite electrodes by Ocana and del Valle, 2013). In particular embodiments wherein a substrate needs to be functionalized, there are a variety of well-known reaction pathways given a specific starting surface. Examples of starting surfaces include carboxy groups, hydroxyl groups, aldehyde groups, carbonyl groups, amine groups, imine groups, amide groups, ester groups, anhydride groups, thiol groups, disulfides groups, phenol groups, guanidine groups, thioether groups, indole groups, imidazole groups, aminoethyl amide groups, alkyne groups, alkene groups, aziridine groups, epoxy groups, isonitrile groups, isocyanide groups, tetrazine groups, a diazonium surface group, an alkyne group, an alkene group, an aziridine group, an epoxy group, an isonitrile group, an isocyanide group, a tetrazine group, alkyl group, an aminoethyl amide group, an ester group, a diazonium group, or a combination thereof. A selected functionalization will dictate the reaction(s) that will require different reagents and different immobilization chemistries (for example, Schiff base to attach to an aldehyde vs. EDC/NHS to attach to a carboxylic acid). In specific embodiments, a reaction is employed wherein a bond is formed with an NH2 group on a protein. As an example, fluoropolymers can achieve such different functionalities in a variety of ways: plasma treatments, chemical modification, grafting, and so forth.

One can benefit from the advantages of using FEP as a substrate, which has a surface non-reactive to monocytes, for capture of the cells with aptamers. Therefore, in particular embodiments there is functionalization of a FEP surface with a biomolecule. FIG. 8 shows exemplary steps for producing tentacles on the surface of a FEP substrate (such as a film). The four exemplary steps describe how one can functionalize a container. The fourth step may occur using an enzyme known as Phi 29 DNA Polymerase. Rolling Circle Amplification (RCA) develops hundreds to thousands of copies of the template strand and is a process well known in the bio industry. The template is a circle composed of two parts: one is the code of the anti-aptamer and the other one is not coding. When the enzyme will develop the tentacles, it will gather the nucleobases complementary to the code of the template and make copies of it. Thus, the tentacle will comprise the sequence of the aptamer and of a non-coding part (also called spacer); it is an alternating copolymer (A-B-A-B-A-B...), in particular aspects. The final length of the tentacles may be controlled by the time of reaction. In particular embodiments, the length of the aptamer tentacle is between nanometers and microns, including hundreds of nanometers to hundreds of microns. The length or number of aptamer repeats in a particular tentacle may vary in relation to another tentacle in the system. In some cases, the number of aptamer repeats is 2 or more, ten or more, tens of repeats or more, hundreds of repeats or more, thousands of repeats or more, tens of thousands of repeats or more, and so forth.

Carboxylic acids are generated on the surface of the FEP substrate. In specific embodiments, any chemical reaction that can provide COOH groups on the surface of a substrate may be employed (see, for example, Tong and Shoichet, 1998). In specific embodiments, the treatment used is plasma, including, for example, a modified plasma treatment for fluoropolymers: the C-treatment. The C-treatment adds the presence of polar groups on the surface of the FEP bags, ultimately allowing for cells to adhere.

In embodiments wherein a C-treated film is employed, and in order to use the C-treated film as a basis to start the development of a COOH functionalized FEP surface, the surface was characterized. In a combination of X-ray Photoelectron Spectroscopy (XPS Analysis), Fourier Transform Infrared Spectroscopy, Scanning Electron Microscopy, Time-of-Flight Secondary Ion Mass Spectrometry (TOF-SIMS), it was determined that the carbonyls present on the surface of the C-treated FEP substrate are aldehydes or ketones in small organic chains that have been grafted on the surface uniformly. Thus, in specific aspects to functionalize the surface with COOH, one further reacts the surface with oxidation.

In embodiments wherein there are more ketones, the strategy of synthesis is to introduce an atom of oxygen in alpha of the carbonyl, turning the ketone into an ester. To process this reaction, one can use a peracid, like MCPBA (meta-chloroperoxibenzoic acid). In a second step, one can process a saponification to cut the ester and get carboxylic acids.

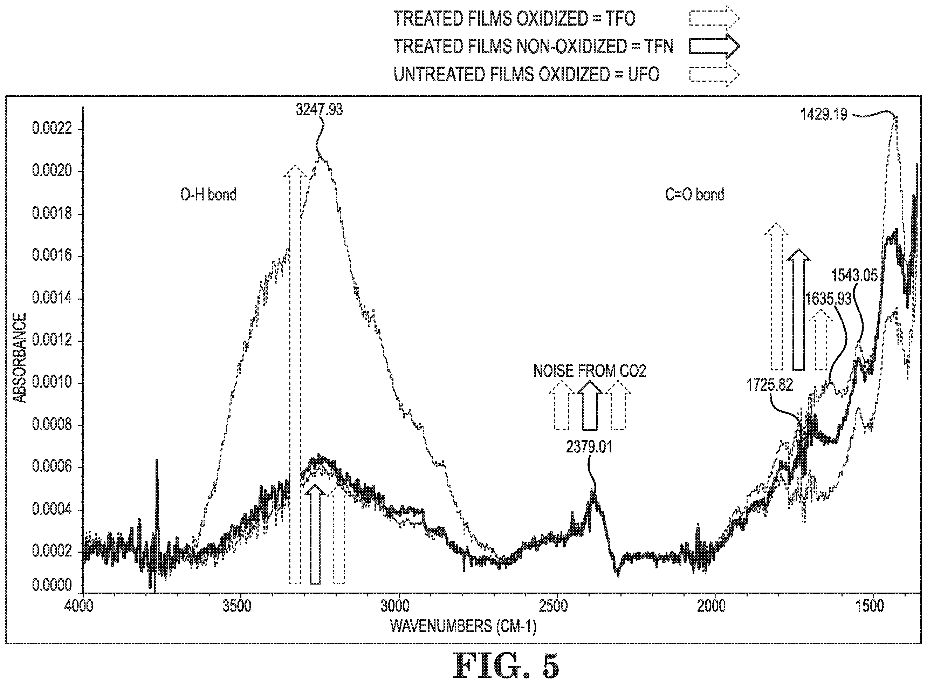

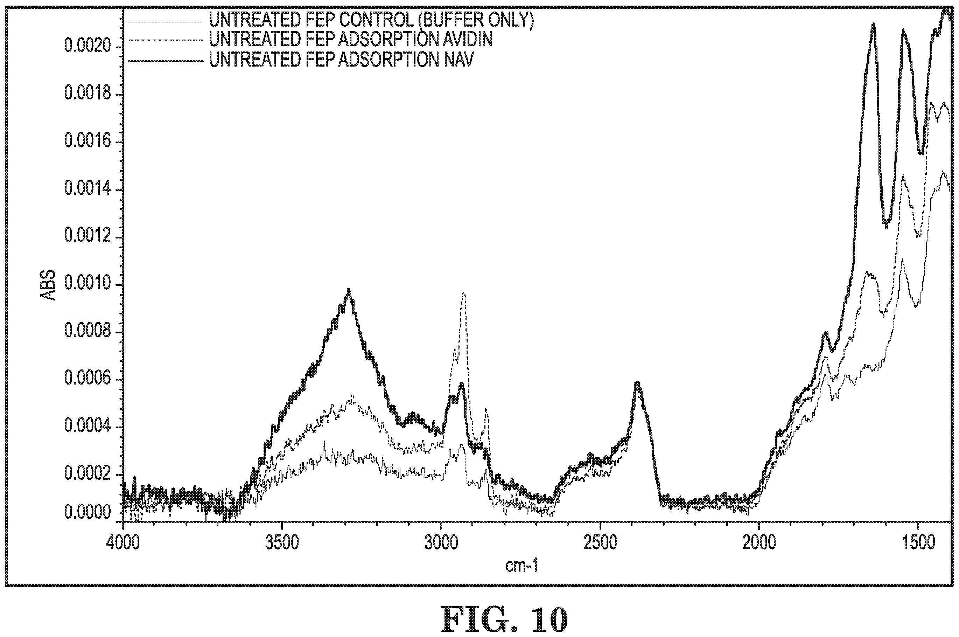

In embodiments wherein there are more aldehydes, one can oxidize strongly the surface, turning the aldehydes into carboxylic acids. In particular aspects, the surface was strongly oxidized by using potassium permanganate ($KMnO_4$) with sulfuric acid ($H_2SO_4$) concentrated solution. The results of the reaction were analyzed by FTIR. As it is shown in FIG. 10, the results show a clear, strong increase in the -OH peak, which, given the reaction, could have only come from the transformation of aldehydes into carboxylic acids. There is a slight increase in the carbonyls peak that in specific embodiments comes from the oxidation of alcohols into carbonyls. On the spectrum in FIG. 5, there is reacted C-treated film spectrum, the non-reacted C-treated film spectrum, and the reacted, untreated film spectrum. The untreated film spectrum was used as a negative control, as non-treated FEP should not react with $KMnO_4$. This film did not have an increase in -OH or C-O, which was an expected result. One can run the reaction at higher temperature and time in order to observe if either of these variables are potentially rate-limiting.

Thus, there was an amount of C=O that represented 3 % of the surface. According to the results, aldehydes are the major part of these groups. They are turned into carboxylic acids, so in specific embodiments at least 1% of the surface that is functionalized with COOH. 1% of the surface represents 1 carboxylic group per 2 $nm^2$. Yet, neutravidin covers about 25 $nm^2$, so as the surface is uniformly covered, there would be at least 10 carboxylic groups for one protein, which is enough to design one covalent bond. However, the percentage calculated for the amount of COOH is based on the results of the XPS, which give the percentages of O on a 10 nanometers depth. Therefore, in reality, one can expect to have more than 0.5 COOH/$nm^2$.

In the case that the oxidation does not design enough carboxylic acids, an alternative reaction may be employed. In a specific embodiment, a Grignard reagent was utilized. Indeed, in a water-free environment, one can introduce an atom of magnesium between a carbon and a halogen. The R-Mg-X designed this way is very reactive and allows the option of adding a carbon chain with specific functions, such as carboxylic acids. In certain cases for design of the Grignard reagent, if the reaction is not too difficult with chlorine or bromine, it may be more complicated with fluorine, although it may still be processed with the right choice of solvent and with a catalyst. An example is described in "Preparation of Alkylmagnesium Fluorides", (Yu, 1971). Therein, under conditions of atmospheric pressure reflux, using iodine as a catalyst, 1,2-dimethoxyethane as solvent, n-hexylmagnesium fluoride was produced in 92% yield in 4 hours.

A variety of options exist for imparting COOH groups onto a surface, however. In specific embodiments, the generation of carboxylic acids on the surface of a substrate includes grafting of acrylic acids, for example (see, for example, Racine *et al.,* 2010). Additional reactions to obtain COOH on an FEP surface include the following: 1) reduction of the surface by exposing untreated FEP to Sodium Naphthalene treatment, followed by oxidization of the surface by exposing treated FEP to $KClO_3$ in Sulfuric Acid; and 2) exposure of untreated FEP to Ammonia Plasma, wherein the sample is kept in an oxygen-free compartment in order to limit oxygen uptake on treated surface, followed by exposure of the treated surface to a solution of glutaric anhydride in acetone.

In certain embodiments, moieties other than carboxylic acid are produced on the surface of the substrate, and such modification can occur by any suitable means in the art. For example, one can conjugate polymers to proteins by acylation reaction starting with an aldehyde on the surface of a substrate (Srivastava *et al.,* 2014). Also, one can employ amino-functionalized cellulose acetate with glutaraldehyde (or cyclic hemiacetal or polymeric hemiacetal, for example) to obtain an activated surface for covalent biomolecule optimization (Heikkinen *et al.,* 2011).

Methods for creating functional groups, such as COOH groups, include plasma activation using treatment with gases such as argon, hydrogen, nitrogen, carbon dioxide, and combinations thereof. In specific embodiments, the plasma activation is achieved at low pressure, such as 0.1 Torr to 0.6 Torr, or closed to atmospheric pressure, such as 700 Torr to 760 Torr. Corona activation of the surface under gases (argon, nitrogen and hydrogen or combination thereof, for example) can be utilized to create active sites in the surface that could be further used in chemical treatments. Chemical treatment can comprise sequential chemical modification of the active or existing surface by chemical reaction that includes grafting polymerization, coupling, click chemistry, condensation, and addition reactions. For example, grafting polymerization in solution can be achieved by polymerizing vinyl monomers (acrylic acid,(metha) acrylates, (metha) alkyl acrylates, styrenes, dienes, alpha-olefines, halogenated alkenes, (meth)acrylonitriles, acrylamides, N-vinyl carbazoles, maleic anhydride, and N-vinyl pyrrolidones) *via* radical polymerization.

**D. Linkage of Avidin Species to a Surface**

In particular embodiments, a surface comprising carboxylic acids have attached thereto a protein species, such as an avidin species, for example. The protein species may be attached to the carboxylic acid groups by any suitable method, including adsorption or conjugation, for example. Adsorption of avidin species on a variety of species is known in the art (Albers *et al.,* 2012; Orelma *et al.,* 2012; Vermette *et al.,* 2003; Vesel and Elersic, 2012; Vesel *et al.,* 2012), and in specific embodiments the skilled artisan takes into consideration that protein adsorbs more on hydrophilic interfaces if the pH of the buffer is at a point where the protein obtains a charge opposite that of the interface. In addition, conjugation of avidin species on an assortment of species is also known in the art. Such methods include activation with EDC and NHS (Orelma *et al.,* 2012; Fabre *et al.,* 2012; Vermette *et al.,* 2003; Vesel *et al.,* 2012; Xia *et al.,* 2012).

In a specific embodiment, the surface of the substrate is C-treated oxidized FEP (functionalized with carboxylic acids). In initial studies, one can utilize a small surface, such as 1cm$^2$. Considering that one has about 1 COOH per nm$^2$, there would be 10$^6$ COOH on 1 cm$^2$. In moles, this corresponds to about 1.7*10$^{-18}$ mol. Another limiting factor is the size of the surface: one protein covers about 20nm$^2$, so one cannot link more than 50 000 proteins to the surface. One can use a large excess of proteins, like 5,000,000 proteins, which corresponds to 8.3*10$^{-18}$mol. Avidin weights about 66kDa; it corresponds to 6.6*10$^4$g.mol$^{-1}$. Then, the mass of protein to dissolve is 5.5*10$^{-13}$g. This number illustrates that minimal amount of protein may be utilized for each initial study. An example of a starting amount is 0.1mg/mL protein in the smallest volume possible.

Exemplary protocol proposal:

i. The first step would be to activate the surface with the sodium acetate buffer solution (10mmol.L$^{-1}$) containing NHS (0.4mol.L$^{-1}$) and EDC (0.1mol.L$^{-1}$).

ii. The second step is the binding of neutravidin by using the same buffer solution (10mM) containing neutravidin (100ug.mL$^{-1}$).

iii. Then, the excess of NHS esters can be removed by washing the system with 0.1M ethanol amine at pH 8.5. However in the process this step is not essential, because an excess of NHS esters is not expected.

iv. The final step is to remove the neutravidin that is nonspecifically bound: for that one washes the system with a 100mM glycine-HCl solution (pH adjusted to 2.5 with HCl and NaOH).

To determine if the protein is linked, one can employ AFM, FTIR, zeta potential, raman, protein 660nm assay, Bradford test, ELISA test, fluorescent microscope methods, or TOF-SIMS to detect the peptide bonds.

**E. Attachment of Aptamers to the FEP Surface**

To demonstrate attachment of DNA to a FEP surface, one may attach a biotinylated object to the neutravidin bound to FEP. In specific embodiments, one can use biotinylated fluorescein that under a UV lamp may allow detection of the fixed proteins. In other cases, one can use polymer beads that are biotinylated; SEM coupled to an EDS detector will detect the polymer beads. In particular embodiments, one can use biotinylated long random DNA chains: SEM coupled to an EDS detector will detect the amount of Phosphorus present in the DNA if it is fixed.

**V. Alternative Embodiment for Cell Isolation**

In an alternative embodiment to utilizing aptamer tentacles to isolate desired cells, one can infuse biotinylated aptamers specific to the desired cells in a sample, followed by providing the mixture to a container coated in neutravidin to selectively trap biotin and then retain the desired cells upon removal of the sample.

In order to isolate desired cells, one can selectively target them with aptamers, and the aptamers may be engineered through cell-SELEX process. Once the sequence of the aptamers specific to the desired cells is known, one can synthesize them in higher quantities. In cases wherein the desired cells are monocytes, at least one surface marker present on monocytes does exist, CD14. Synthesized chemically, these aptamers are functionalized with biotin. One can also prepare a container of FEP having an interior surface coated with neutravidin or another biotin-binding protein (avidin, streptavidin). To do that, one can first functionalize the FEP surface with carboxylic groups and then link the proteins with a peptide bond to the surface (reaction catalyzed by NHS and EDC).

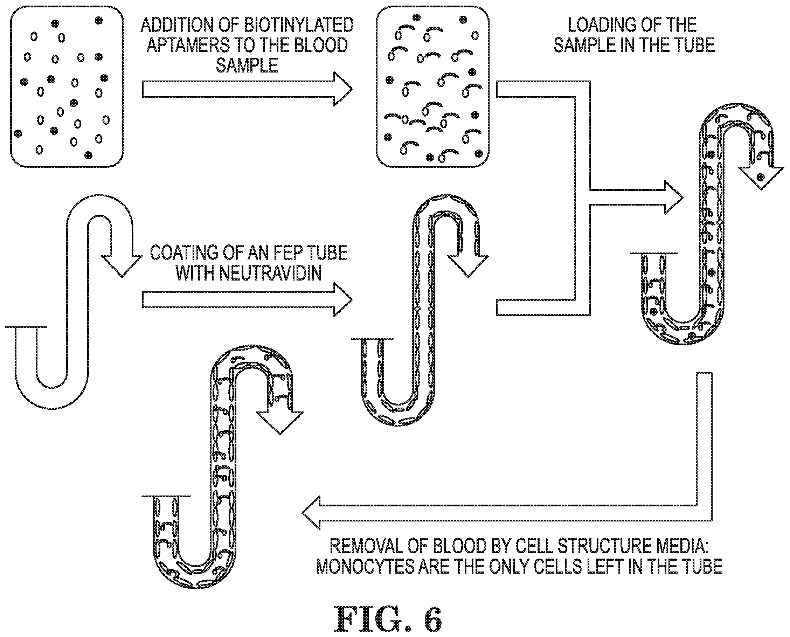

An exemplary process is illustrated in FIG. 6. The first step of the process is to mix the apatmers with the samples, such as 100 mL blood samples. The monocytes are labeled in the end with biotinylated aptamers. Then, in a second step one can fill the tube with blood sample, allowing the biotin to link specifically to the surface through the neutravidin (the Kd of the biotin-neutravidin complex is $10^{15}$). In a third step, one can remove the sample by filling the tube with media, such as the media that will be used for the culture of the cells. The fourth step is to detach the cells from the walls of the tube. One can use enzymes to cut the aptamers or enzymes to digest the proteins present on the surface of the cells. Or, one can also heat the tube (for example, 48 degrees for 2 minutes); the heating will deform the aptamers and release the cells. In a final step, the cells are collected in another container, such as one comprised of FEP.

## VI. Exemplary Applications for Isolated Cells from the System

The cells isolated with particular methods of the disclosure may be utilized for one or more applications upon release of the cells from the aptamers, or the cells may be utilized following further processing steps. In specific embodiments, cells isolated by the system of this disclosure may be utilized for storage, followed by future use, including future clinical use for therapy for one or more individuals. In particular embodiments, cells isolated by the system of the disclosure are delivered to an individual in need thereof, including directly to an individual in need thereof, in certain embodiments. The cells may be further processed, such as further concentration of the cells, addition of a pharmaceutical carrier, addition of a biological agent (such as cytokines (including one or more interleukins), chemokines, growth factors, or any factor that activates antigen-presenting dendritic cells, for example), recombinant manipulation of the cells, such as engineering the cells to express one or more particular T-cell receptors, chimeric antigen receptors, and so forth . However, in certain embodiments the cells are sufficiently prepared by the system of the disclosure such that they are useful for direct delivery to an individual in need of therapy of the cells. The individual may or may not be the person from whom the original sample comprising the cells was obtained. Thus, the cells delivered to the individual following isolation with the system may be autologous to the individual (belonging to that same individual) or allogeneic to the individual (belonging to an individual other than the individual from whom the sample was taken). The person performing the system steps may or may not be the person that obtains the sample from an individual or the person that delivers the sample to an individual in need thereof.

Particular embodiments of the disclosure utilize the cells isolated by the system for cell therapy for a mammal. In specific embodiments, the cells are employed as personalized medicine for an individual with a medical condition. In specific embodiments, the medical condition is cancer, joint repair (including spinal discs), nervous system repair, or autoimmune disorders. In particular aspects, the cells are employed as immunogenic compositions, including vaccines, for example. In embodiments wherein the individual has cancer, the cells isolated from the system may be specific for a tumor antigen for the cancer of the individual.

The capture of a specific cell-type onto a surface using the methods outlined in this disclosure in particular embodiments may be utilized for applications beyond the separation of a cell type from a cell mixture. For example, in specific embodiments, in some co-culture applications, it might be useful to have one cell type anchored to the surface, whilst still interacting with the cells in suspension, in order to be able to distinguish the two populations. Furthermore, for cells that prefer to grow as an adherent culture, in some embodiments it might be useful to utilize the methods outlined to allow for reversible docking of the cells to a surface during culture; whether the surface be a flat surface or a spherical surface like that found on a microcarrier.

## VII. Kits of the Disclosure

Any of the components of the systems or methods described herein may be comprised in a kit. In a non-limiting example, an apparatus for capture of biological agents may be comprised in a kit in suitable container means. The apparatus may be of any kind, so long as it is configured to allow placement of a polymer thereon. The apparatus may be a bag and in some cases is an elongated bag that may be considered tubing. The kits may comprise one or more fluoropolymers (including for a bag of the system) for use in the apparatus, an apparatus comprising fluoropolymer, one or more reactive moieties, one or more linkers for use with the reactive moiety, suitable reagents, and/or one or more devices and/or reagents for sample extraction from an individual. Kits of the disclosure may further comprise one or more of an antigen; an apparatus for sample collection or storage (such as a vial, syringe, cup, scalpel, or a combination thereof); a polymerase (such as phi29 polymerase); an endonuclease; and a buffer.

The kits may comprise suitably aliquoted liquids for use in the methods or for use in preparation of the apparatuses for use in the methods. Certain components of the kits may be packaged either in aqueous media or in lyophilized form. The container means of the kits may generally include at least one vial, test tube, flask, bottle, syringe or other container means, into which a component may be placed, and preferably, suitably aliquoted. Where there are more than one component in the kit, the kit also will generally contain a second, third or other additional container into which the additional components may be separately placed. The kits of the present invention also will typically include a means for containing any of the components of the kit in close confinement for commercial sale. Such containers may include injection or blow-molded plastic containers into which the desired components are retained, for example.

When the certain reagents of the kit are provided in one or more liquid solutions, the liquid solution may be an aqueous solution, including a sterile aqueous solution, for example. In which case, the container means may itself be a syringe, pipette, and/or other such like apparatus. However, the components of the kit may be provided as dried powder(s), in some cases. When reagents and/or components are provided as a dry powder, the powder can be reconstituted by the addition of a suitable solvent. It is envisioned that the solvent may also be provided in another container means.

EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

EXAMPLE 1

ESTIMATION OF NUMBERS OF ISOLATED MONOCYTES

The present example provides an estimation of the number of monocytes that are isolated in embodiments of the disclosure.

Given that Tube: R=0.5cm, L=1.2m => V=94mL, S=$3.8*10^{-2}m^2$

One monocyte has a radius about $7\mu m$. Then, the average surface it will cover if it is fixed is $1.5*10^{-10}m^2$ . The maximum number of monocytes that can be captured on the tube surface is $2.5*10^8$. In an exemplary 94mL of blood, there are $7.5*10^7$ monocytes (assuming a concentration of $8*10^8$monocytes/L in an average blood sample). Assuming a cell capture efficiency of 25%, a release of 80% and a final viability of 80%, then around $1.2*10^7$ viable monocytes would be isolated. In certain aspects, $10^6$ to $10^7$ monocytes are needed for processing a dendritic cells vaccine.

EXAMPLE 2

SURFACE PREPARATION

This example concerns surface functionalization and protein immobilization on fluorinated ethylene propylene (FEP) as an example of a material of which a container is comprised or has a wall prepared therewith. In some embodiments, immobilization of protein can be performed by two distinct methods: physical adsorption and chemical conjugation. All studies described in this example were performed with one of two types of protein (Avidin and Neutravidin) on three different FEP film surfaces (Untreated, C-Treated and C-Treated oxidized). Examples of protocols to demonstrate adsorption and chemical conjugation as well as film oxidation are provided herein below.

**Adsorption**

The adsorption embodiment involves the physical adsorption of protein on a surface by attractive forces, such as electrostatic, hydrophobic and hydrophilic interactions, or Van-der-Waals forces. This method is straightforward and the adsorption is spontaneous, only taking a few seconds to initiate. Several internal and external parameters that can affect the adsorption process and, ultimately, the protein's behavior on the surface, can be considered. External parameters such as temperature, pH and ionic strength can change the equilibrium state and kinetics of adsorption. For example, increasing the temperature will allow an entropy gain and help to release adsorbed molecules and salt ions from the surface and help the structural rearrangements of the proteins that will enable more proteins to adsorb to the surface. Buffer pH will affect the electrostatic state of proteins and will create negative or positive charges that will change the attractive or repulsive interactions with the surface depending of the specific isoelectric point (IEP) of the protein. Finally, high ionic strength in solution will increase the tendency of protein to aggregate (Table 1; Rabe 2011)

To complete this method, the surface is in contact with the protein solution for only a short period of time (e.g., seconds or minutes). After this step, the surface is gently washed and ready to use for the next step. Simplicity and speed of this method makes it a prime candidate to obtain a protein layer on the surface. One can consider a variety of parameters that may affect reaction reversibility (desorption) and the stability of the protein after adsorption.

Table 1: General adsorption tendency of Proteins (Rabe, 2011)

| External conditions | |
|---|---|
| Temperature | Increase in temperature will increase the amount of protein adsorb |
| pH | Electrostatic interactions are minimized when pH=IEP. Proteins densities on the surface at this point should be higher. |
| Ionic Strength | High ionic strength increases protein aggregation |
| **Surface properties** | |
| Surface energy | Proteins seem to adsorb more to hydrophobic surfaces except for glycoproteins that adsorb on hydrophilic surface. |
| **Proteins properties** | |
| IEP | Proteins charge will depend on the pH condition. Positive when pH<IEP and negative when pH>IEP |
| Protein structure | Small and rigid proteins (like lysozyme and β-Lactoglobulin) will undergo less confirmation change and reorientation than intermediate proteins (like Albumin, transferrin and immunoglobulin) |

**Conjugation**

In another embodiment, there is chemical conjugation of proteins to the surface. With this method, proteins are covalently coupled to the substrate by a specific chemical reaction. To complete this method, the surface is modified by adding carboxylic acid groups onto the surface of FEP. One can use a two-step method to achieve this functionalization. The first method is called C-Treatment, a surface that is commercially available (Saint-Gobain), and the second method is an oxidation reaction.

When carboxyl groups are present on the surface, a carboxyl-to-amine conjugation can be used to bind the protein to the surface. In specific embodiments, one activates the carboxyl group by using a water-soluble carbodiimide crosslinker 1-Ethyl-3-(3-dimethylaminopropyl) carbodiimide hydrochloride commonly known as EDC (Drumheller and Hubbell 2003).

After activation of the surface with EDC/NHS, protein comes into contact with the NHS ester reagent and binds to surface. One can optimize the incubation time in order to improve the scalability of this method. After the conjugation reaction, the surface is washed with a solution of glycine-hydrogen chloride (Glycine-HCl) to remove all electrostatically bound proteins. (Orelma, *et al.* 2012)

This attachment method induces a more stable protein layer to the surface, in particular embodiments.

**Examples of Film Selections**

In specific embodiments, the container for the system comprises fluoroethylene propylene (FEP), and in certain aspects the FEP may be untreated FEP, C-Treated FEP, or Oxidized C-Treated FEP. Untreated FEP is rather hydrophobic (water contact angle: 99°) and has a relatively low surface energy (19 mJ/m$^2$). Protein adsorption can occur on this surface, although in particular embodiments the surface is modified. In embodiments wherein the conjugation method is performed, the FEP surface is modified in order to obtain the starting carboxylic acid functional groups.

C-Treated FEP (Saint Gobain) provides the necessary polar surface functionalities for a chemical reaction to add -COOH groups to the surface. An effect of the C-Treatment is that it raises the surface energy of the film (water contact angle: 66° and surface energy: 38 mJ/m$^2$) whilst also creating a more hydrophilic surface compared to its untreated counterpart. Surface energy has been known to affect the adsorption of protein to the film making the C-Treated FEP film an alternative candidate for the adsorption method, in particular embodiments.

For Oxidized C-Treated FEP, an oxidation reaction is performed to add carboxylic acid groups to the surface to covalently attach the protein to FEP. The -COOH group present on the film allows EDC/NHS to react and create an amide bond to bind avidin. This reaction may be performed with two products: 0.8 mM potassium permanganate (KMnO$_4$) and 0.12 M sulfuric acid (H$_2$SO$_4$). In particular embodiments, the addition of carboxylic acid on the surface is a prerequisite for being able to covalently bind the protein to the surface. In specific embodiments, this oxidation reaction makes the C-Treated FEP film more hydrophilic and further increases the surface energy. Besides conjugation, in specific embodiments this oxidized surface is also a starting material for the adsorption process, as it changes the surface energy and wettability.

Table 2: Comparison between three different types of FEP film

| | Untreated | C-Treated | Oxidized C-Treated |
|---|---|---|---|
| **Elemental and chemical presence** | C and F only | C, F, N and O Presence of carbonyl groups; aldehydes and ketones | C, F N and O Addition of carboxylic acid groups –COOH |
| **Surface energy** | Highly hydrophobic surface | More "hydrophilic" than untreated | More "hydrophilic" than C-treated |
| **Proteins attachment** | Adsorption | Adsorption | Conjugation and adsorption |

SURFACE CHEMISTRY

Methods are used to evaluate elements and chemical groups present on film surface. For surface characterization, one considers the presence of oxygen atoms and/or aldehydes, ketones and carboxylic acid groups. In specific embodiments, only C and F are seen on untreated FEP, there is the presence of N, O and aldehydes/ketones group on C-Treated FEP, and there is the presence of COOH group on oxidized C-Treated. For the sample with protein, there is focus on the high presence of C, N and O and a small amount of S. The main chemical groups present on protein are carboxylic acid group (COOH), amide bond (CNO) and primary amine ($NH_2$).

X-Ray Photoelectron Spectroscopy (XPS)

XPS is used to determine quantitative atomic composition and chemistry on a surface. A monoenergetic X-Rays bombard the sample and cause electrons to be ejected. Identification of the elements is made from kinetic energies of the ejected electrons. Analysis is done on a depth range of -50-100 Å and on a surface area of 2mm X 0.8 mm. (Brundle, Evans Jr and Wilson 1992). XPS does not detect H and He presence. Data are given in atomic % or carbon % for the chemical state analysis.

The following shows that on Untreated FEP there is only C and F atoms as expected. The presence of N (2.6%) and O (3%) are also detected on the C-Treated film. Those elements are added to the surface by the C-Treatment. Oxygen atoms on surface could be an indicator of the presence of aldehydes and ketones. In Table 3 below, the presence of C=O bond (3%C) with the carbon chemical state analysis confirms that carbonyl (C=O) group are present on the surface. A small increase of O on the oxidized C-Treated is observed (3.4%) but that does not match with the expected result. Duplication of the amount in oxygen was expected because of the oxidation reaction that is supposed to add another O to the aldehyde group. Moreover, the double bond O (=O) seems to reduce after the oxidation. This could indicate that the reaction is removing the aldehyde or ketone group from the surface instead of modify them in carboxyl group.

Table 3: Carbon chemical state (in atom% of C) from XPS measurement

| ID | Carbon Chemical State (in Atom% C) | |
|---|---|---|
| | C-(O,N) | C=O |
| Untreated FEP | 0 | 0 |
| C-Treated FEP | 4 | 3 |
| Oxidized C-Treated FEP | 3.5 | 1.8 |
| Oxidized Untreated FEP | 0.5 | 0.4 |

The XPS method is used to verify the presence of protein on the surface.
Table 4 shows XPS measurement of different types of FEP film.

|  | Carbon-based | Nitrogen-based | Oxygen-based | Fluorine-based | Sulfur-based |
|---|---|---|---|---|---|
| Untreated FEP | 32.4 | 0 | 0 | 67.6 | 0 |
| C-Treated FEP | 40.7 | 2.6 | 3.0 | 53.7 | 0 |
| Oxidized C-Treated FEP | 37.7 | 1.2 | 3.4 | 57.4 | 0 |
| Oxidized Untreated FEP | 32.7 | 0 | 0 | 66.7 | 0.5 |

Data shown in atomic %

Table 5 shows a distinction between the untreated film and the untreated film with adsorbed Neutravidin. The second film shows the presence of N and O. The same type of information could be detected on the oxidized C-Treated with conjugated Neutravidin. The high presence of N and O compared to the oxidized C-Treated sample indicates that there are proteins on the surface of the film.

Table 5 shows XPS measurements on different types of FEP film.

|  | C-based | N-based | O-based | F-based | S-based |
|---|---|---|---|---|---|
| Untreated | 32.4 | 0 | 0 | 67.6 | 0 |
| Untreated+ Adsorbed Neutravidin | 39.2 | 3.5 | 4.2 | 53.0 | 0 |
| C-Treated | 40.7 | 2.6 | 3.0 | 53.7 | 0 |
| Oxidized C-Treated | 37.7 | 1.2 | 3.4 | 57.4 | 0 |
| Oxidized C-Treated +Conjugated Neutravidin | 53.2 | 10.0 | 14.1 | 21.3 | 0 |

This method gives information about the presence and the absence of protein, while other methods may be utilized to determine the amount of protein and the protein layer uniformity.

Fourier Transform InfraRed spectroscopy (FTIR)

FTIR is a non-destructive method that provides information about chemical bonding of element on solid or thin film. The FTIR-ATR method was used to focus on the surface and obtain the spectrum of the molecular vibration. Spectrums were background and atmospheric corrected . Data were modified to obtain the information in the absorbance option between the spectra area of 4000-1400 cm$^{-1}$. Focus was done on this area to avoid the high signal of the CF bond (1100-1300 cm$^{-1}$) of the FEP. The correlation between particular chemical groups on FTIR spectra and their group frequency is known in the art (Coates 2000).

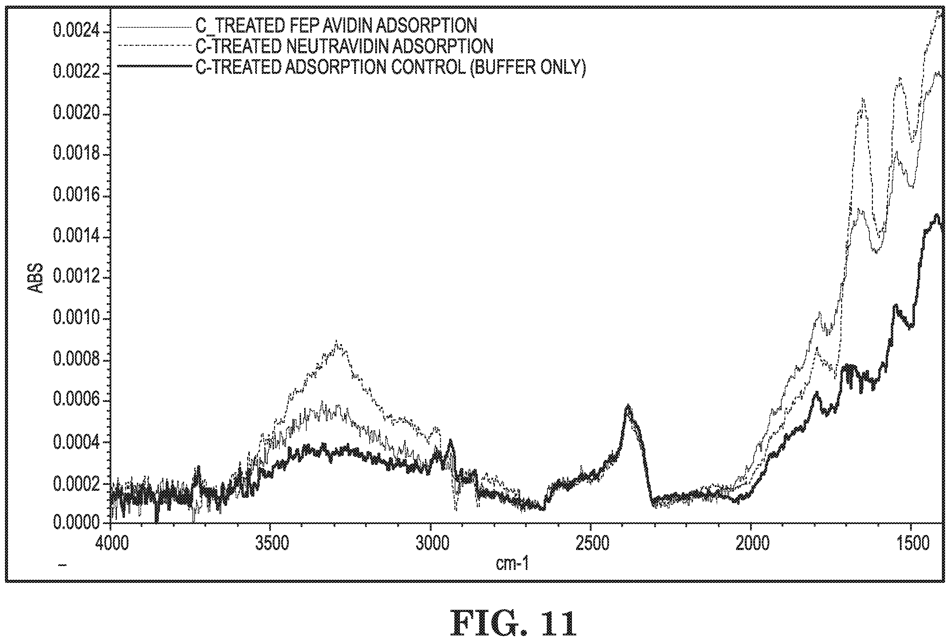

To verify the presence of proteins on a sample, one should consider the oxygen double bond (C=O) of the amide group area at ~1640 cm$^{-1}$. One can definitively see a peak in this area on the adsorbed protein samples compared to the control film where surface was only in contact with the NaOAc buffer. This peak is present on all films with adsorbed proteins and indicates that both avidin and neutravidin could be adsorbed on the FEP surface. There is a slight difference in peak intensity between avidin and neutravidin on the Untreated and C-Treated film. In specific embodiments, this difference is from the adsorbed proteins amount, and this can be verified by other quantitative methods. FIGS. 10-11 show spectrums of samples with immobilized proteins.

Methylene Blue Dye (MB)

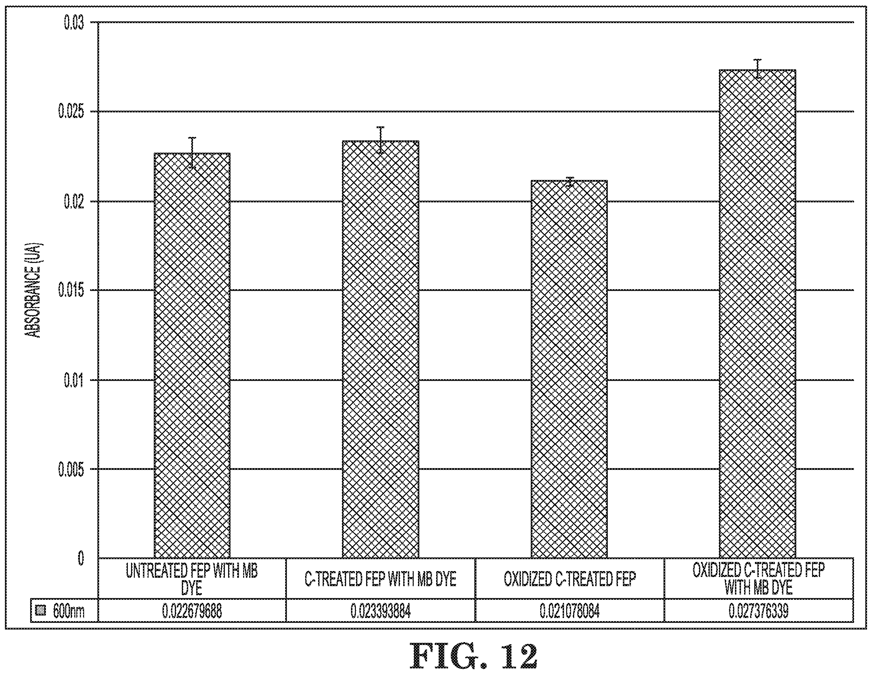

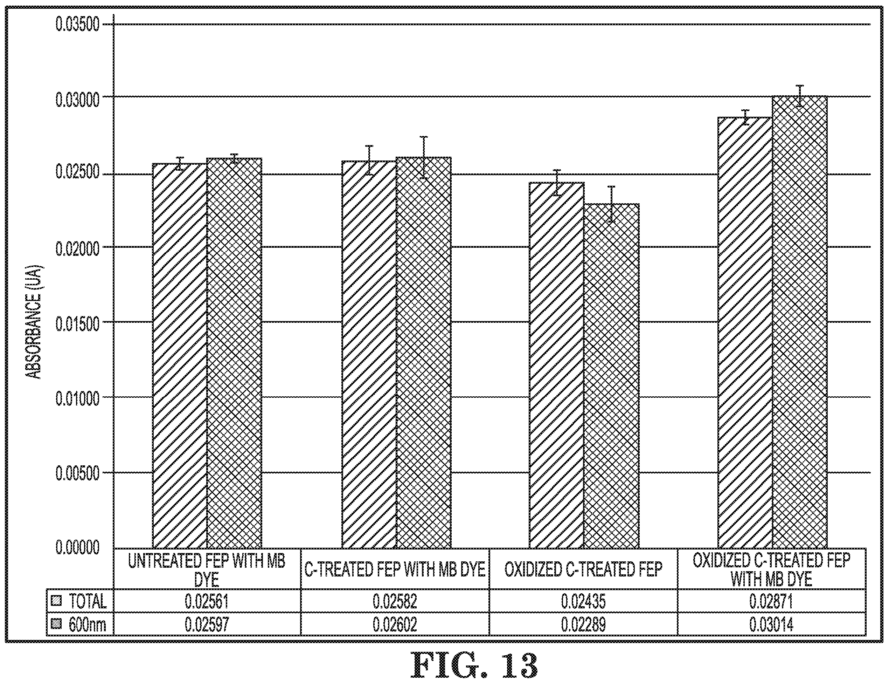

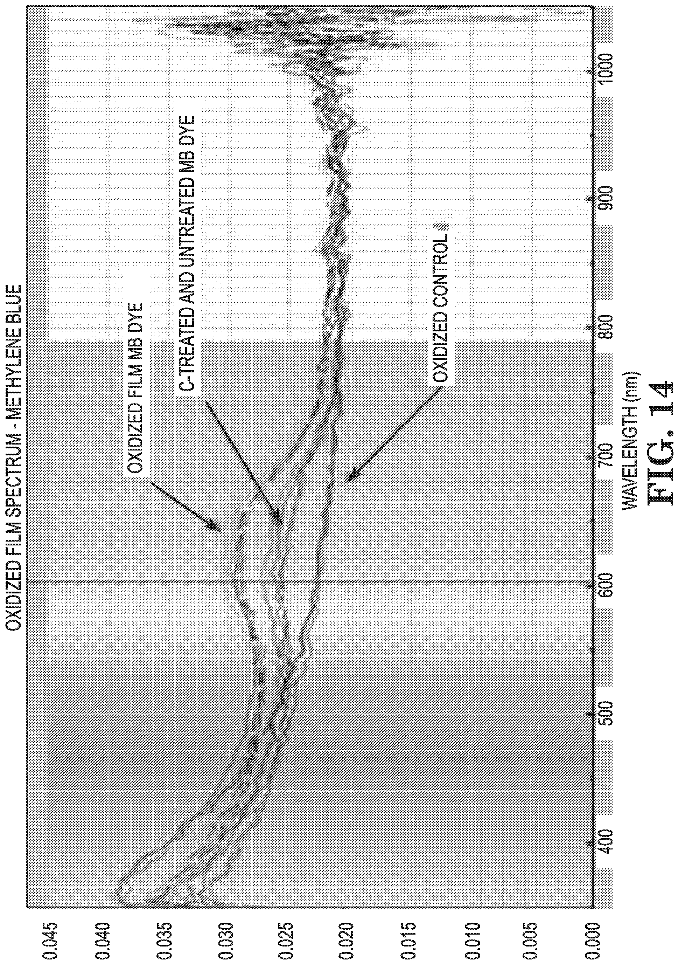

This method is developed to identify and measure the presence of carboxyl groups on a film after is oxidation and/or functionalization. Methylene blue is used to identify the carboxylic acid (-COOH) and non-polycation bound carboxylate groups (-COO$^-$) on a surface. This positively charged dye is adsorbed on the functionalized surface and is measured by visible spectroscopy at a wavelength of 600 nm. In particular embodiments, the procedure utilizes a dipping step where the functionalized film is in contact for 10 min with the methylene blue solution. Thereafter, the film is washed and dried before measurement. By taking a measurement of the sample before and after to stain, one could observe the presence of absorption at the 600 nm wavelength. This shows the presence of the methylene blue as it is bound with the available carboxyl and carboxylate group on the surface of the film. This change in absorption is visualized in FIGS. 12, 13, and 14 on the oxidized C-Treated sample after being dyed in methylene blue solution. A control is useful because background could change in time. One way to realize the comparison includes subtraction of the control (blank) from the final absorbance measurement.

## TOPOGRAPHY and OPTICAL ANALYSIS

Methods concern the evaluation of topography of the surface, and there is visual data to see how chemical modification and protein immobilization change FEP surface.

Scanning Electron Microscopy (SEM)

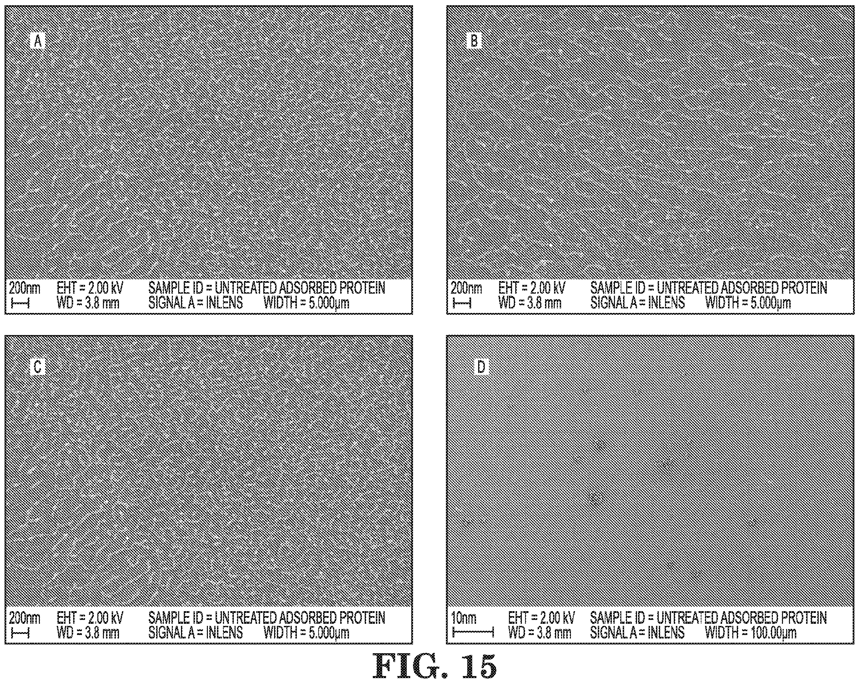

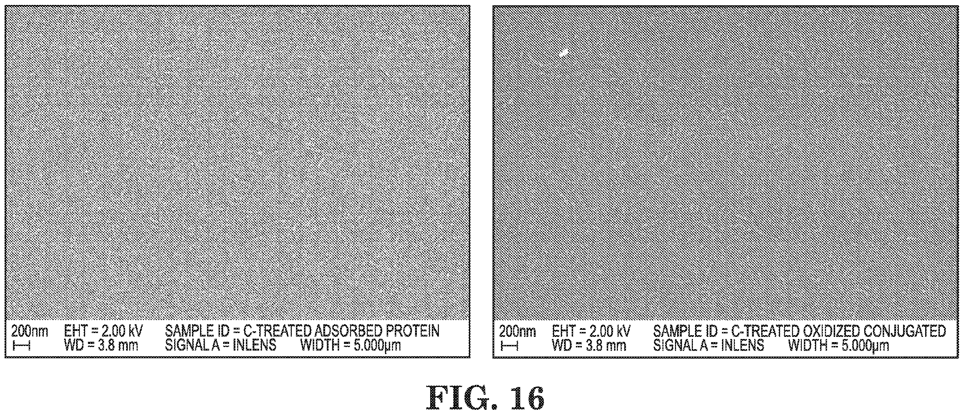

One method for analysis includes Scanning Electron Microscopy (SEM). This method provides high-resolution and long-depth-of-field images of the sample surface. SEM is used to see the protein layer to the surface. Different patterns of layers are observed on the FEP surface. The net-liked layer observe on untreated FEP (FIG. 15) could be explained by the possible protein aggregation because of the high hydrophobic interaction of the untreated film. In that case, protein-to-protein interactions are preferentially promoted to obtain the equilibrium state. This kind of layer is not observed on the other types of FEP film (FIG. 16).

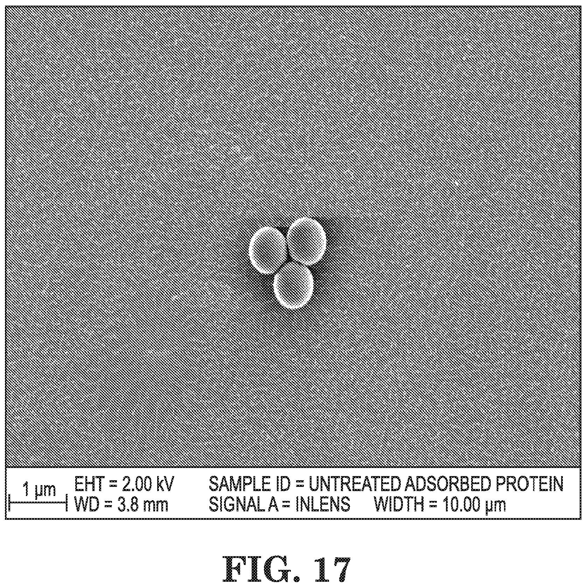

FIG. 15D shows some biotin coating polystyrene microbeads attached to the surface. A complete layer of those beads was expected to test the reactivity of the biotin binding site (biological activity) of the adsorbed Neutravidin. Only disperse beads were observed on the Untreated film and a few of them were on the C-Treated and Oxidized C-Treated films. In specific embodiments, this difference is explained by the big size difference between beads (d= 1 $\mu$m) and proteins (~20 nm). FIG. 17 shows bead attachment in more detail. A different pattern of the protein layer is observed under the beads. Another spot with the same layer pattern is also observed on the left side corner. In specific embodiments, this is explained by proteins that change their original adsorbed conformation because attractive force of the biotin presented to the beads. The combination of an attractive biotin force and the rinsing step during the sample preparation may result in loss of the biotin beads of the surface, allowing for a different pattern trace of the Neutravidin conformation modification on surface.

Optical microscopy

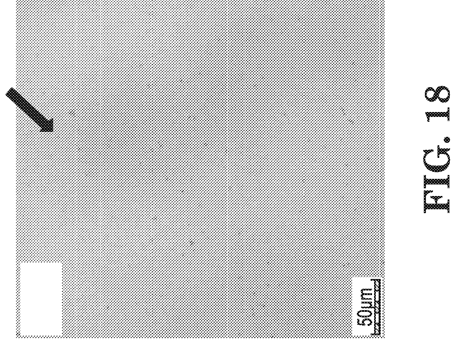

Optical microscopy may be used to obtain magnified images of small sample with the help of visible light and lenses. The FIG. 18 image shows the presence of the biotin microbeads attached to the protein layer on the untreated film. This technique can help to observe the uniformity of the protein layer with the help of a label like the microbeads or a chromogenic dye.

## SURFACE ENERGY

Zeta Potential with SurPASS

The SurPASS is an instrument to measure the streaming potential/streaming current of a solid surface. The zeta potential of a solid surface can be calculated from the streaming current for planar samples like FEP film. Using an installed automatic titration system, the zeta potential as a function of pH or the zeta potential as a function of surfactant concentration can be measured. (Anton Paar 2013). This method is sensitive and gives information about the surface energy of the sample at different pH and the IEP point of the surface. In specific embodiments, this device is helpful to characterize surface modification after the C-Treatment, the oxidation reaction and the protein adsorption. In certain embodiments, an increase of the IEP may be seen after the C-Treament and the oxidation as an increase of the zeta-potential value taken at the same ionic and pH conditions. Data from SurPASS studies may indicate the increase of the hydrophilic character of the FEP film after treatment. Studies on oxidized C-Treated FEP may be performed.

## PROTEIN ASSAY

Methods described immediately below are used to test directly the proteins. Methods to visualize directly the protein and to verify their stability are demonstrated.

660 nm Protein Assay

This method uses a dye-metal complex that binds to primarily basic amino acid residues in proteins, such as histidine, arginine, lysine, tyrosine, tryptophan and phenylalanine (Antharavally, *et al.* 2009). The reagent, in acidic conditions, is reddish-brown but changes to green when the dye-metal complex binds to protein. The maximum absorption of the dye is measured at 660nm with a UV-Vis spectrophotometer. This technique is primarily used to measure protein concentration in a solution but is modified herein to identify protein on a surface. The functionalized surface with proteins is in contact with the reagent for at least 5 minutes, in specific embodiments. Thereafter, the film is washed and dried before the absorption is measured by UV-Vis spectroscopy.

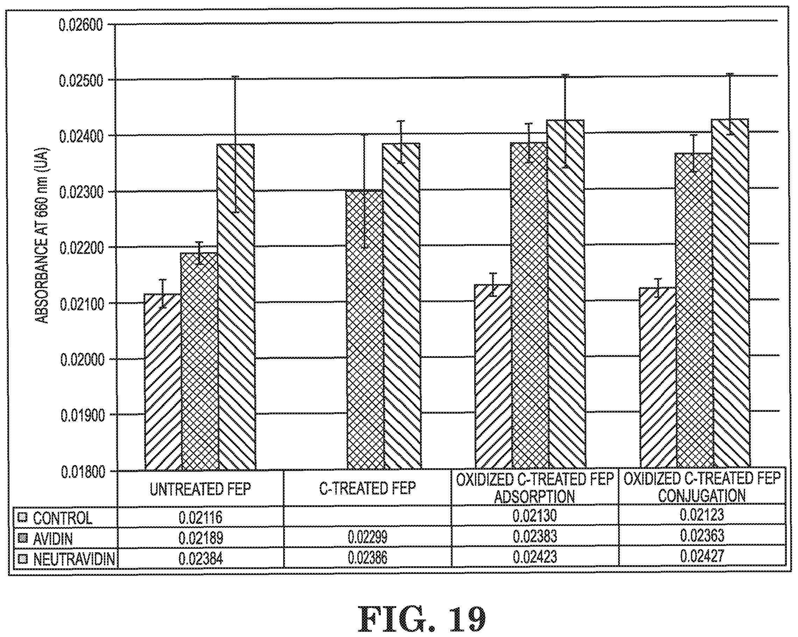

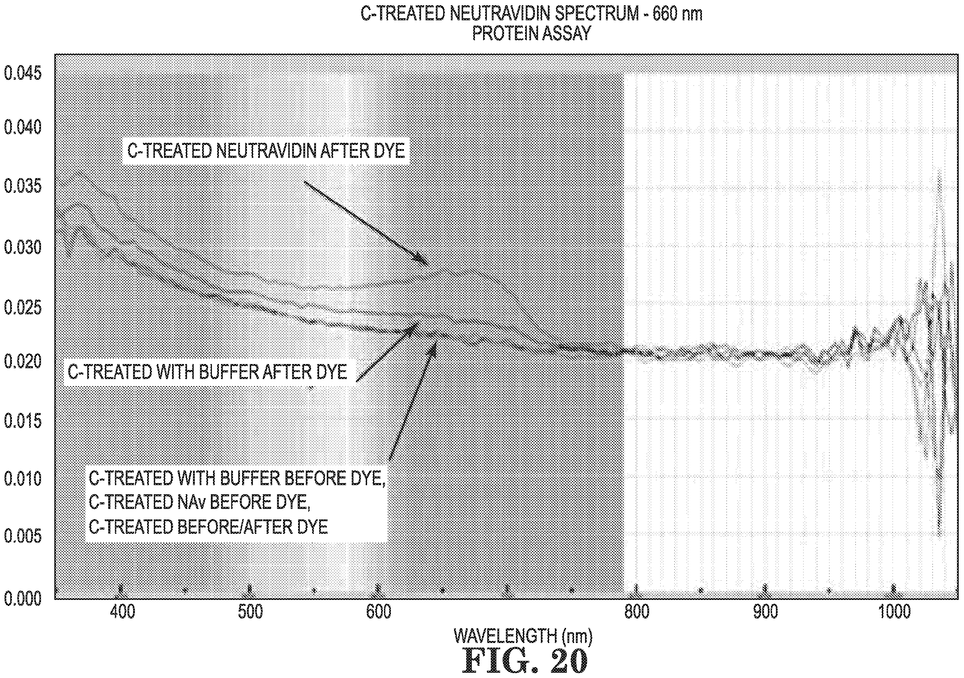

By taking a measurement of the sample before and after to stain protein, the observation can be made at the 660 nm wavelength. The increased absorption shows the presence of protein on the surface of the film. This effect can be visualized in FIG. 19 when looking at the second and third columns labeled "avidin" and "neutravidin" that represent stained proteins on film compare to the column labeled "control" where only the film with buffer residues react with the reagent. One can see the presence of proteins on all types of films with both proteins; Avidin and Neutravidin when using the conjugation and the adsorption method. Avidin on Untreated FEP seems to show less attachment to surface than all other methods. One can give consideration to the buffer choice used to prepare the protein solution, as some buffers can interact with the "Protein Assay Reagent" and give false positive results. FIG. 20 shows the C-Treated neutravidin spectrum 660 nm protein assay.

Washing experiments

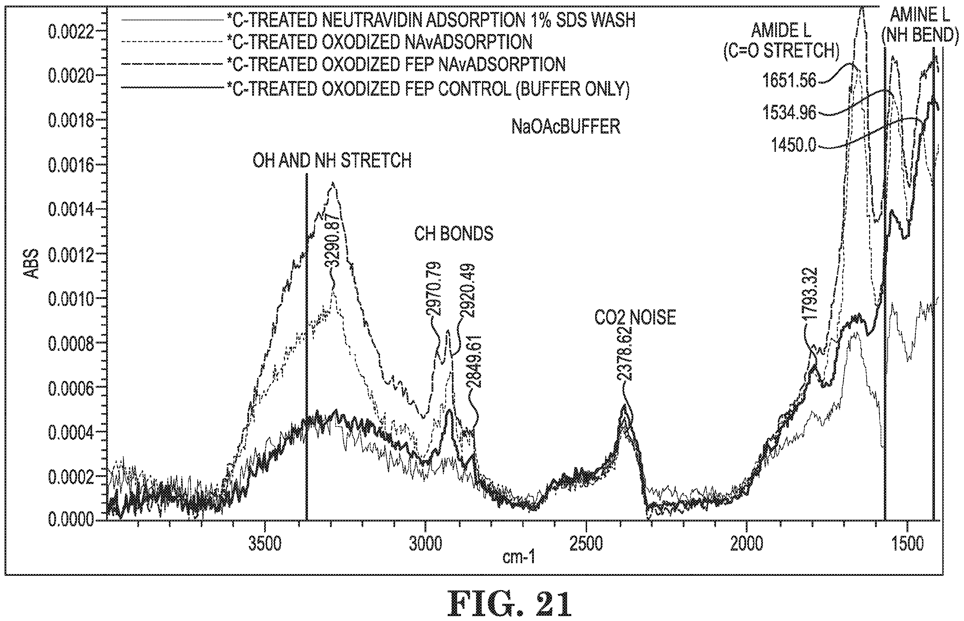

In specific embodiments, one can characterize protein stability using a two-washing step following protein functionalization on film. The first method involves the use of a harsh ionic detergent, such as Sodium Dodecyl Sulfate (SDS), which is usually used to rinse a surface to remove protein. This detergent affects the molecular 3D conformation of protein by disrupting non-covalent bonds and denaturing proteins. FIG. 21 shows Oxidized C-Treated film with adsorbed Neutravidin before and after the SDS wash. Like expected, SDS remove nearly completely the protein layer and other buffer residues. The amide bond peak on the SDS curve (yellow) is below the value of the control (red). With this experiment, one can determine the limit of the rinsing step that could be perform on the protein functionalized surface.

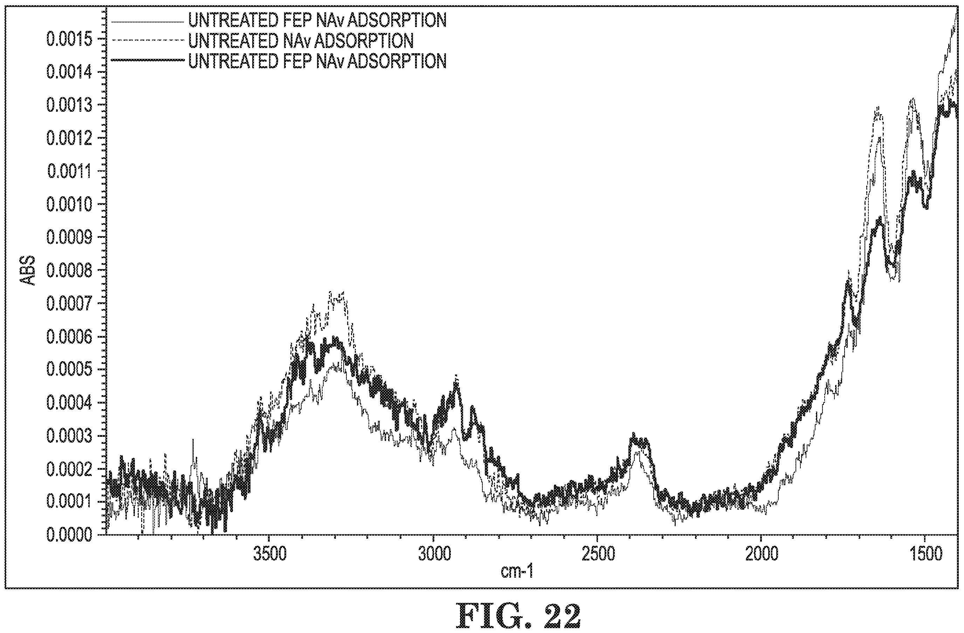

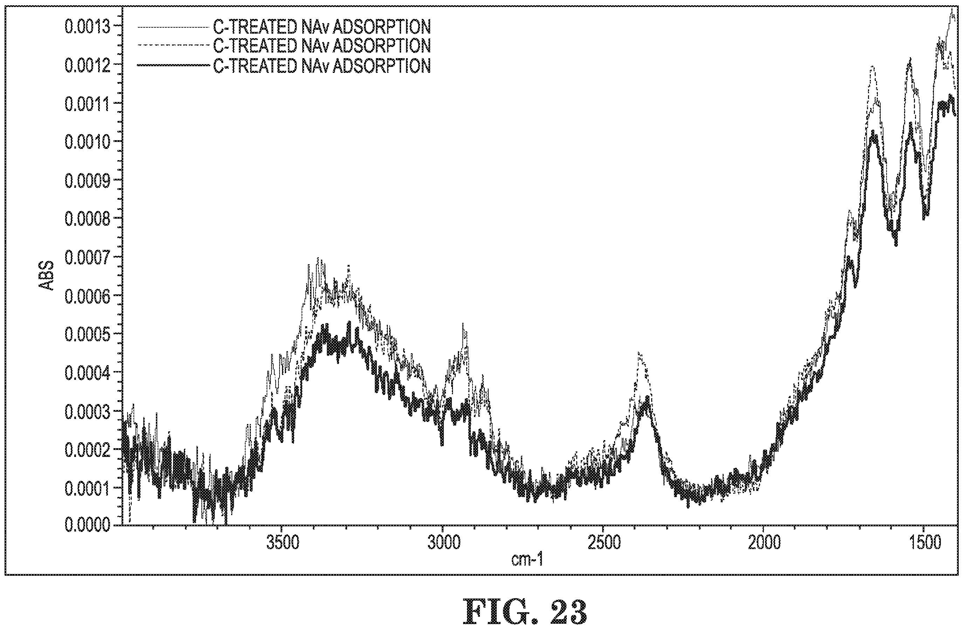

The second method involves the use of an ultrasonicator. This machine is generally used to wash a surface and remove salt and dust residues by impulsion. FIGS. 22 and 23 show a result of the ultrasonication on the untreated and C-Treated FEP with adsorbed Neutravidin. After ultrasonication step, an amide peak ($1630$ cm$^{-1}$) is still present and gives an indication of the stability of the protein on the film.

**EXAMPLES OF METHODS TO CHARACTERIZE PROTEIN STABILITY, LAYER UNIFORMITY, AND BIOTIN BINDING ACTIVITY**

Fluorescence Labeling

By using fluorescent microscopy, a fluorophore conjugated to the biotin is used to observe the protein adsorbed to the surface (Sromqvist, *et al.* 2011). Biotin-4-fluorescein quenches when bind to Avidin and is used in some studies to titrate the biotin binding site of the avidin. (Fluorescein label to a protein typically reduces fluorescein's quantum yields 60% but only decreases its extinction coefficient by 10% (Thermo Scientific 2011)) . In specific embodiments, this molecule is used in the same way on the film in order to verify the biological activity of the immobilized avidin.

To avoid quenching effect when fluorophore is attached to the protein, a longer arm spacer could be used. This spacer could be made with polyethyleneglycol (PEG) molecules, in specific embodiments. In specific embodiments, a biotin-fluorophore that keeps a fluorescence signal when the molecule is bind to Avidin may be employed.

Enzyme-Linked ImmunoSorbent Assay (ELISA)

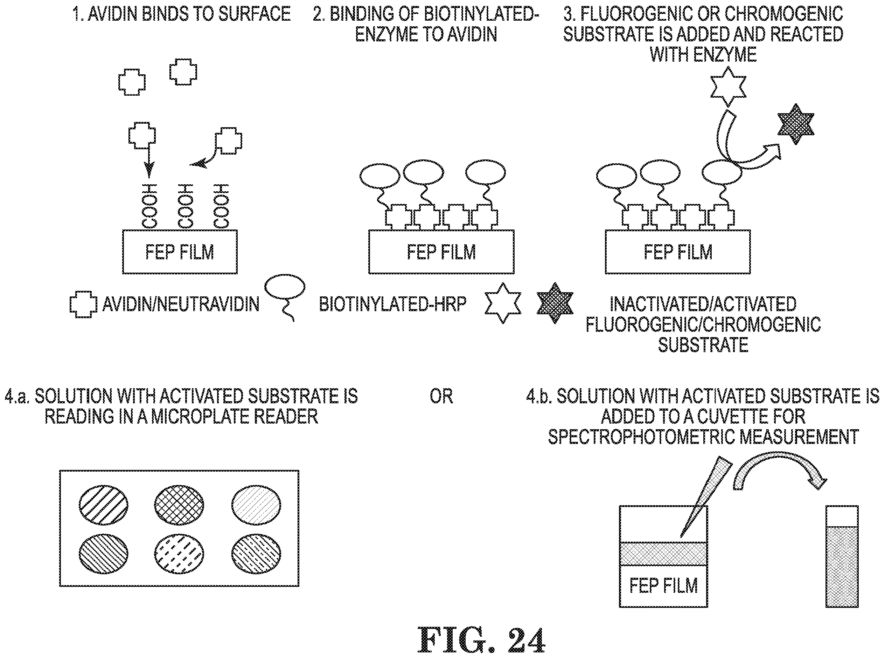

ELISA is an assay designed for detecting and quantifying different molecules such as peptides, antibodies and proteins. This method may be used to determine the amount of protein on a sample and to verify if attached proteins are active. (Vermette, *et al.* 2003). FIG. 24 shows an embodiment of how ELISA may be performed on a FEP sample. First, avidin/neutravidin is attached to the surface. After this step, FEP surface is washed with a solution of bovine serum albumin (BSA) to block the surface and avoid unspecific enzyme binding. Second, biotinylated-enzyme solution is added to the functionalized surface and bound to the biotin-binding site. Calf intestinal alkaline phosphatase (CIP) and Horseradish peroxidase (HRP) enzyme may be conjugated to the biotin molecule and used for this assay on FEP film. After incubation, the surface is rinsed again to remove non-binding enzyme. Third, a reactive substrate is added on the surface and incubated for a specific amount of time depending of the substrate. The choice of substrate depends on the assay sensitivity and the instrumentation available (spectrophotometer or microplate reader, for example). Luminescent and fluorescent substrates are more sensitive and may be used to detect a very small amount of proteins (picometer range),

In particular embodiments, an ELISA method gives a good idea of the biological activity of the attached protein. If Neutravidin stays active after its attachment, the biotinylated-enzyme will be able to bind to the specific site of Avidin/Neutravidin. Those bound enzymes will react with the substrate and generate the chromogenic or fluorogenic product. Those products will be recorded by measuring the absorbance of the solution. The absorbance will increase when chromogenic or fluorogenic product concentration will increase in the solution, so there is a direct correlation between the substrate product concentration and the amount of bound proteins of the film.

Atomic Force Microscopy (AFM)

Atomic force microscopy is employed to characterize the morphology of a polymer. It measures the force between a sample surface and a very sharp probe tip mounted on a cantilever beam. This method allows observation of the presence of immobilized protein to a surface. This method is visual and could map attached protein on a few $\mu$m, in specific embodiments. A tapping mode could be also used to verify the stability and the biological activity of the protein. By using a biotin tips on the cantilever, one can tap the protein on the surface and analyze the energy needed to remove the adsorbed protein on the surface. If this energy is similar to the linking energy between Avidin and biotin, one can know that Avidin adsorption to the surface is stronger than biotin-avidin interactions.

## EXAMPLES OF EXPERIMENTAL PROTOCOLS

Provided below are examples of experimental protocols referred to in this Example.

**FEP surface staining with methylene blue** (identifies -COOH and carboxylate groups on a surface):

1. Prepare $10^{-3}$ M methylene blue solution at pH 7.0.

   a. Mix 0.32g of methylene blue in 1 liter of $dH_2O$

   b. Adjust pH with HCl or NaOH to reach pH 7.0

2. Immerse film in methylene blue solution for 10 min.

3. Rinse film by soaking surface in dH2O (pH=7.0) for 1 minute and spray surface

4. Dry with a mild air flow or incubate in fumes hood.

Analysis: determine the methylene blue absorption by UV/Vis spectroscopy. Measure absorbance on film at 600 nm.

**Staining attached proteins on film** (allows identification of the presence of protein attached on polymer films

1. Place the 2"$\times$1.5" film on a watchglasse and recover the functionalized surface with 10 mL of reagent (Reagent volume could be adjust to recover completely the sample)

2. Cover and incubate for 5 minutes at room temperature.

3. Remove sample from the solution and wash with plenty of $dH_2O$ (dip 5X and use bottle to spray $dH_2O$ on surface)

4. Dry sample with mild air flow or let dry under the hood

Analysis: determine the protein reagent absorption by UV/Vis spectroscopy and measure absorbance on film at 660 nm.

**Proteins concentration in solution before/after surface functionalization**

1. Prepare a standard curve within the assay's working range (25-2000 $\mu$g/mL). Mix 10 $\mu$L of the 1000 $\mu$g/mL standard with 390 $\mu$L of 0.9% saline and 0.05% sodium azide to obtain 25$\mu$g/mL standard solution. Or, prepare a standard curve with 100 μg/mL Neutravidin solution in 10mM NaOAc buffer.

2. Add 0.1 mL of each replicate of standard, sample blank sample into test tube. (Keep a ratio of 1:15 if using smaller sample volume)

3. Add 1.5 mL of the Protein Assay Reagent to each tube and vortex to mix well.

4. Cover and incubate for 5 minutes at room temperature.

Analysis: determine the protein reagent absorption by UV/vis spectroscopy. Measure absorbance of liquid at 660 nm. Prepare the standard curve (absorbance vs protein concentration) before test sample and correct absorbance by subtracting absorbance of the blank solution (10 mM NaOAc buffer or 0.9% saline and 0.05% of sodium azide)

**Neutravadin stability on surface: SDS wash**

1. Prepare the SDS 1% washing solution (2% can also be used) (SDS concentration test range: 0.1 mM (0.0288g/L) SDS for human serum albumin to >10 mM; (2.88 g/L) SDS for SOD and streptavidin)

    a. Mix 1 g of SDS in 100 mL of $dH_2O$

2. Wash film surface by diving the film in the SDS washing solution for 5 min.

3. Rinse surface with $dH_2O$ and dry the film

Perform FTIR measurement on film before and after the washing step.
Protein concentration in the washing solution could be analyzed with the Piece 660 nm protein assay. The ionic detergent compatibility reagent (IDCR #22663) is required will a concentration of SDS >0.0125%.

Neutravadin stability on surface: ultrasonication was

1. Put sample in a 50 mL conic tube and fill tube with the appropriate buffer. Make sure tube is completely closed and sealed.

2. Place the conical tube in the sonicator filled with $dH_2O$

3. Run sample in sonicator for 5 min between 30kHz to 200 kHz.

4. Remove sample from tube and wash with $dH_2O$ (Dip 5X)

5. Dry at room temperature under hood.

Analysis:

1. Perform FTIR measurement on film before and after the ultrasonication.

2. Protein concentration in the buffer could be analyzed with the Piece 660 nm protein assay.

**Biotin beads conjugation with attached avidin/neutravidin to film**

1. Add functionalized surface to 100 mL of 1X PBS buffer

    a. 1X PBS buffer: 50 mL of 10X PBS in 450 mL of $dH_2O$.

2. Add 0.2 mL of Biotin-polystyrene beads solution. Vortex beads solution before use

3. Incubate at room temperature under hood for 1 hour.

4. Wash with PBS (1X) and with plenty of dH$_2$O (5X).

5. Let surface dry in the hood.

Analysis: Use FTIR and/or SEM

**Adsorption of Avidin/Neutravidin on FEP film**

1. Prepare 10 mM NaOAc buffer solution

    a. In a volumetric flask, add 1.7 mL of 3M NaOAc to 498.3 mL of dH$_2$O

2. Prepare 0.1mg/mL protein solution. One can utilize 10 mL of protein solution per 1.5"X2" film sample

    a. In a 50 mL cylindrical single-use tube, add 0.004g of protein (Avidin or Neutravidin) in 40 ml of 10 mM NaOAc

3. Set down the film sample in a watchglasse and cover the film with 10mL of 0.1mg/mL protein solution.

4. Incubate for 1 minute at room temperature

5. Wash sample with 10 mM NaOAc (dip 5X)

6. Wash sample with dH$_2$O (dip 5X)

7. Dry in petri dish at room temperature

8. Store in freezer at -80°C

**Chemical conjugation of Avidin/Neutravidin on FEP film**

** Used oxidized C-treated FEP film sample for this experiment

1. Prepare 10 mM NaOAc buffer solution

    a. In a volumetric flask, add 1.7 mL of 3M NaOAc to 498.3 mL of dH$_2$O

2. Prepare 0.14mg/mL NHS and 0.20mg/mL EDC solution

    a. In a volumetric flask, add 0.07g of NHS in 500 mL of 10 mM NaOAc

    b. Add 0.1 g of EDC in the solution

3. Prepare 0.1mg/mL protein solution. One can utilize 10 mL of protein solution per 1.5"X2" film sample

    a. In a 50 mL cylindrical single-use tube, add 0.004g of protein (Avidin or Neutravidin) in 40 ml of 10 mM NaOAc

4. Add film in the NHS/EDC solution and incubate at room temperature for 10 minutes

5. Wash film by dipping it 5 times in 10 mM NaOAC solution.

6. Set down the film sample in a watchglasse and recover the film with 10mL of 0.1mg/mL protein solution.

7. Incubate for 1 hour at room temperature

8. Prepare 100 mM glycine-HCl solution

    a. In a volumetric flask, add 2.79 g of glycine-HCl in 250 mL of $dH_2O$

9. Wash sample with Glycine-HCl solution (dip 5X)

10. Wash sample with $ddH_2O$ (5X)

11. Wash sample with 10 mM NaOAc (dip 5X)

12. Wash sample with $dH_2O$ (dip 5X)

13. Dry in petri dish at room temperature

14. Store in freezer at -80°C

**Oxidation of C-Treated FEP film**

1. Prepare oxidation solution in a volumetric flask

    a. 1g $KMnO_4$

    b. 5 mL $H_2SO_4$, 98%

    c. Ad 1000 mL $dH_2O$

2. In a beaker, dip sample in the oxidation solution (100mL/sample, do not place more than 2 films in the same beaker)

3. Incubate at room temperature for 2 hours.

4. Rinse with plenty of $dH_2O$ (dip 5X and spray)

5. Dry sample overnight in vacuum oven at 65°C and -20 in Hg.

6. Store at room temperature. Keep away from light

EXAMPLE 3

EXEMPLARY EXPERIMENTAL PROCEDURE

**Experimental**

The grafting polymerization onto FEP films was achieved via plasma activation followed by free radical polymerization in solution. The methodology for the grafting polymerization is described in the following steps; (i) cleaning the film, (ii) treating the film in the low pressure plasma system, (iii) polymerization in solution (iv) cleaning of the film and (v) characterization.

**_Cleaning of the films_**

The film was cut and then washed in acetone, rinse with DI water, dry in air and store inside a film of aluminum foil. Later the cleaning process included washing first the film with soap, rinse with water and then rinse with acetone. The acetone at the end helps to dry the excess of water from the surface.

*Plasma treatment*

The film is placed in the bottle and adhered with double side tape if they are concerns with being activated in both sides. Films of 4" × 5" fit the side of the bottle and no tape is used in the process. The conditions used in the instrument are:

 a. Gas: Argon MCF 1

 b. Gas flow: 20 sccm (cm$^3$/min) as recommended in the literature

 c. Time: 10 min

 d. Power: 30 % (max 100W)

*Polymerization reaction:*

The solution was prepared 30 min before activating the surface to purge the oxygen dissolved in the solution using the in-house nitrogen. The conditions are:

 e. Solvent: DI water

 f. Monomer: Acrylic acid

 g. Atmosphere: $N_2$

 h. Temperature: 60 - 70 °C

 i. Time: Heated for 2- 4 hr and then allowed to stay overnight under nitrogen.

*Cleaning of the final film:*

The film is taken out of the reactor and then it is rinse in DI water, ultrasonic bath for 5 min in water, rinse in acetone and dry with air. The samples are covered with aluminum foil to avoid contamination.

The FEP film is now functionalized with many carboxylic acid groups from the polyacrylic acid grafting polymerization reaction.

EXAMPLE 4

PROTEIN IMMOBILIZATION ON PAA-FEP SURFACE

Two exemplary protein immobilization methods, protein conjugation and protein absorption, were used in this Example. To chemically conjugate the protein, two-step EDC/NHS chemistry was utilized to activate the carboxylic acid group on the pAA-FEP surface. Briefly, 45 mM EDC and 15 mM NHS were freshly prepared and mixed in 0.1 M 2-(N-morpholino)ethanesulfonic acid (MES) buffer solution. Next, 10 ml of this solution was placed on a 100 ml watch glass, and the pAA-treated side of a FEP film sample (3.5 × 3.5 cm$^2$) was placed on the liquid and fully treated for 15 minutes. The amine-reactive NHS-ester intermediates were then generated on the film surface. The coupling reaction was then achieved by immersing the film in 10 ml of avidin or neutr-avidin (NAv) solution (0.1 mg/ml) in DI water for 2 hours.

The protein absorption film sample was prepared by treating the pAA treated surface with 10 ml of 0.1 mg/ml protein in DI water solution for 2 hours without activated by EDC/NHS beforehand. The film sample only activated by EDC/NHS was prepared as a negative control sample, which was treated only by DI water for 2 hours without any protein. And the buffer only sample was prepared by treating the film sample with MES buffer for 15 min only.

All the film samples were rinsed by 10 mM sodium acetate solution, 100 mM glycine-HCl solution, and deionized water subsequently, dried overnight, and stored in - 20°C freezer before further analysis.

Fourier Transform Infrared Characterization

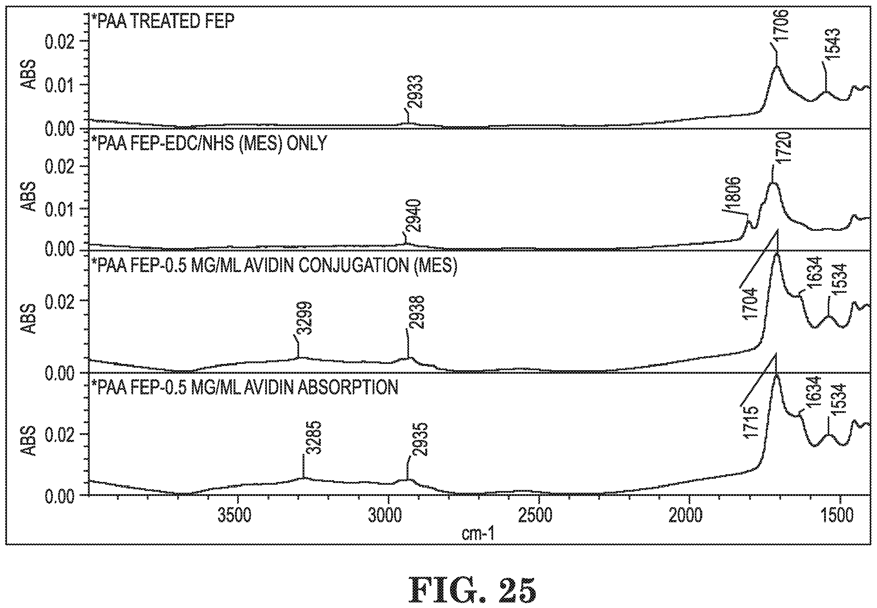

In order to verify the existence of protein on the pAA-FEP film surface, the attenuated total reflectance Fourier Transform Infrared (ATR-FTIR) characterization was used. The film samples (pAA-FEP conjugated with protein, pAA-FEP with protein absorption, pAA-FEP treated by EDC/NHS only and untreated pAA-FEP) were tested. The ATR-FTIR characterization setting parameters are: resolution=4, number of scans= 512, and range of wavenumbers: 4000-1400 cm$^{-1}$ (FIG. 25).

Characterization of Avidin/NeutrAvidin with Biocytin Fluorophore

Because a biotin molecule can bind to avidin or neutravidin protein with high affinity and selectivity, fluorophores conjugated with biotin are able to detect the existence of the protein. Here, biocytin tetramethylrhodamine (B-TMR, excitation wavelength= 554 nm, emission wavelength= 581 nm) was used to stain the stably immobilized protein. Firstly, each of the film samples (1 × 1 cm$^2$) was placed on a well of a 24-well plate with the treated side up, and 2 mg/ml of bovine serum albumin solution was added to block all the non-specific binding sides on the film for 15 minutes. Any avidin or neutravidin not linked to the pAA-FEP surface by the EDC/NHS reaction is removed by this step. After washing by 1 ml of PBS for 5 times, the samples were stained by 1 μM of B-TMR solution dissolved in PBS for 30 minutes protected from light. The fluorophore solution was then removed, and samples were washed by deionized water and dried overnight. The samples were then observed by stereomicroscope fluorescence adapter (EM Sciences, Hatfield, PA) with the Cyan Blue filter (excitation: 490-515 nm, emission: 550 nm, long pass).

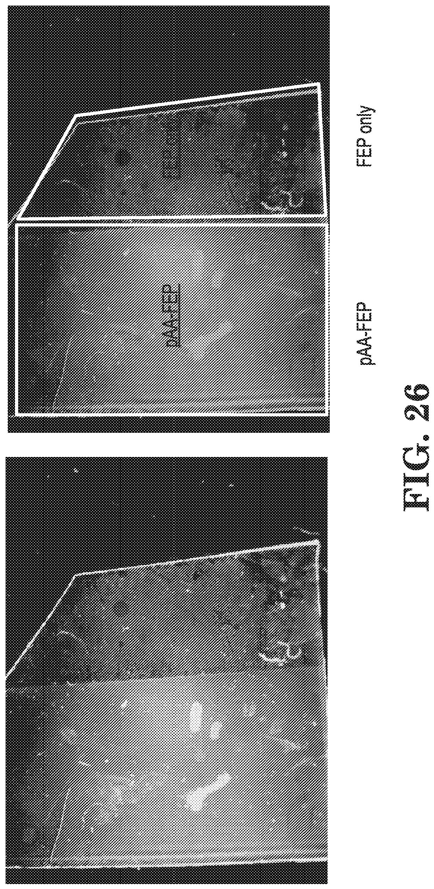

Image of fluorophore-tagged chemically attached avidin to pAA-FEP surfaces adjacent to a section of FEP that was not functionalized with pAA as an internal control. (FIG. 26)

EXAMPLE 5

DNA IMMOBILIZATION ON PAA-FEP SURFACE

Preparation of Aptamer DNA Solution

The lyophilized aptamer DNA oligonucleotides (3'end amine modified, 5'-aptamer-amine-3') and their FITC fluorophore conjugated form were purchased from Base Pair Biotechnologies (47-G06, Oligo#603, Pearland, TX, USA). All the lyophilized aptamers were stored at -20 °C protected from light before use. To resuspend aptamers in solution, the aptamer pellets were centrifuged in a mini-centrifuge for 5 min to ensure all the pellets were on the bottom of the tube. The pellets were then dissolved in nuclease-free water to obtain 100 μM concentration followed by 30 min of incubation. Next, an aliquot of 50 μL of this aptamer solution was taken for further dilution, while the rest of the solution was stored at -20 °C for long-term storage. The aliquot was diluted to its working concentration at 50 nM in folding buffer (1 mM MgCl$_2$ in 1X phosphate buffered saline, nuclease-free, pH 7.4). The aptamers at working concentration were folded by water bathing at 85-95 °C for 5 min followed by cooling in room temperature. No more folding step is required once the aptamers were folded.

The solution folded aptamers at working concentration was separated into 10 aliquots and stored in -20 °C for long-term storage. To avoid repeated freeze-thaw cycles, aptamer solution was kept in 4 °C once it is thawed. At 4 °C when dissolved in nuclease-free water, oligonucleotides are recommended to be used up within 4 weeks ]( www.atbio.com/content/52/Storage -of-oligonucleotides).

Fourier Infrared Transform (FTIR) Characterization for DNA oligonucleotides

To verify the characteristic peaks of DNA oligonucleotides in a FTIR spectrum, 1 mL of 500 pM DNA solution was dipped onto an IR polyethylene (PE) standard film and then dried in air overnight. The FTIR spectrum was tested by ATR mode (number of scan 512, resolution 4 cm$^{-1}$). An untreated PE standard was firstly collected as a background of the spectrum, and the PE film mounted with DNA was then characterized. The spectra were automatic baseline corrected and common scale adjusted.

Covalent Immobilization of DNA on Poly (Acrylic Acid) Fluorinated Polyethylene Propylene (pAA-FEP)

The amine-modified aptamer DNA oligonucleotides were chemically conjugated to pAA-FEP by EDC/NHS chemistry. The procedures are similar to protein conjugation experiment expect the use of coupling buffer. First, the folded aptamer buffer at its working concentration was diluted in coupling buffer (100 mM sodium phosphate, 150 mM sodium chloride, nuclease-free, pH 7.2). The surface carboxylic acid groups of pAA-FEP were activated by 2 mM EDC (0.038 g in 100 ml) and 5 mM NHS (0.06 g in 100 ml) in 0.1 M nuclease-free MES buffer for 15 min. Upon the removal of EDC/NHS reagents, the activated film surface was reacted with the diluted DNA aptamer solution for 2 h. For the DNA absorption samples, the pAA-FEP film sample was immersed in DNA solution for 2 h. The FITC fluorophore tagged DNA (5'-FITC-aptamer-Amine-3') oligonucleotides were immobilized by the same methods and protected from light in the entire procedure. And the negative control samples included pAA-FEP film treated with coupling buffer only after activated by EDC/NHS (namely buffer treated only film) and film treated by EDC/NHS reagent only (Table 6). At the end of the reaction, the film samples were rinsed by folding buffer and nuclease-free water subsequently for three times, and the samples with immobilized DNA were immersed in folding buffer solution and stored in 4 °C before further analysis.

| | DNA conjugation | DNA absorption | EDC/NHS | Buffer only |
|---|---|---|---|---|
| MES | Yes | | Yes | Yes |
| EDC/NHS | Yes | | Yes | Yes |
| DNA | Yes | Yes | | |
| Coupling buffer | Yes | Yes | | Yes |

Table 6: Chemicals and buffers used to prepare different film samples

Fourier Infrared Transform (FTIR) Characterization for Immobilized DNA on pAA-FEP

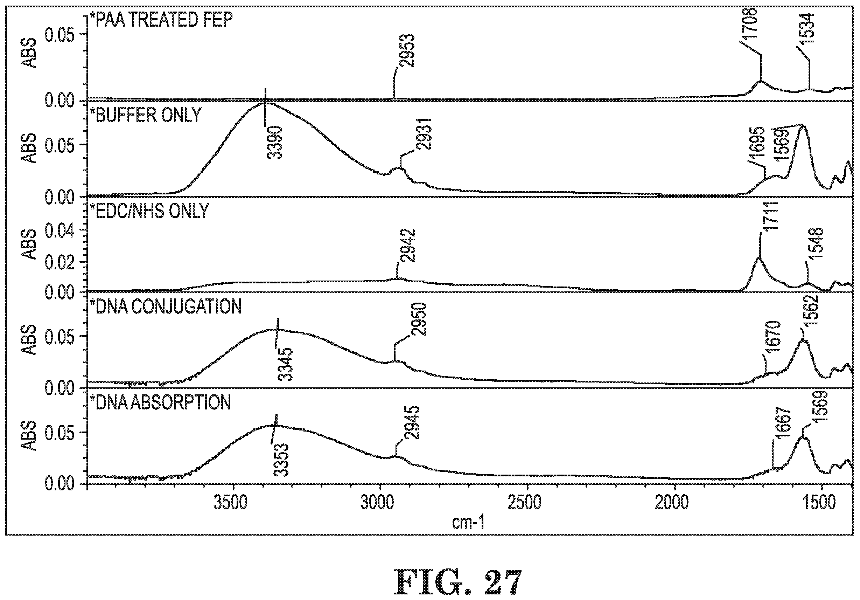

The ATR-FTIR characterization method was also used to verify the existence of DNA on the film samples. The ambient air was firstly corrected as background. As mentioned earlier, each dried sample (DNA conjugation film, DNA absorption film, EDC/NHS treated only film and buffer treated only film) was placed on the ATR crystal and tested by the same condition of section 3.2. The spectra were automatic baseline corrected and common scale adjusted. (FIG. 27).

EXAMPLE 6

SURFACE MODIFICATION EXAMPLES

Biological substances including avidin protein and aptamer DNA oligonucleotides may be covalently immobilized on poly (acrylic acid) grafted fluorinated polyethylene propylene (pAA-FEP). The main modification method is EDC/NHS chemistry, followed by various characterization methods. These procedures of surface modification are useful to produce an aptamer DNA immobilized FEP bag.

**1. Avidin Immobilization Using Two-Step EDC/NHS Chemistry**

**Materials:** 1-ethyl-3-(3-dimethylaminopropyl) carbodiimide hydrochloride (EDC or EDAC, stored at -20° C), N-hydroxysuccinimide (NHS, stored at 4 °C), BupH MES Buffered Saline Packs (stored in room temperature), avidin protein (stored at 4 °C), glycine-HCl, 10 mM sodium acetate buffer and pAA-FEP

**Steps:**

1. Dissolve one pack of MES buffered saline packs into 500 ml of DI water to get 0.1 M MES buffer

2. Weight 0.43 g of EDC and 0.085 NHS and dissolve them together in 50 ml of MES buffer to get 45 mM EDC and 15 mM NHS solution. Avoid any components containing carboxylates and amines

3. Cut a piece of pAA-FEP film samples. To distinguish the pAA grafted side, attach a small piece of labelling tape on the untreated FEP side, or simply fold a corner up

4. Pipette the EDC/NHS solution to fully cover a piece of clean glass watch glass. Normally, 3 ml liquid can fully cover a 50 ml watch glass (for 3*3 cm$^2$ film sample), and 10 ml liquid can cover a 100 ml watch glass (for 5*5 cm$^2$ film sample)

5. Place the film sample on the EDC/NHS solution. Make sure the pAA grafted side can fully contact with the solution

6. Carry out the reaction for 15 min

7. Quickly wash the treated film surface with DI water (optional)

8. Dissolve avidin protein in DI water to acquire 0.1 mg/ml protein concentration, and pipette this solution to fully cover another clean glass watch glass

9. Carry out the reaction for 2 hours

10. Other sample groups are prepared as followed:

   a. avidin absorption sample: treat the pAA-FEP film only with avidin solution for 2 hours

   b. EDC/NHS only sample: treat the sample with EDC/NHS solution for 15 min and then treat it with DI water without avidin

   c. Buffer only sample: treat the sample with 0.1 M MES buffer only

11. Dissolve 2.79 g of glycine-HCl in 100 ml of DI water to get 100 mM glycine-HCl solution

12. Wash all the film samples with 10 mM sodium acetate solution, 100 mM glycine-HCl and DI water subsequently by dipping them in the washing solution in a beaker. Dry the film samples overnight for further investigation.

## 2. Avidin Protein Characterization with Biocytin Fluorophore

**Materials**: 5-(and-6)-Tetramethylrhodamine Biocytin (B-TMR, stored at - 20°C), 10X phosphate buffer saline (PBS), 2 mg/ml bovine serum albumin (BSA), Cyan Blue fluorescent filter set, 24 well plates

**Steps:**

1. Dilute the 10X PBS buffer by 10 times

2. Dissolve 1.7 mg of B-TMR fluorophore pellets in 2 ml PBS to get 10 mM solution, then dilute this solution by 1000 times to get 10 $\mu$M B-TMR solution

3. Cut a 1*1 cm$^2$ piece from each of the film samples, and place these small samples on a 24 well plate in separate wells with treated side up

4. Treat the film samples for 15 min with 1 ml of 2 mg/ml BSA solution to block the non-specific binding sites. To ensure the films do not flip over, use a small glass rod to hold a corner when adding and removing liquid

5. Discard the BSA solution and wash the film samples with PBS buffer for 5 times

6. Dilute the 10 $\mu$M B-TMR solution to the working concentration at 1 $\mu$M by PBS buffer

7. Stain the samples with 1 $\mu$M B-TMR solution for 30 min

8. Wash the samples by dipping them in DI water and dry them overnight

9. When viewing the samples under the stereomicroscope fluorescent attachment, assemble the filter set according to the vendor's manual, and cover the whole microscope by a black plastic bag to avoid seeing the light directly by eyes, and to block the ambient background light

10. Place a sample on a clean black background (i.e. a small sample container). Tilt the sample slightly to minimize the reflection from the excitation light and then adjust the focus.

### 3. Preparation of Aptamer DNA Working Solution

**Materials:** Nuclease-free water, aptamer DNA pellets (amine modified, with and without FITC), magnesium chloride solution ($MgCl_2$, 1 M), nuclease-free PBS buffer, mini-centrifuge, mini-centrifuge tubes, 500 ml sterile jars

**Steps:**

1. Spin down the lyophilized aptamer DNA pellets to the bottom of the tube by centrifuging them for 5 min

2. Resuspend the aptamer DNA pellets by dissolving them in nuclease-free water to achieve 100 $\mu$M (the volume of solvent required is listed in the product information from the vendor). Incubate this solution for 30 min

3. Prepare the folding buffer by mixing 0.1 ml of 1 M $MgCl_2$ solution with 100 ml 1X nuclease-free PBS buffer, and then store the buffer in a 500 ml sterile jar

4. Heat up about 50 ml water in a beaker to 95 $^0$C

5. To fold the aptamer DNA, take an aliquot of the 100 $\mu$M aptamer solution and dilute it to a working concentration (i.e., 2 $\mu$M) in a 50 ml sterile tube. Next, water bath the aptamer DNA solution in the heated water for 5 min, and the cool down the solution in room temperature for about 15 min

6. Separate the aptamer solutions by aliquots in nuclease-free tubes. Store the unused solution in -20 $^0$C for long term storage. Once the solution is thaw, store it in 4 $^0$C to avoid repeated freeze-thaw circles. The FITC-aptamer solution should be prepared protected from light

### 4. Aptamer DNA Immobilization Using Two-Step EDC/NHS Chemistry

**Materials:** sodium chloride and sodium phosphate, EDC/NHS reagent

**Steps:**

1. Prepare the coupling buffer: dissolve 0.87 g sodium chloride and 1.64 g sodium phosphate in nuclease-free water, and then store the buffer in a 500 ml sterile jar
2. Weight 0.019 g of EDC and 0.03 g NHS and dissolve them together in 50 ml of MES buffer to get 2 mM EDC and 5 mM NHS solution. Avoid any components containing carboxylates and amines
3. Cut a piece of pAA-FEP film samples. To distinguish the pAA grafted side, attach a small piece of labelling tape on the untreated FEP side, or simply fold a corner up
4. Pipette the EDC/NHS solution to fully cover a piece of clean glass watch glass. Normally, 3 ml liquid can fully cover a 50 ml watch glass (for 3*3 $cm^2$ film sample), and 10 ml liquid can cover a 100 ml watch glass (for 5*5 $cm^2$ film sample)
5. Place the film sample on the EDC/NHS solution. Make sure the pAA grafted side can fully contact with the solution
6. Carry out the reaction for 15 min
7. Quickly wash the treated film surface with DI water (optional)
8. Dilute the aptamer DNA solution to desired concentration for immobilization by coupling buffer, and pipette the aptamer solution to fully cover a piece of clean watch glass, then put the treated film surface onto the aptamer solution
9. Carry out the reaction for 2 hours
10. Other sample groups are prepared as followed:

a. Aptamer DNA absorption sample: treat the pAA-FEP film only with DNA solution for 2 hours

b. EDC/NHS only sample: treat the sample with EDC/NHS solution for 15 min and then treat it with nuclease-free water

c. Buffer only sample: after performing the EDC/NHS reaction, treat the sample with the same amount of folding buffer and coupling buffer in aptamer solution (i.e. if the aptamer solution is in 50% volume ratio of folding buffer and 50% volume ratio of coupling buffer, the buffer used here is the same ingredient)

11. Wash all the samples with nuclease-free water, and immerse the DNA-containing sample in folding buffer and store at 4 °C.

REFERENCES

All publications mentioned in this specification are indicative of the level of those skilled in the art to which the invention pertains. Albers, Willem M., Jani M. Pelto, Clement Suspène, Juha A. Määttä, Abderrahim Yassar, Vesa P. Hytönen, Inger M. Vikholm-Lundin, and Kirsi Tappura. "Structural and Functional Characteristics of Chimeric Avidins Physically Adsorbed onto Functionalized Polythiophene Thin Films." ACS Applied Materials & Interfaces 4.8 (2012): 4067-077.

Antharavally, Babu S, Krishna A Mallia, Priya Rangaraj, Paul Haney, and Peter A Bell. "Quantitation of Proteins using a Dye-metal-based Colorimetric Protein Assay." Analytical Biochemistry, 2009: 342-345.

Anton Paar. SurPASS Electrokinetic Analyzer: Instruction manual. Instruction manual, Graz: Anton Paar, 2013.

Balamurugan, Subramanian, Anne, Obubuafo, et al. "Surface immobilization methods for aptamer diagnostic applications" Anal Bioanal Chem, 2008: 1009-1021.

Brundle, Richard C, Charles A Evans Jr, and Shaun Wilson. Encyclopedia of Materials Characterization. Greenwich: Butterworth-Heinemann, 1992.

Coates, John. "Interpretation of Infrared Spectra, A Practical Approach." In Encyclopedia of Analytical Chemistry, 10815-10837. Chichester: John Wiley & Sons, 2000.

Drumheller, Paul D, and Jeffrey A Hubbell. "Surface Immobilization of Adhesion Ligands for Investigation of Cell-Substreate Interactions." 9.1 -9.14. CRC Press LLC, 2003.

Fabre, Bruno, Cristian Samori, and Alberto Bianco. "Immobilization of Double Functionalized Carbon Nanotubes on Glassy Carbon Electrodes for the Electrochemical Sensing of the Biotin-avidin Affinity." Journal of Electroanalytical Chemistry 665 (2012): 90-94.

Gavreau, Virginie, Pascale Chevallier, et al. "Engineering Surfaces for Bioconjugation: Developing Strategies and Quantifying the Extent of the Reactions." Bioconjugate Chem., 2004: 1146-1156.

Heikkinen, J.J., et al. "Covalent Biofunctionalization of Cellulose Acetate with Thermostable Chimeric Avidin." ACS Appl. Mater. Interfaces, 2011: 2240-2245.

Lachmann, K., Dohse, A., et al. "Surface modification of closed plastic bags for adherent cell cultivation." Eur. Phys. J. Appl. Phys., 2011:13812-p1-7.

McGettrick, J.D., et al. "A Substrate-Independent Approach for the Surface Immobilization of Oligonucleotides using Aldehyde Functionalized Surfaces." Chem. Vap. Deposition, 2009:122-127.

Ocana, C. and M. del Valle. "A Comparison of four protocols for the immobilization of an aptamer on graphite composite electrodes." Microchim Acta, 2013.

Oh, S. J. et al. "Surface Modification for DNA and Protein Microarrays." Journal of Integrative Biology, 2006: 327-343.

Orelma, Hannes, Leena-Sisko Johansson, Ilari Filpponen, Orlando J Rojas, and Janne Laine. "Generic Method for Attaching Biomolecules via Avidin-Biotin Complexes Immobilized on Films of Regenerated and Nanofibrillar Cellulose." Biomacromolecules, 2012: 2802-2810.

Ozer, A., et al. "New Technologies Provide Quantum Changes in the Scale, Speed, and Success of SELEX Methods nd Aptamer Characterization" Molecular Therapy-Nucleic Acids, 2014: 1-18.

Ponche, A. et al. "Protein/Material Interfaces: Investigation on Model Surfaces." Journal of Adhesion Science and Technology, 2010: 2141-2164.

Rabe, M. Verdes, D. Seeger, S. "Understanding Protein Adsorption Phenomena at Solid Surfaces." Advances in Colloid and Interface Science, 2011: 87-106.

Racine, J., E. Luong-Van, Y. Sadikin, R.K.C. Kang, Y.S. Chu, V. Racine, J.P. Thiery, and W.R. Birch. "A Versatile Gradient of Biomolecules for Regulating Cell Behaviour." Surface and Interfacial Aspects of Cell Adhesion, 2010: 301-318.

Srivastava, A. et al. "Polymer-antibody fragment conjugates for biomedical applications." Progress in Polymer Science, 2014: 308-329.

Sromqvist, J, et al. "Binding of biotin to Streptavidin: A combined fluorescence correlation spectroscopy and time-resolved fluorescence study." The European Physical Journal, 2011: 181-194.

Sun, H., et al. "Oligonucleotide Aptamers: New Tools for Targeted Cancer Therapy" Molecular Therapy-Nucleic Acids, 2014: 1-14.

Thermo Scientific. "Assay Development Technical Handbook." In Thermo Scientific Pierce Assay Development Technical Handbook, by Thermo Scientific. 2011.

Tong, Yen W., and Molly S. Shoichet. "Peptide surface modification of poly(tetrafluoroethylene-co-hexafluoropropylene) enhances its interaction with central nervous system neurons." Journal of Biomedical Materials Research, 1998: 85-95.

Vermette, Patrick, Thomas Gengenbach, Upulie Divisekera, Peter A Kambouris, Hans J Griesser, and Laurence Meagher. "Immobilization and Surface Characterization of Neutravidin Biotin-binding Protein on Different Hydrogel Interlayers." Journal of Colloid and Interface Science, 2003: 13-26.

Vesel, Alenka, and Kristina Elersic. "Adsorption of Protein Streptavidin to the Plasma Treated Surface of Polystyrene." Applied Surface Science 258.15 (2012): 5558-560.

Vesel, Alenka, Kristina Elersic, and Miran Mozetic. "Immobilization of Protein Streptavidin to the Surface of PMMA Polymer." Vacuum 86.6 (2012): 773-75.

Xia, Hui, Kermit Murray, Steven Soper, and June Feng. "Ultra Sensitive Affinity Chromatography on Avidin-functionalized PMMA Microchip for Low Abundant Post-translational Modified Protein Enrichment." Biomedical Microdevices 14.1 (2012): 67-81.

Ye, M., et al., "Generating Aptamers by Cell-SELEX for Applications in Molecular Medicine" Int. J. Mol. Sci., 2012: 3341-3353.

Yun, J.K., K. DeFife, et al., "Human monocyte/macrophage adhesion and cytokine production on surface-modified poly(tetrafluoroethylene/hexafluoropropylene) polymers with and without protein preadsorption." Journal of Biomedical Materials Research, 1995: 257-268.

Zhou, J. and J. J. Rossi, "Cell-type-specific, Aptamer-functionalized Agents for Targeted Disease Therapy" Molecular Therapy Nucleic Acids, 2014: 1-13.

## Claims

1. A closed isolation system for isolating and culturing cells, the system comprising a container in the form of a cell culture bag, wherein said cell culture bag comprises a sheet of film, the sheet of film defining an inner surface of the cell culture bag, the inner surface of the cell culture bag comprising

   a polymer having a total organic carbon (TOC) in water of less than 0.1 mg/cm$^2$, the total organic carbon (TOC) measured according to Pharmacopeia (USP) 643, and
   a plurality of functional groups covalently attached to the polymer,
   wherein the polymer is fluorinated ethylene propylene (FEP),
   wherein the functional groups are directly or indirectly attached to a cell-capturing moiety
   that is a nucleic acid,
   wherein the functional groups are carboxy groups or amine groups.

2. The system of claim 1, wherein the functional groups are attached to the polymer through a linker,

   wherein the linker preferably is a linear alkylene group, a branched alkylene group, a cyclic alkylene group, an arenediyl group, or a combination thereof,
   wherein the linear alkylene group preferably is a methylene group, an ethylene group, a propylene group, a butylene group, a pentylene group, or a hexylene group.

3. The system of claim 1, wherein the carboxy groups or amine groups are made by a process comprising plasma treatment of the polymer.

4. The system of claim 1, wherein the functional groups have attached directly thereto a first member of a binding pair and the cell - capturing moiety comprises a second member of a binding pair, wherein said first and second members of the binding pair are bound to each other,

   wherein when the first member of the binding pair is an avidin species, the second member of the binding pair is biotin, and
   wherein when the first member of the binding pair is biotin, the second member of the binding pair is an avidin species.

**5.** The system of any one of the preceding claims,
wherein the cell-capturing moiety is a nucleic acid aptamer configured to bind the cells directly.

**6.** The system of any one of the preceding claims,wherein the cell-capturing moiety is configured to indirectly bind the biological agent or to produce a molecule that directly binds the cells.

**7.** The system of claim 4,

wherein the cell-capturing moiety is a nucleic acid template,
wherein the nucleic acid template preferably is a circular DNA template.

**8.** The system of any of claims 1-5, wherein the container further comprises a polymerase,
wherein the polymerase preferably is phi29 polymerase.

**9.** The system of any of claims 1-7, further comprising one or more additional containers attached in-line to the container,

preferably wherein there is a second container that comprises one or more cell growth agents, and that further comprises two or more membranes,
preferably wherein there is a third container that comprises one or more antigens, preferably wherein there is a fourth container that is configured to concentrate cells, wherein the cells preferably are blood cells, immune cells, or a mixture thereof.

**10.** A method for isolating particular cells from a sample comprising a mixture of cells from at least one individual, comprising the steps of:

providing a closed isolation system for isolating the cells according to any one of the preceding claims, and releasing the isolated particular cells from the cell-capturing moiety or the molecule produced by the cell-capturing moiety.

**11.** The method according to claim 10, further comprising culturing the isolated particular cells.

**12.** The method according to any of claims 10-11, wherein the functional groups are carboxy groups or amine groups made by plasma treatment of the polymer.

**Patentansprüche**

**1.** Geschlossenes Isolierungssystem zum Isolieren und Kultivieren von Zellen, wobei das System einen Behälter in Form eines Zellkulturbeutels umfasst, wobei der Zellkulturbeutel eine Filmfolie umfasst, wobei die Filmfolie eine Innenfläche des Zellkulturbeutels definiert, wobei die Innenfläche des Zellkulturbeutels

ein Polymer mit einem gesamten organischen Kohlenstoff (TOC) in Wasser von weniger als 0,1 mg/cm$^2$, wobei der gesamte organische Kohlenstoff (TOC) gemäß der Pharmacopeia (USP) 643 gemessen wird, und eine Vielzahl von funktionellen Gruppen umfasst, die kovalent an das Polymer gebunden sind, wobei das Polymer fluoriertes Ethylenpropylen (FEP) ist, wobei die funktionellen Gruppen direkt oder indirekt an eine zelleinfangende Einheit gebunden sind, die eine Nukleinsäure ist, wobei die funktionellen Gruppen Carboxygruppen oder Amingruppen sind.

**2.** System nach Anspruch 1, wobei die funktionellen Gruppen über einen Linker an das Polymer gebunden sind,

wobei der Linker vorzugsweise eine unverzweigte Alkylengruppe, eine verzweigte Alkylengruppe, eine cyclische Alkylengruppe, eine Arendiylgruppe oder eine Kombination davon ist, wobei die unverzweigte Alkylengruppe vorzugsweise eine Methylengruppe, eine Ethylengruppe, eine Propylengruppe, eine Butylengruppe, eine Pentylengruppe oder eine Hexylengruppe ist.

**3.** System nach Anspruch 1, wobei die Carboxygruppen oder Amingruppen durch ein Verfahren hergestellt werden, das eine Plasmabehandlung des Polymers umfasst.

4.  System nach Anspruch 1, wobei an die funktionellen Gruppen direkt ein erstes Glied eines Bindungspaares gebunden ist und die zelleinfangende Einheit ein zweites Glied eines Bindungspaares umfasst, wobei das erste und das zweite Glied des Bindungspaares aneinander gebunden sind,

    wobei, wenn das erste Glied des Bindungspaares eine Avidin-Spezies ist, das zweite Glied des Bindungspaares Biotin ist, und
    wobei, wenn das erste Glied des Bindungspaares Biotin ist, das zweite Glied des Bindungspaares eine Avidin-Spezies ist.

5.  System nach einem der vorhergehenden Ansprüche,
    wobei die zelleinfangende Einheit ein Nukleinsäure-Aptamer ist, das konfiguriert ist, um die Zellen direkt zu binden.

6.  System nach einem der vorhergehenden Ansprüche, wobei die zelleinfangende Einheit so konfiguriert ist, dass sie das biologische Mittel indirekt bindet oder ein Molekül produziert, das die Zellen direkt bindet.

7.  System nach Anspruch 4,

    wobei die zelleinfangende Einheit eine Nukleinsäurematrize ist,
    wobei die Nukleinsäurematrize vorzugsweise eine zirkuläre DNA-Matrize ist.

8.  System nach einem der Ansprüche 1 bis 5, wobei der Behälter ferner eine Polymerase umfasst,
    wobei die Polymerase vorzugsweise phi29-Polymerase ist.

9.  System nach einem der Ansprüche 1-7, das ferner einen oder mehrere zusätzliche Behälter umfasst, die in Reihe an dem Behälter angebracht sind,
    wobei vorzugsweise ein zweiter Behälter vorhanden ist, der ein oder mehrere Zellwachstumsmittel umfasst und der ferner zwei oder mehr Membranen umfasst, wobei es vorzugsweise einen dritten Behälter gibt, der ein oder mehrere Antigene umfasst, wobei es vorzugsweise einen vierten Behälter gibt, der konfiguriert ist, um Zellen zu konzentrieren, wobei die Zellen vorzugsweise Blutzellen, Immunzellen oder eine Mischung davon sind.

10. Verfahren zum Isolieren bestimmter Zellen aus einer Probe, die eine Mischung von Zellen von mindestens einem Individuum umfasst, umfassend die Schritte:
    des Bereitstellens eines geschlossenen Isolierungssystems zum Isolieren der Zellen nach einem der vorhergehenden Ansprüche und des Freisetzens der isolierten bestimmten Zellen von der zelleinfangenden Einheit oder dem Molekül, das von der zelleinfangenden Einheit produziert wird.

11. Verfahren nach Anspruch 10, das ferner das Kultivieren der isolierten bestimmten Zellen umfasst.

12. Verfahren nach einem der Ansprüche 10 bis 11, wobei die funktionellen Gruppen Carboxygruppen oder Amingruppen sind, die durch Plasmabehandlung des Polymers hergestellt wurden.


**Revendications**

1.  Système d'isolement fermé pour isoler et cultiver des cellules, le système comprenant un contenant sous la forme d'un sac de culture cellulaire, dans lequel ledit sac de culture cellulaire comprend une feuille de film, la feuille de film définissant une surface intérieure du sac de culture cellulaire, la surface intérieure du sac de culture cellulaire comprenant

    un polymère ayant un carbone organique total (COT) dans l'eau inférieur à 0,1 mg/cm$^2$, le carbone organique total (COT) étant mesuré selon la pharmacopée (USP) 643, et
    une pluralité de groupes fonctionnels liés de manière covalente au polymère,
    dans lequel le polymère est de l'éthylène propylène fluoré (FEP),
    dans lequel les groupes fonctionnels sont directement ou indirectement liés à une fraction de capture de cellules qui est un acide nucléique,
    dans lequel les groupes fonctionnels sont des groupes carboxy ou des groupes amine.

2.  Système selon la revendication 1, dans lequel les groupes fonctionnels sont liés au polymère par l'intermédiaire d'un lieur,

dans lequel le lieur est de préférence un groupe alkylène linéaire, un groupe alkylène ramifié, un groupe alkylène cyclique, un groupe arènediyle ou une combinaison de ceux-ci,
dans lequel le groupe alkylène linéaire est de préférence un groupe méthylène, un groupe éthylène, un groupe propylène, un groupe butylène, un groupe pentylène ou un groupe hexylène.

3. Système selon la revendication 1, dans lequel les groupes carboxy ou les groupes amine sont préparés par un procédé comprenant le traitement au plasma du polymère.

4. Système selon la revendication 1, dans lequel les groupes fonctionnels ont un premier élément d'une paire de liaison lié directement à ceux-ci et la fraction de capture de cellules comprend un second élément d'une paire de liaison, dans lequel lesdits premier et second éléments de la paire de liaison sont liés l'un à l'autre,

dans lequel lorsque le premier élément de la paire de liaison est une espèce d'avidine, le second élément de la paire de liaison est la biotine, et
dans lequel lorsque le premier élément de la paire de liaison est la biotine, le second élément de la paire de liaison est une espèce d'avidine.

5. Procédé selon l'une quelconque des revendications précédentes,
dans lequel la fraction de capture de cellules est un aptamère d'acide nucléique configuré pour se lier directement aux cellules.

6. Système selon l'une quelconque des revendications précédentes, dans lequel la fraction de capture de cellules est configurée pour se lier indirectement à l'agent biologique ou pour produire une molécule qui se lie directement aux cellules.

7. Système selon la revendication 4,

dans lequel la fraction de capture de cellules est une matrice d'acide nucléique,
dans lequel la matrice d'acide nucléique est de préférence une matrice d'ADN circulaire.

8. Système selon l'une quelconque des revendications 1 à 5, dans lequel le contenant comprend en outre une polymérase,
dans lequel la polymérase est de préférence la polymérase phi29.

9. Système selon l'une quelconque des revendications 1 à 7, comprenant en outre un ou plusieurs contenants supplémentaires attachés en ligne au contenant,
de préférence dans lequel il y a un deuxième contenant qui comprend un ou plusieurs agents de croissance cellulaire, et qui comprend en outre deux membranes ou plus, de préférence dans lequel il y a un troisième contenant qui comprend un ou plusieurs antigènes, de préférence dans lequel il y a un quatrième contenant qui est configuré pour concentrer les cellules, les cellules étant de préférence des cellules sanguines, des cellules immunitaires ou un mélange de celles-ci.

10. Procédé pour isoler des cellules particulières d'un échantillon comprenant un mélange de cellules d'au moins un individu, comprenant les étapes de :
fourniture d'un système d'isolement fermé pour isoler les cellules selon l'une quelconque des revendications précédentes, et libération des cellules particulières isolées de la fraction de capture de cellules ou de la molécule produite par la fraction de capture de cellules.

11. Procédé selon la revendication 10, comprenant en outre la culture des cellules particulières isolées.

12. Procédé selon l'une quelconque des revendications 10 à 11, dans lequel les groupes fonctionnels sont des groupes carboxy ou des groupes amine préparés par traitement au plasma du polymère.

WHOLE BLOOD (50-100ML)

BAG 1 ISOLATE CELLS

BAG 2 GROW CELLS

BAG 3 CONVERT CELLS

BAG 4 CONCENTRATE CELLS

CELL THERAPY DOSE (40 ML)

**FIG. 1A**

FIG. 1B

FIG. 2

EP 3 237 595 B1

FIG. 3

EP 3 237 595 B1

FIG. 4

FIG. 5

ADDITION OF BIOTINYLATED APTAMERS TO THE BLOOD SAMPLE

LOADING OF THE SAMPLE IN THE TUBE

COATING OF AN FEP TUBE WITH NEUTRAVIDIN

REMOVAL OF BLOOD BY CELL STRUCTURE MEDIA: MONOCYTES ARE THE ONLY CELLS LEFT IN THE TUBE

FIG. 6

**FIG. 7**

**FIG. 8**

FIG. 9

**FIG. 10**

EP 3 237 595 B1

FIG. 11

EP 3 237 595 B1

**FIG. 12**

| | UNTREATED FEP WITH MB DYE | C-TREATED FEP WITH MB DYE | OXIDIZED C-TREATED FEP | OXIDIZED C-TREATED FEP WITH MB DYE |
|---|---|---|---|---|
| TOTAL | 0.02561 | 0.02582 | 0.02435 | 0.02871 |
| 600nm | 0.02597 | 0.02602 | 0.02289 | 0.03014 |

**FIG. 13**

EP 3 237 595 B1

OXIDIZED FILM SPECTRUM - METHYLENE BLUE

OXIDIZED FILM MB DYE

C-TREATED AND UNTREATED MB DYE

OXIDIZED CONTROL

WAVELENGTH (nm)

FIG. 14

FIG. 15

200nm EHT = 2.00 kV SAMPLE ID = C-TREATED ADSORBED PROTEIN
WD = 3.8 mm SIGNAL A = INLENS WIDTH = 5.000μm

200nm EHT = 2.00 kV SAMPLE ID = C-TREATED OXIDIZED CONJUGATED
WD = 3.8 mm SIGNAL A = INLENS WIDTH = 5.000μm

**FIG. 16**

EP 3 237 595 B1

**FIG. 17**

FIG. 18

| | UNTREATED FEP | C-TREATED FEP | OXIDIZED C-TREATED FEP ADSORPTION | OXIDIZED C-TREATED FEP CONJUGATION |
|---|---|---|---|---|
| ☐ CONTROL | 0.02116 | | 0.02130 | 0.02123 |
| ▦ AVIDIN | 0.02189 | 0.02299 | 0.02383 | 0.02363 |
| ☐ NEUTRAVIDIN | 0.02384 | 0.02386 | 0.02423 | 0.02427 |

FIG. 19

C-TREATED NEUTRAVIDIN SPECTRUM - 660 nm
PROTEIN ASSAY

FIG. 20

FIG. 21

EP 3 237 595 B1

FIG. 22

EP 3 237 595 B1

FIG. 23

EP 3 237 595 B1

1. AVIDIN BINDS TO SURFACE

2. BINDING OF BIOTINYLATED-ENZYME TO AVIDIN

3. FLUOROGENIC OR CHROMOGENIC SUBSTRATE IS ADDED AND REACTED WITH ENZYME

FEP FILM

AVIDIN/NEUTRAVIDIN    BIOTINYLATED-HRP    INACTIVATED/ACTIVATED FLUOROGENIC/CHROMOGENIC SUBSTRATE

4.a. SOLUTION WITH ACTIVATED SUBSTRATE IS READING IN A MICROPLATE READER

OR

4.b. SOLUTION WITH ACTIVATED SUBSTRATE IS ADDED TO A CUVETTE FOR SPECTROPHOTOMETRIC MEASUREMENT

FIG. 24

EP 3 237 595 B1

FIG. 25

pAA-FEP

FEP only

**FIG. 26**

FIG. 27

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014255976 A1
- US 62095197
- US 62095116
- US 3030290 A
- US 3255099 A
- US 3274089 A
- US 3274090 A
- US 3274091 A
- US 3275540 A
- US 3284331 A

- US 3291712 A
- US 3296011 A
- US 3391314 A
- US 3397132 A
- US 3485734 A
- US 3507763 A
- US 3676181 A
- US 4549921 A
- US 6726979 B

**Non-patent literature cited in the description**

- **CHAN-HEE JUNG et al.** Poly(acrylic acid)-Grafted Fluoropolymer Films for Highly Sensitive Fluorescent Bioassays. *ACS APPLIED MATERIALS & INTERFACES,* 14 March 2013, vol. 5 (6), 2155-2160
- **SADURNI PATRICIA et al.** Immobilization of streptavidinhorseradish peroxidase onto a biotinylated poly(acrylic acid) backbone that had been radiation-grafted to a PTFE film. *JOURNAL OF BIOMATERIALS SCIENCE. POLYMER EDIT. VSP, UTRECHT, NL,* 01 January 2005, vol. 16 (2), 181-187
- **T. G. VARGO et al.** Synthesis and characterization of fluoropolymeric substrata with immobilized minimal peptide sequences for cell adhesion studies. 1. *Journal of biomedical materials research,* vol. 101 (1), 767
- **J. P. RANIERI et al.** Neuronal cell attachment to fluorinated ethylene propylene films with covalently immobilized laminin oligopeptides YIGSR and IKVAV. II. *Journal of biomedical materials research,* 01 June 1995, 779-785
- **K. LACHMANN et al.** *Surface modification of closed plastic bags for adherent cell cultivation,* 31 December 2011
- Ultra-Clean Technology Handbook. CRC Press, 1993, vol. 1, 497-517
- **ALBERS, WILLEM M. ; JANI M. PELTO ; CLEMENT SUSPÈNE ; JUHA A. MÄÄTTÄ ; ABDERRAHIM YASSAR ; VESA P. HYTÖNEN ; INGER M. VIKHOLM-LUNDIN ; KIRSI TAPPURA.** Structural and Functional Characteristics of Chimeric Avidins Physically Adsorbed onto Functionalized Polythiophene Thin Films. *ACS Applied Materials & Interfaces,* 2012, vol. 4.8, 4067-077

- **ANTHARAVALLY, BABU S ; KRISHNA A MALLIA ; PRIYA RANGARAJ ; PAUL HANEY ; PETER A BELL.** Quantitation of Proteins using a Dye-metal-based Colorimetric Protein Assay. *Analytical Biochemistry,* 2009, 342-345
- **ANTON PAAR.** SurPASS Electrokinetic Analyzer: Instruction manual. *Instruction manual, Graz: Anton Paar,* 2013
- **BALAMURUGAN ; SUBRAMANIAN ; ANNE ; OBUBUAFO et al.** Surface immobilization methods for aptamer diagnostic applications. *Anal Bioanal Chem,* 2008, 1009-1021
- **BRUNDLE, RICHARD C ; CHARLES A EVANS JR ; SHAUN WILSON.** Encyclopedia of Materials Characterization. *Greenwich: Butterworth-Heinemann,* 1992
- Interpretation of Infrared Spectra, A Practical Approach. **COATES, JOHN.** Encyclopedia of Analytical Chemistry. Chichester: John Wiley & Sons, 2000, 10815-10837
- **DRUMHELLER, PAUL D ; JEFFREY A HUBBELL.** Surface Immobilization of Adhesion Ligands for Investigation of Cell-Substreate Interactions. CRC Press LLC, 2003
- **FABRE ; BRUNO ; CRISTIAN SAMORI ; ALBERTO BIANCO.** Immobilization of Double Functionalized Carbon Nanotubes on Glassy Carbon Electrodes for the Electrochemical Sensing of the Biotin-avidin Affinity. *Journal of Electroanalytical Chemistry,* 2012, vol. 665, 90-94
- **GAVREAU, VIRGINIE ; PASCALE CHEVALLIER et al.** Engineering Surfaces for Bioconjugation: Developing Strategies and Quantifying the Extent of the Reactions. *Bioconjugate Chem.,* 2004, 1146-1156

- **HEIKKINEN, J.J. et al.** Covalent Biofunctionalization of Cellulose Acetate with Thermostable Chimeric Avidin. *ACS Appl. Mater. Interfaces,* 2011, 2240-2245
- **LACHMANN, K. ; DOHSE, A. et al.** Surface modification of closed plastic bags for adherent cell cultivation. *Eur. Phys. J. Appl. Phys.,* 2011
- **MCGETTRICK, J.D. et al.** A Substrate-Independent Approach for the Surface Immobilization of Oligonucleotides using Aldehyde Functionalized Surfaces. *Chem. Vap. Deposition,* 2009, 122-127
- **OCANA, C ; M. DEL VALLE.** A Comparison of four protocols for the immobilization of an aptamer on graphite composite electrodes. *Microchim Acta,* 2013
- **OH, S. J. et al.** Surface Modification for DNA and Protein Microarrays. *Journal of Integrative Biology,* 2006, 327-343
- **ORELMA, HANNES ; LEENA-SISKO JOHANSSON ; ILARI FILPPONEN ; ORLANDO J ROJAS ; JANNE LAINE.** Generic Method for Attaching Biomolecules via Avidin-Biotin Complexes Immobilized on Films of Regenerated and Nanofibrillar Cellulose. *Biomacromolecules,* 2012, 2802-2810
- **OZER, A. et al.** New Technologies Provide Quantum Changes in the Scale, Speed, and Success of SELEX Methods nd Aptamer Characterization. *Molecular Therapy-Nucleic Acids,* 2014, 1-18
- **PONCHE, A. et al.** Protein/Material Interfaces: Investigation on Model Surfaces. *Journal of Adhesion Science and Technology,* 2010, 2141-2164
- **RABE, M. VERDES ; D. SEEGER, S.** Understanding Protein Adsorption Phenomena at Solid Surfaces. *Advances in Colloid and Interface Science,* 2011, 87-106
- **RACINE, J. ; E. LUONG-VAN ; Y. SADIKIN ; R.K.C. KANG ; Y.S. CHU ; V. RACINE ; J.P. THIERY ; W.R. BIRCH.** A Versatile Gradient of Biomolecules for Regulating Cell Behaviour. *Surface and Interfacial Aspects of Cell Adhesion,* 2010, 301-318
- **SRIVASTAVA, A. et al.** Polymer-antibody fragment conjugates for biomedical applications. *Progress in Polymer Science,* 2014, 308-329
- **SROMQVIST, J et al.** Binding of biotin to Streptavidin: A combined fluorescence correlation spectroscopy and time-resolved fluorescence study. *The European Physical Journal,* 2011, 181-194
- **SUN, H. et al.** Oligonucleotide Aptamers: New Tools for Targeted Cancer Therapy. *Molecular Therapy-Nucleic Acids,* 2014, 1-14
- **THERMO SCIENTIFIC ; THERMO SCIENTIFIC.** Assay Development Technical Handbook. *Thermo Scientific Pierce Assay Development Technical Handbook,* 2011
- **TONG, YEN W. ; MOLLY S. SHOICHET.** Peptide surface modification of poly(tetrafluoroethylene-co-hexafluoropropylene) enhances its interaction with central nervous system neurons. *Journal of Biomedical Materials Research,* 1998, 85-95
- **VERMETTE, PATRICK ; THOMAS GENGENBACH ; UPULIE DIVISEKERA ; PETER A KAMBOURIS ; HANS J GRIESSER ; LAURENCE MEAGHER.** Immobilization and Surface Characterization of Neutravidin Biotin-binding Protein on Different Hydrogel Interlayers. *Journal of Colloid and Interface Science,* 2003, 13-26
- **VESEL, ALENKA ; KRISTINA ELERSIC.** Adsorption of Protein Streptavidin to the Plasma Treated Surface of Polystyrene. *Applied Surface Science,* 2012, vol. 258.15, 5558-560
- **VESEL, ALENKA ; KRISTINA ELERSIC ; MIRAN MOZETIC.** Immobilization of Protein Streptavidin to the Surface of PMMA Polymer. *Vacuum,* 2012, vol. 86.6, 773-75
- **XIA, HUI ; KERMIT MURRAY ; STEVEN SOPER ; JUNE FENG.** Ultra Sensitive Affinity Chromatography on Avidin-functionalized PMMA Microchip for Low Abundant Post-translational Modified Protein Enrichment. *Biomedical Microdevices,* 2012, vol. 14.1, 67-81
- **YE, M. et al.** Generating Aptamers by Cell-SELEX for Applications in Molecular Medicine. *Int. J. Mol. Sci.,* 2012, 3341-3353
- **YUN, J.K. ; K. DEFIFE et al.** Human monocyte/macrophage adhesion and cytokine production on surface-modified poly(tetrafluoroethylene/hexafluoropropylene) polymers with and without protein preadsorption. *Journal of Biomedical Materials Research,* 1995, 257-268
- **ZHOU, J. ; J. J. ROSSI.** Cell-type-specific, Aptamer-functionalized Agents for Targeted Disease Therapy. *Molecular Therapy Nucleic Acids,* 2014, 1-13